(19) Europäisches Patentamt
European Patent Office
Office européen des brevets

(11) EP 3 909 614 A1

(12) **EUROPEAN PATENT APPLICATION**

(43) Date of publication:
**17.11.2021 Bulletin 2021/46**

(21) Application number: **20196027.5**

(22) Date of filing: **14.09.2020**

(51) Int Cl.:
*A61K 47/69* (2017.01) *A61K 39/12* (2006.01)
*A61K 39/00* (2006.01) *A61P 35/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.05.2020 EP 20174156**

(71) Applicants:
- **Life Science Inkubator Betriebs GmbH & Co. KG 53175 Bonn (DE)**
- **DKFZ Deutsches Krebsforschungszentrum 69120 Heidelberg (DE)**

(72) Inventors:
- **RIEMER, Angelika 69120 Heidelberg (DE)**
- **KÜBELBECK, Armin 53175 Bonn (DE)**
- **GRABOWSKA, Agnieszka 53175 Bonn (DE)**
- **KRUSE, Sebastian 69120 Heidelberg (DE)**
- **FEIDT, Eva 53175 Bonn (DE)**
- **JUNGLAS, Ellen 53175 Bonn (DE)**

(74) Representative: **von Renesse, Dorothea et al
König-Szynka-Tilmann-von Renesse
Patentanwälte Partnerschaft mbB
Mönchenwerther Str. 11
40545 Düsseldorf (DE)**

# (54) COMPOSITION OF NANOPARTICLES AS CARRIER FOR HPV-DERIVED IMMUNOGENIC FRAGMENTS

(57) The disclosure relates to a composition of nanoparticles as carrier for HPV-derived immunogenic fragments and the use of the composition for medical purposes, in particular for immunoprophylaxis or immunotherapy. The invention also relates to a vaccine containing the composition and/or nanoparticles.

EP 3 909 614 A1

## Description

## FIELD OF THE INVENTION

The present invention relates to a composition of nanoparticles as carrier for HPV-derived immunogenic fragments and the use of the composition for medical purposes, in particular for immunoprophylaxis or immunotherapy. The invention also relates to a vaccine containing the composition and/or nanoparticles.

## BACKGROUND OF THE INVENTION

Cervical cancer is one of the most frequent cancer types in the world and commonly induced by human papillomavirus (HPV). Furthermore, HPV infection can cause several other premalignant and malignant conditions and leads to hundreds of thousands deaths per year worldwide. Therefore, effective treatment modalities are urgently needed.

Over 100 individual HPV genotypes have been described. "High-risk" HPV genotypes are associated with the risk of developing a malignant condition. These high-risk genotypes include HPV types 16, 18, 31, 33, 35, 39, 45, 51, 52, 56, 58, 59, 66, 68, 73, and 82, which can lead to cervical cancer and are associated with other mucosal anogenital and head and neck cancers (zur Hausen H., Appl Pathol 1987, 5: 19-24; Bosch FX, et al., J Clin Pathol 55: 244-65; Gillison ML, et al., Vaccine 30 Suppl 5: F34-541-3, 2012*)*. HPV16 and HPV18 are the predominant oncogenic types, cumulatively responsible for over 70-80% of all invasive cervical cancer cases. Accumulating data suggest that both cytotoxic CD8+ T cell and CD4+ T helper cell responses play a pivotal role in the control and clearance of HPV infection (Stanley MA, J Reprod Immunol 52: 45-59, 2001*;* Welters MJ, et al., Cancer Res 63: 636-4, 2003*;* Nakagawa M, et al., J Infect Dis 182: 595-8, 2000*;* van der Burg SH, et al., Virus Res 89: 275-84, 2002). The HPV proteins E6 and E7 are especially regarded as being crucial for HPV immune escape and malignant progression. E6 and E7 as major transforming proteins are constitutively expressed in both premalignant and advanced lesions, making them ideal targets for immunotherapeutic approaches for HPV-induced malignancies (zur Hausen H., J Natl Cancer Inst 92: 690-8, 2000*;* Tan S. et al., Curr Cancer Drug Targets 12: 170-84, 2012).

Immunotherapy is an attractive option for treatment of infection and (pre)cancerous conditions. Since CD4+ and CD8+ T cells have been shown to be crucial for the induction and maintenance of cytotoxic T cell responses, and also to be important for direct anti-tumor immunity, immunogenic fragments presented by major histocompatibility complex (MHC) class I and MHC class II are intensively investigated to improve the efficacy of peptide-based HPV immunotherapy, such as HPV vaccines. Each human being expresses three to six MHC class I and at least as many MHC class II molecules, also called human leukocyte antigen (HLA) molecules. To date, more than 3000 variants of human MHC class I and 1000 variants of MHC class II have been characterized (Robinson J, et al., Nucleic Acids Res 37: D1013-D1017, 2009). Thus, the use of immunogenic fragments having the ability to bind to a wide variety of different HLA molecules for effective immunoprophylaxis and immunotherapy is desired. Hence, to focus the cytotoxic T cell response on E6 and/or E7 proteins, it is crucial to define the immunogenic fragments presented on the HLA molecules of malignant cells.

A key advance in coping with HPV infection and related conditions has been the development of virus-like particle (VLP) prophylactic vaccines. To date, the three HPV vaccines available on the market comprise the recombinantly produced HPV16- and HPV18-derived major capsid protein L1. *Cervarix*® (GlaxoSmithKline) comprises recombinantly produced HPV16 and HPV18 VLP and is formulated with the immunostimulant 3-0-desacyl-4'-monophosphoryl lipid A (3D MPL, also known as MPL) and aluminium hydroxide salt. *Gardasil*® and *Gardasil*®*9* (Merck Sharp & Dohme) contain recombinantly produced HPV16 and HPV18 VLP and are formulated with amorphous aluminium hydroxyphosphate sulphate salt. While *Gardasil*® also contains VLP of HPV6 and HPV11, *Gardasil*®*9* additionally comprises VLP of HPV31, HPV33, HPV45, HPV52, and HPV58. For these approved vaccines, specific protection against infection with oncogenic types HPV16 and HPV18 and associated precancerous lesions has been demonstrated in randomized clinical trials.

The synthetic DNA vaccine VGX-3100, targeting HPV16 and HPV18 E6 and E7 proteins, is currently investigated in clinical trials for the possible use in immunotherapy of HPV16 and HPV18 infection and precancerous lesions of the cervix (phase III) and vulva (phase II).

Several immunogenic fragments of the E6 and E7 proteins of HPV suitable for the treatment of HPV-related conditions are known in the art.

For instance, the safety and efficacy of the HPV vaccine DPX-E7, comprising the synthetic immunogenic fragment $E7_{11-19}$ of HPV16, is currently investigated in a phase Ib/II clinical trial as a possible treatment option for HPV or HPV-related head and neck, cervical or anal cancer.

WO 2015/086354 A2 relates to novel amino acid sequences of peptides derived from HPV16 that are able to bind to MHC class II, and elicit an immune response (columns 4/5, tables A and A1, SEQ ID Nos: 4 and 7). Furthermore, said peptides are suggested for the use in pharmaceutical products, such as vaccines.

WO 2018/085751 A1 relates to proteins or polypeptides derived from a HPV E6 protein or polypeptide (p.30,

table 1, SEQ ID NOs: 4, 5, 7) and a HPV E7 protein or polypeptide (p.30, table 1, SEQ ID NOs: 8, 10, 12). Moreover, a method of inhibiting HPV infection or HPV-associated cancer in a subject by administering a composition comprising the protein or peptide to the subject is described.

Since antigens, when injected alone, are usually ignored by antigen-presenting cells, cleared rapidly and, thus, do not induce an immune response, its administration in combination with a suitable adjuvant is required. Thus, there is a high demand on providing a carrier system that ensures adequate delivery of HPV-derived immunogenic fragments and, hence, proper presentation of those immunogenic fragments bound to MHC class I and MHC class II to CD4+ and CD8+ T cells.

Nanomedicine and nano-delivery systems are a relatively new but rapidly developing science where materials in the nanoscale range are employed to serve as means of diagnostic or therapeutic tools or to deliver therapeutic agents to specific targeted sites in the body. In particular nanoparticles are known for delivery of chemotherapeutic agents, biological agents, immunotherapeutic agents and as vaccines in the immunotherapy.

Functionalized nanoparticles are well-known as drug-delivery systems, e.g. as carrier for antigens. The literature describes in particular carrier systems in which the antigens are either encapsulated or bound to the surface of the nanoparticles.

WO 2006/037979 A2 describes gold nanoparticles (GNPs) comprising adjuvants and antigens, such as tumor and pathogen antigens, and their use in a range of applications such as for the treatment of cancer and infectious diseases. Also disclosed are immunogenic structures based on nanoparticles or antibodies with carbohydrate ligands, and their use for therapeutic and prophylactic purposes, and for the isolation and detection of antibodies directed against the carbohydrate structures.

WO 2013/034741 A1 and WO 2013/034726 A1 relate to nanoparticles having an epitopic peptide bound via a linker and which find use as vaccines, e.g. in the prophylactic or therapeutic treatment of a tumor in a mammalian subject.

WO 2011/154711 A1 describes glycated gold nanoparticles that act as carriers for delivery of peptides such as insulin.

WO 2010/006753 A2 discloses monodisperse nanoparticles of silicon dioxide with at least one antigen attached to their surface. The nanoparticles are used for the immunoprophylaxis or immunotherapy of cancer.

Although many nanoparticles have been investigated and developed in particular in the context of treating cancer there is still a high demand on providing substances with improved characteristics, in particular in terms of its adjuvant effects. Furthermore there is a need to provide nanoparticles as a flexible and convenient system to present agents of pharmacological relevance, such as HPV-derived immunogenic fragments, to the immune system of the body.

## SUMMARY OF THE INVENTION

It is thus objective of the present invention to provide improved nanoparticles and compositions as carrier for HPV-derived immunogenic fragments comprising them in particular for use in the immunoprophylaxis or immunotherapy. It is furthermore an objective of the invention to provide improved HPV vaccine compositions for use in the prevention and treatment of HPV-related conditions.

These objectives are solved by providing a composition comprising nanoparticles with silicon dioxide and functional groups on the surface, which are loaded with pharmaceutically acceptable compounds comprising HPV-derived immunogenic fragments. Alternatively, the nanoparticles are loaded with pharmaceutically acceptable compounds comprising HPV-derived immunogenic fragments and poly(I:C) or any derivatives thereof. The pharmaceutically acceptable compounds (poly(I:C) and HPV-derived immunogenic fragments) represent the payload of the nanoparticles. The nanoparticles have a particle size below 150 nm. The functional groups on the surface of the nanoparticles are suitable for carrying and/or stabilizing negative and positive charges of such compounds. The composition has a zeta potential of at least $\pm$ 15 mV.

Poly(I:C) is a synthetic dsRNA that can activate multiple elements of the host defense in a pattern that parallels that of a viral infection. Derivatives of poly(I:C) are for example polyI:polyC$_{12}$U and poly-ICLC.

It was found that compositions according to the invention show an immunomodulatory efficacy for immunoprophylaxis and immunotherapy of HPV-related conditions.

Moreover, with a composition according to the invention one fundamental problem of nanoparticles for drug delivery is overcome, which is the lack of stability of the colloidal suspension even under physiological conditions.

In aqueous systems, surface charges essentially ensure the stability of colloidal systems. These charges can be positive or negative. To substantially reduce or even avoid agglomeration of the particles it is important that there are enough functionalities of the same charge, which leads to electrostatic repulsion. If the charge of the colloidal carrier system is compensated by the adsorption of the oppositely charged molecules, agglomerates will be formed and the colloidal system collapses.

The agglomerates have significantly larger particle diameters, which jeopardizes an effective transport of these large particles within the body and leads to a less effective immunomodulatory efficacy.

The composition according to the invention with nanoparticles having a particle size of below 150 nm and a zeta potential of at least ± 15 mV ensures that the composition is sufficiently stable. In particular under physiological conditions, the nanoparticles dispersed therein can effectively be transported to their site of main activity within the body. The compositions according to the invention thus allow the improved transport of the nanoparticles into the lymphatic system, in particular from the administration site to the lymph nodes in which dendritic cells are located.

When administered by subcutaneous injection the particles according to the invention are too large to enter the blood circulation, hence their fast elimination as well as severe systemic adverse effects are essentially avoided. At the same time, they are small enough to penetrate lymphatic vessels and to enter naive dendritic cells, located in lymph nodes, by phagocytosis. The same applies when the particles are administered intradermally, intraperitoneally or intramuscularly. It is thus preferred to administer the particles parenterally with the exception of intravenous or intraarterial administration.

The inventors have found that the particles in the composition according to the invention can carry a high number of pharmaceutically acceptable compounds on the surface. The total amount of the compounds can be up to 5 % by weight with regard to the surface of the nanoparticle in $nm^2$ (surface loading density). The suitability of the nanoparticles to carry huge amounts of compounds is particularly important when high doses of such compounds (e.g. antigens or drug substances) are to be delivered.

The compositions according to the invention comprise nanoparticles with silicon dioxide and functional groups on the surface. The functional groups can be directly or indirectly connected to the surface of the nanoparticle. In a preferred embodiment of the invention the functional groups are connected to the surface of the nanoparticle via a linker (hereinafter linker compound L). The linker compound L can be connected to the surface of the nanoparticles by any way, in particular by covalent or adsorptive bond, most preferred by covalent bond.

In a particularly preferred embodiment the linker compound L comprises at least one functional group selected from the group consisting of carboxyl (-COOH) or carboxylate ($-COO^-$) group and/or at least one functional group selected from the group consisting of guanidino-group ($-NHC(=NH)NH_2$ or $-NHC(=NH_2^+)NH_2$) or amino-group ($-NH_2$ or $-NH_3^+$). Preferably, it comprises both, a carboxyl (-COOH) or carboxylate ($-COO^-$) group and at least one group selected from the group consisting of guanidino-group ($-NHC(=NH)NH_2$ or $-NHC(=NH_2^+)NH_2$) or amino-group ($-NH_2$ or - $NH_3^+$). Most preferred is that the linker compound L comprises a carboxyl (-COOH) or carboxylate ($-COO^-$) group and at least one guanidino-group ($-NHC(=NH)NH_2$ or - $NHC(=NH_2^+)NH_2$).

Such a linker compound L allows the adsorptive binding of anionic or cationic and also of nonpolar pharmaceutically acceptable compounds (such as e.g. hydrophobic peptides). Therewith the linker provides a simple coupling of HPV-derived immunogenic fragments and alternatively in combination with poly(I:C) to the surface of the nanoparticles.

## DETAILED DESCRIPTION OF THE INVENTION

The composition of the present invention contains nanoparticles which are loaded with one or more human papilloma virus (HPV)-derived immunogenic fragments or a variant thereof.

An "immunogenic fragment" according to the present invention is a peptide that is derived from a pathogenic antigen or epitope of a high-risk HPV genotype and able to induce an immune response in a patient. An immunogenic fragment of the invention is artificially produced.

A "peptide" according to the present invention is composed of any number of amino acids of any type, preferably naturally occurring amino acids, which preferably are linked by peptide bonds. The peptides can also exhibit posttranslational modifications, as e.g. phosphorylations, glycosylations, lipidations (like myristoylation or palmitoylation), citrullinations, acetylations (of lysine), hydroxylations (of proline or lysine).

An "antigen" according to the invention is a structure, which is capable of inducing a cellular or humoral immune response. Antigens are preferably proteinogenic, i.e. they are proteins, polypeptides or peptides. In principle, they can have any size, origin and molecular weight. They contain at least one antigenic determinant or an antigenic epitope.

In an alternative embodiment the antigen is a nucleic acids per se or is encoded by a nucleic acid, which, after transport into the nucleus of antigen-presenting cells, are translated into the proteinogenic antigen which is then presented by MHC molecules.

The nucleic acids can be single- and double-stranded DNA or RNA or oligonucleotides. The nucleic acids may also be a constituent of complexes with lipids, carbohydrates, proteins or peptides.

An "epitope" according to the invention, also known as antigenic determinant, is the part of an antigen that is recognized by the immune system, specifically by antibodies, B cells, or T cells. T cell epitopes are presented on the surface of an antigen-presenting cell, where they are bound to MHC molecules. In humans, professional antigen-presenting cells are specialized to present MHC class II peptides, whereas most nucleated somatic cells present MHC class I peptides. T cell epitopes presented by MHC class I molecules are typically peptides between 8 and 11 amino acids in length, whereas MHC class II molecules present longer peptides, 13-17 amino acids in length, and non-classical MHC molecules also present non-peptidic epitopes such as glycolipids.

A "variant" according to the present invention is an immunogenic fragment that is at least 75% identical to an immunogenic fragment derived from a pathogenic antigen or epitope of a high-risk HPV genotype.

The zeta potential of a nanoparticle is a commonly used parameter known to the skilled person to characterize the surface charge property of nanoparticles. It reflects the electrical potential of particles. To avoid agglomeration of the particles it is important that there are enough functionalities of the same charge which repel each other. Agglomerates cause the colloidal system to collapse. The resulting agglomerates have significantly larger particle diameters than the individual particles and therefore the desired transport mechanism via the fenestrated endothelium of the lymphatic vessels is no longer guaranteed. It is accepted as a measure for the stability of colloidal systems.

Current characterization methodologies are based on ensemble measurements (e.g. phase analysis light scattering, Doppler velocimetry, streaming potentiometry) that measure the average electrophoretic mobility of particles in suspension (Sci. Rep. 12 Dec. 2017; 7(1): 17479, PMCID: PMC5727177). It is particularly preferred to measure the zeta potential of the composition according to the invention with Dynamic Light Scattering (DLS) (ZetaSizer Nano ZS, Malvern Instruments, UK).

The zeta potential of the composition according to the present invention has a value of at least ± 15 mV, preferred ± 30 mV, more preferred ± 60 mV and most preferred between ± 25 and ± 40 mV. Therewith the composition, which is desirably a colloidal suspension, is stabilized, which means that the collapse of the system is essentially avoided.

The compositions according to the invention contain nanoparticles with a particle size below 150 nm, preferably 100 nm or less. More preferred are nanoparticles with a particle size of 50 nm or less, most preferred with a particle size between 20 and 30 nm. The particle size defined herein should be interpreted in such a way that a random distribution over the entire range is not present, but instead a defined particle size within the range is selected, of which the standard deviation is a maximum of 15%, preferably a maximum of 10%, wherein the standard deviation always relates to the local maximum in case of a bi- or multimodal distribution.

An indicator for the particle size of nanoparticles is the Z-average diameter. The Z-average diameter measured in dynamic light scattering is a parameter also known as the cumulant mean. It is the primary and most reliable parameter produced by the technique. The Z-average diameter is typically used in a quality control setting according to ISO 22412:2017. Dynamic light scattering techniques will give an intensity weighted distribution, where the contribution of each particle in the distribution relates to the intensity of light scattered by the particle. It is preferred to measure the intensity weighted distributions with a ZetaSizer Nano ZS (Malvern Instruments, UK).

In a preferred embodiment the Z-average diameter of the nanoparticles according to the invention is in the range of ≥5 and <150 nm, preferably ≥15 and ≤60nm, more preferably ≥20 and ≤40nm and still more preferably between 20 and 30nm, measured according to ISO 22412:2017.

The particle size of the nanoparticles and the stability of the composition according to the invention are furthermore confirmed by means of filtration with a sterile filter with maximum 0.2 μm pore size. However, this is practical only for nanoparticles with a diameter less than 50 nm.

The stability of the composition of the invention can be demonstrated by its polydispersity index (PDI). The PDI in general is an indicator for the uniformity of a system; in the context of the invention for the uniformity and stability of the colloidal nanoparticle suspension. The PDI reflects the nanoparticle size distribution. Samples with a wider range of particle sizes have higher PDI, while samples consisting of evenly sized particles have lower PDI. The skilled person is aware of methods and instruments for the measurement of the PDI, in particular a ZetaSizer Nano ZS (Malvern Instruments, UK).

A PDI greater than 0.7 indicates that the sample has a broad size distribution. A PDI below 0.1 is considered to be monodisperse. The various size distribution algorithms work with data that falls between these two extremes. The calculations for these parameters are defined in the ISO standard document 13321:1996 E and ISO 22412:2008.

The compositions according to the invention show a PDI between 0 and 0.32, preferably between 0.1 and 0.3, more preferably between 0.1 and 0.2, most preferred less than 0.1. The compositions according to the invention have a PDI less than 0.1 and are preferably monodisperse.

In a preferred embodiment the Z-average diameter of the nanoparticles according to the invention is in the range of ≥20 and ≤40nm and a PDI between 0.1 and 0.30 and a zeta potential between ± 20 and ± 40 mV.

In a more preferred embodiment the Z-average diameter of the nanoparticles according to the invention is in the range of 20 and 30nm and a PDI between 0.1 and 0.2 and a zeta potential between ± 25 and ± 40 mV.

In a most preferred embodiment the Z-average diameter of the nanoparticles according to the invention is in the range of 20 and 30nm and a PDI less than 0.1 and a zeta potential between ± 25 and ± 40 mV.

It is also possible to measure the particle size of the loaded and unloaded particles by TEM or SEM.

As used herein, the term "transmission electron microscopy (TEM)" refers to a microscopy technique whereby a beam of electrons is transmitted through an ultra thin specimen, interacting with the specimen as it passes through it. An image is formed from the electrons transmitted through the specimen, magnified and focused by an objective lens and appears on an imaging screen, a fluorescent screen in most TEMs, plus a monitor, or on a layer of photographic film, or to be detected by a sensor such as a CCD camera.

As used herein, the term "scanning electron microscope (SEM)" refers to a type of electron microscope that produces images of a sample by scanning the surface with a focused beam of electrons. The electrons interact with atoms in the sample, producing various signals that contain information about the surface topography and composition of the sample. The electron beam is scanned in a raster scan pattern, and the position of the beam is combined with the intensity of the detected signal to produce an image.

In **Fig. 1a** to **1d** show TEM images of the following $SiO_2$ particles:

**Fig. 1a** shows a TEM image of $SiO_2$ nanoparticles with a particle size of 68 nm
**Fig. 1b** shows a TEM image of $SiO_2$ nanoparticles with a particle size of 40 nm
**Fig. 1c** shows a TEM image of $SiO_2$ nanoparticles with a particle size of 25 nm
**Fig. 1d** shows a TEM image of $SiO_2$ nanoparticles with a particle size of 15 nm
**Fig. 2** shows a SEM of $SiO_2$ nanoparticles with a particle size of 150 nm.

In connection with the present invention, a "nanoparticle" is taken to mean a particulate binding matrix which has functionalities on its surface which function as recognition points for pharmaceutically acceptable compounds, e.g. antigens ultimately to be bound or adsorbed. The surface here encompasses all areas, i.e. besides the outer surface, also the inner surface of cavities (pores) in the particle. The functionalities may be directly or indirectly bound to the surface.

Any immunogenic fragment derived from a pathogenic antigen or epitope associated with any of the diseases or conditions provided herein can be used in the compositions and methods described herein. These include immunogenic fragments derived from a pathogenic antigen or epitope associated with cancer, infections or infectious disease. Preferred immunogenic fragments derived from a pathogenic antigen or epitope are those which are associated with a HPV infection, preferably caused by high-risk HPV genotypes.

So-called high-risk HPV genotypes are associated with the risk of developing a malignant condition, such as HPV types 16, 18, 31, 33, 35, 39, 45, 51, 52, 56, 58, 59, 66, 68, 73, and 82. Moreover, the HPV proteins E6 and E7 are especially regarded as being crucial for HPV immune escape and malignant progression.

Thus, in a preferred embodiment of the invention the immunogenic fragment bound to the nanoparticles is derived from a high-risk HPV genotype, preferably selected from HPV types 16, 18, 31, 33, 35, 39, 45, 51, 52, 56, 58, 59, 66, 68, 73, and 82.

In a more preferred embodiment of the present invention the immunogenic fragment is derived from the E6 and/or E7 proteins of HPV16 and/or HPV18 (SEQ ID NOs: 1-4), preferably selected from SEQ ID NOs: 5-257.

**Table 1: Sequence listing**

| SEQ ID NO | HPV type | protein / variant | amino acids | sequence |
|---|---|---|---|---|
| 1 | HPV16 | E6 | 1-158 | MHQKRTAMFQDPQERPRKLPQLCTELQTTI<br>HDIILECVYCKQQLLRREVYDFAFRDLCIVYR<br>DGNPYAVCDKCLKFYSKISEYRHYCYSLYG<br>TTLEQQYNKPLCDLLIRCINCQKPLCPEEKQ<br>RHLDKKQRFHNIRGRWTGRCMSCCRSSRT<br>RRETQL |
| 2 | HPV16 | E7 | 1-98 | MHGDTPTLHEYMLDLQPETTDLYCYEQLND<br>SSEEEDEIDGPAGQAEPDRAHYNIVTFCCK<br>CDSTLRLCVQSTHVDIRTLEDLLMGTLGIVC<br>PICSQKP |
| 3 | HPV18 | E6 | 1-158 | MARFEDPTRRPYKLPDLCTELNTSLQDIEIT<br>CVYCKTVLELTEVFEFAFKDLFVVYRDSIPH<br>AACHKCIDFYSRIRELRHYSDSVYGDTLEKL<br>TNTGLYNLLIRCLRCQKPLNPAEKLRHLNEK<br>RRFHNIAGHYRGQCHSCCNRARQERLQRR<br>RETQV |
| 4 | HPV18 | E7 | 1-105 | MHGPKATLQDIVLHLEPQNEIPVDLLCHEQL<br>SDSEEENDEIDGVNHQHLPARRAEPQRHTM<br>LCMCCKCEARIKLVVESSADDLRAFQQLFLN<br>TLSFVCPWCASQQ |
| 5 | HPV 16 | E6 | 1-30 | MHQKRTAMFQDPQERPRKLPQLCTELQTTI |

(continued)

| SEQ ID NO | HPV type | protein / variant | amino acids | sequence |
|---|---|---|---|---|
| 6 | HPV 16 | E6 | 7-15 | AMFQDPQER |
| 7 | HPV 16 | E6 | 8-18 | MFQDPQERPRK |
| 8 | HPV 16 | E6 | 9-18 | FQDPQERPRK |
| 9 | HPV 16 | E6 | 11-19 | DPQERPRKL |
| 10 | HPV 16 | E6 | 11-32 | DPQERPRKLPQLCTELQTTIHD |
| 11 | HPV 16 | E6 | 13-22 | QERPRKLPQL |
| 12 | HPV 16 | E6 | 15-22 | RPRKLPQL |
| 13 | HPV 16 | E6 | 15-23 | RPRKLPQLC |
| 14 | HPV 16 | E6 | 15-24 | RPRKLPQLCT |
| 15 | HPV 16 | E6 | 15-25 | RPRKLPQLCTE |
| 16 | HPV 16 | E6 | 18-26 | KLPQLCTEL |
| 17 | HPV 16 | E6 | 18-28 | KLPQLCTELQT |
| 18 | HPV 16 | E6 | 19-26 | LPQLCTEL |
| 19 | HPV 16 | E6 | 19-28 | LPQLCTELQT |
| 20 | HPV 16 | E6 | 23-31 | CTELQTTIH |
| 21 | HPV 16 | E6 | 25-33 | ELQTTIHDI |
| 22 | HPV 16 | E6 | 26-34 | LQTTIHDII |
| 23 | HPV 16 | E6 | 28-38 | TTIHDIILECV |
| 24 | HPV 16 | E6 | 29-37 | TIHDIILEC |
| 25 | HPV 16 | E6 | 29-38 | TIHDIILECV |
| 26 | HPV 16 | E6 | 29-39 | TIHDIILECVY |
| 27 | HPV 16 | E6 | 30-39 | IHDIILECVY |
| 28 | HPV 16 | E6 | 31-38 | HDIILECV |
| 29 | HPV 16 | E6 | 31-39 | HDIILECVY |
| 30 | HPV 16 | E6 | 31-41 | HDIILECVYCK |
| 31 | HPV 16 | E6 | 32-41 | DIILECVYCK |
| 32 | HPV 16 | E6 | 33-41 | IILECVYCK |
| 33 | HPV 16 | E6 | 34-41 | ILECVYCK |
| 34 | HPV 16 | E6 | 34-44 | ILECVYCKQQL |
| 35 | HPV 16 | E6 | 35-44 | LECVYCKQQL |
| 36 | HPV 16 | E6 | 37-46 | CVYCKQQLLR |
| 37 | HPV 16 | E6 | 37-54 | CVYCKQQLLRREVYDFAF |
| 38 | HPV 16 | E6 | 37-68 | CVYCKQQLLRREVYDFAFRDLCIVYRDGNP YA |
| 39 | HPV 16 | E6 | 38-45 | VYCKQQLL |
| 40 | HPV 16 | E6 | 38-46 | VYCKQQLLR |
| 41 | HPV 16 | E6 | 38-47 | VYCKQQLLRR |
| 42 | HPV 16 | E6 | 40-50 | CKQQLLRREVY |

(continued)

| SEQ ID NO | HPV type | protein / variant | amino acids | sequence |
|---|---|---|---|---|
| 43 | HPV 16 | E6 | 41-50 | KQQLLRREVY |
| 44 | HPV 16 | E6 | 42-50 | QQLLRREVY |
| 45 | HPV 16 | E6 | 42-52 | QQLLRREVYDF |
| 46 | HPV 16 | E6 | 43-52 | QLLRREVYDF |
| 47 | HPV 16 | E6 | 43-57 | QLLRREVYDFAFRDL |
| 48 | HPV 16 | E6 | 44-52 | LLRREVYDF |
| 49 | HPV 16 | E6 | 44-54 | LLRREVYDFAF |
| 50 | HPV 16 | E6 | 45-68 | LRREVYDFAFRDLCIVYRDGNPYA |
| 51 | HPV 16 | E6 | 47-54 | REVYDFAF |
| 52 | HPV 16 | E6 | 48-55 | EVYDFAFR |
| 53 | HPV 16 | E6 | 48-57 | EVYDFAFRDL |
| 54 | HPV 16 | E6 | 49-57 | VYDFAFRDL |
| 55 | HPV 16 | E6 | 49-58 | VYDFAFRDLC |
| 56 | HPV 16 | E6 | 49-59 | VYDFAFRDLCI |
| 57 | HPV 16 | E6 | 50-59 | YDFAFRDLCI |
| 58 | HPV 16 | E6 | 51-59 | DFAFRDLCI |
| 59 | HPV 16 | E6 | 52-60 | FAFRDLCIV |
| 60 | HPV 16 | E6 | 52-61 | FAFRDLCIVY |
| 61 | HPV 16 | E6 | 52-62 | FAFRDLCIVYR |
| 62 | HPV 16 | E6 | 53-61 | AFRDLCIVY |
| 63 | HPV 16 | E6 | 53-62 | AFRDLCIVYR |
| 64 | HPV 16 | E6 | 54-68 | FRDLCIVYRDGNPYA |
| 65 | HPV 16 | E6 | 55-86 | RDLCIVYRDGNPYAVCDKCLKFYSKISEYRH Y |
| 66 | HPV 16 | E6 | 57-67 | LCIVYRDGNPY |
| 67 | HPV 16 | E6 | 58-67 | CIVYRDGNPY |
| 68 | HPV 16 | E6 | 59-67 | IVYRDGNPY |
| 69 | HPV 16 | E6 | 59-68 | IVYRDGNPYA |
| 70 | HPV 16 | E6 | 59-69 | IVYRDGNPYAV |
| 71 | HPV 16 | E6 | 60-67 | VYRDGNPY |
| 72 | HPV 16 | E6 | 60-69 | VYRDGNPYAV |
| 73 | HPV 16 | E6 | 61-82 | YRDGNPYAVCDKCLKFYSKISE |
| 74 | HPV 16 | E6 | 65-75 | NPYAVCDKCLK |
| 75 | HPV 16 | E6 | 66-74 | PYAVCDKCL |
| 76 | HPV 16 | E6 | 66-75 | PYAVCDKCLK |
| 77 | HPV 16 | E6 | 66-76 | PYAVCDKCLKF |
| 78 | HPV 16 | E6 | 67-75 | YAVCDKCLK |
| 79 | HPV 16 | E6 | 67-76 | YAVCDKCLKF |

(continued)

| SEQ ID NO | HPV type | protein / variant | amino acids | sequence |
|---|---|---|---|---|
| **80** | HPV 16 | E6 | 67-77 | YAVCDKCLKFY |
| **81** | HPV 16 | E6 | 68-75 | AVCDKCLK |
| **82** | HPV 16 | E6 | 68-76 | AVCDKCLKF |
| **83** | HPV 16 | E6 | 68-77 | AVCDKCLKFY |
| **84** | HPV 16 | E6 | 68-78 | AVCDKCLKFYS |
| **85** | HPV 16 | E6 | 69-79 | VCDKCLKFYSK |
| **86** | HPV 16 | E6 | 71-78 | DKCLKFYS |
| **87** | HPV 16 | E6 | 72-80 | KCLKFYSKI |
| **88** | HPV 16 | E6 | 73-83 | CLKFYSKISEY |
| **89** | HPV 16 | E6 | 73-105 | CLKFYSKISEYRHYCYSLYGTTLEQQYNKPL CD |
| **90** | HPV 16 | E6 | 74-83 | LKFYSKISEY |
| **91** | HPV 16 | E6 | 74-88 | LKFYSKISEYRHYCY |
| **92** | HPV 16 | E6 | 75-83 | KFYSKISEY |
| **93** | HPV 16 | E6 | 75-84 | KFYSKISEYR |
| **94** | HPV 16 | E6 | 75-85 | KFYSKISEYRH |
| **95** | HPV 16 | E6 | 76-83 | FYSKISEY |
| **96** | HPV 16 | E6 | 76-85 | FYSKISEYRH |
| **97** | HPV 16 | E6 | 76-86 | FYSKISEYRHY |
| **98** | HPV 16 | E6 | 77-86 | YSKISEYRHY |
| **99** | HPV 16 | E6 | 78-86 | SKISEYRHY |
| **100** | HPV 16 | E6 | 78-88 | SKISEYRHYCY |
| **101** | HPV 16 | E6 | 79-86 | KISEYRHY |
| **102** | HPV 16 | E6 | 79-87 | KISEYRHYC |
| **103** | HPV 16 | E6 | 79-88 | KISEYRHYCY |
| **104** | HPV 16 | E6 | 80-88 | ISEYRHYCY |
| **105** | HPV 16 | E6 | 81-88 | SEYRHYCY |
| **106** | HPV 16 | E6 | 81-90 | SEYRHYCYSL |
| **107** | HPV 16 | E6 | 81-91 | SEYRHYCYSL Y |
| **108** | HPV 16 | E6 | 82-90 | EYRHYCYSL |
| **109** | HPV 16 | E6 | 82-91 | EYRHYCYSL Y |
| **110** | HPV 16 | E6 | 83-90 | YRHYCYSL |
| **111** | HPV 16 | E6 | 83-91 | YRHYCYSL Y |
| **112** | HPV 16 | E6 | 84-91 | RHYCYSLY |
| **113** | HPV 16 | E6 | 84-94 | RHYCYSLYGTT |
| **114** | HPV 16 | E6 | 85-95 | HYCYSLYGTTL |
| **115** | HPV 16 | E6 | 86-96 | YCYSLYGTTLE |
| **116** | HPV 16 | E6 | 87-95 | CYSLYGTTL |

(continued)

| SEQ ID NO | HPV type | protein / variant | amino acids | sequence |
|---|---|---|---|---|
| **117** | HPV 16 | E6 | 87-96 | CYSLYGTTLE |
| **118** | HPV 16 | E6 | 88-95 | YSLYGTTL |
| **119** | HPV 16 | E6 | 89-99 | SLYGTTLEQQY |
| **120** | HPV 16 | E6 | 90-99 | LYGTTLEQQY |
| **121** | HPV 16 | E6 | 91-100 | YGTTLEQQYN |
| **122** | HPV 16 | E6 | 91-101 | YGTTLEQQYNK |
| **123** | HPV 16 | E6 | 91-112 | YGTTLEQQYNKPLCDLLIRCIN |
| **124** | HPV 16 | E6 | 92-99 | GTTLEQQY |
| **125** | HPV 16 | E6 | 92-101 | GTTLEQQYNK |
| **126** | HPV 16 | E6 | 93-101 | TTLEQQYNK |
| **127** | HPV 16 | E6 | 94-101 | TLEQQYNK |
| **128** | HPV 16 | E6 | 95-103 | LEQQYNKPL |
| **129** | HPV 16 | E6 | 97-106 | QQYNKPLCDL |
| **130** | HPV 16 | E6 | 98-106 | QYNKPLCDL |
| **131** | HPV 16 | E6 | 98-107 | QYNKPLCDLL |
| **132** | HPV 16 | E6 | 98-108 | QYNKPLCDLLI |
| **133** | HPV 16 | E6 | 99-106 | YNKPLCDL |
| **134** | HPV 16 | E6 | 101-108 | KPLCDLLI |
| **135** | HPV 16 | E6 | 101-111 | KPLCDLLIRCI |
| **136** | HPV 16 | E6 | 101-122 | KPLCDLLIRCINCQKPLCPEEK |
| **137** | HPV 16 | E6 | 105-115 | DLLIRCINCQK |
| **138** | HPV 16 | E6 | 106-115 | LLIRCINCQK |
| **139** | HPV 16 | E6 | 107-115 | LIRCINCQK |
| **140** | HPV 16 | E6 | 107-117 | LIRCINCQKPL |
| **141** | HPV 16 | E6 | 109-119 | RCINCQKPLCP |
| **142** | HPV 16 | E6 | 113-121 | CQKPLCPEE |
| **143** | HPV 16 | E6 | 118-126 | CPEEKQRHL |
| **144** | HPV 16 | E6 | 121-142 | EKQRHLDKKQRFHNIRGRWTGR |
| **145** | HPV 16 | E6 | 122-132 | KQRHLDKKQRF |
| **146** | HPV 16 | E6 | 125-133 | HLDKKQRFH |
| **147** | HPV 16 | E6 | 125-135 | HLDKKQRFHNI |
| **148** | HPV 16 | E6 | 127-134 | DKKQRFHN |
| **149** | HPV 16 | E6 | 127-135 | DKKQRFHNI |
| **150** | HPV 16 | E6 | 127-141 | DKKQRFHNIRGRWTG |
| **151** | HPV 16 | E6 | 128-135 | KKQRFHNI |
| **152** | HPV 16 | E6 | 128-136 | KKQRFHNIR |
| **153** | HPV 16 | E6 | 129-138 | KQRFHNIRGR |
| **154** | HPV 16 | E6 | 129-139 | KQRFHNIRGRW |

(continued)

| SEQ ID NO | HPV type | protein / variant | amino acids | sequence |
|---|---|---|---|---|
| **155** | HPV 16 | E6 | 131-139 | RFHNIRGRW |
| **156** | HPV 16 | E6 | 134-144 | NIRGRWTGRCM |
| **157** | HPV 16 | E6 | 136-144 | RGRWTGRCM |
| **158** | HPV 16 | E6 | 137-146 | GRWTGRCMSC |
| **159** | HPV 16 | E6 | 139-148 | WTGRCMSCCR |
| **160** | HPV 16 | E6 | 142-151 | RCMSCCRSSR |
| **161** | HPV 16 | E6 | 148-158 | RSSRTRRETQL |
| **162** | HPV 16 | E6 | 149-158 | SSRTRRETQL |
| **163** | HPV 16 | E6 | 151-158 | RTRRETQL |
| **164** | HPV 16 | E7 | 1-12 | MHGDTPTLHEYM |
| **165** | HPV 16 | E7 | 2-11 | HGDTPTLHEY |
| **166** | HPV 16 | E7 | 4-11 | DTPTLHEY |
| **167** | HPV 16 | E7 | 5-12 | TPTLHEYM |
| **168** | HPV 16 | E7 | 5-13 | TPTLHEYML |
| **169** | HPV 16 | E7 | 5-18 | TPTLHEYMLDLQPE |
| **170** | HPV 16 | E7 | 7-15 | TLHEYMLDL |
| **171** | HPV 16 | E7 | 7-17 | TLHEYMLDLQP |
| **172** | HPV 16 | E7 | 7-27 | TLHEYMLDLQPETTDLYCYEQ |
| **173** | HPV 16 | E7 | 11-18 | YMLDLQPE |
| **174** | HPV 16 | E7 | 11-19 | YMLDLQPET |
| **175** | HPV 16 | E7 | 11-20 | YMLDLQPETT |
| **176** | HPV 16 | E7 | 11-21 | YMLDLQPETTD |
| **177** | HPV 16 | E7 | 11-26 | YMLDLQPETTDLYCYE |
| **178** | HPV 16 | E7 | 12-19 | MLDLQPET |
| **179** | HPV 16 | E7 | 12-20 | MLDLQPETT |
| **180** | HPV 16 | E7 | 12-26 | MLDLQPETTDLYCYE |
| **181** | HPV 16 | E7 | 14-23 | DLQPETTDLY |
| **182** | HPV 16 | E7 | 15-23 | LQPETTDLY |
| **183** | HPV 16 | E7 | 15-24 | LQPETTDLYC |
| **184** | HPV 16 | E7 | 15-25 | LQPETTDLYCY |
| **185** | HPV 16 | E7 | 17-38 | PETTDLYCYEQLNDSSEEEDEI |
| **186** | HPV 16 | E7 | 18-25 | ETTDLYCY |
| **187** | HPV 16 | E7 | 18-26 | ETTDLYCYE |
| **188** | HPV 16 | E7 | 19-27 | TTDLYCYEQ |
| **189** | HPV 16 | E7 | 19-29 | TTDLYCYEQLN |
| **190** | HPV 16 | E7 | 21-40 | DLYCYEQLNDSSEEEDEIDG |
| **191** | HPV 16 | E7 | 21-42 | DLYCYEQLNDSSEEEDEIDGPA |
| **192** | HPV 16 | E7 | 35-50 | EDEIDGPAGQAEPDRA |

(continued)

| SEQ ID NO | HPV type | protein / variant | amino acids | sequence |
|---|---|---|---|---|
| **193** | HPV 16 | E7 | 42-52 | AGQAEPDRAHY |
| **194** | HPV 16 | E7 | 43-51 | GQAEPDRAH |
| **195** | HPV 16 | E7 | 43-52 | GQAEPDRAHY |
| **196** | HPV 16 | E7 | 43-53 | GQAEPDRAHYN |
| **197** | HPV 16 | E7 | 43-77 | GQAEPDRAHYN IVTFCCKCDSTLRLCVQST HVDIR |
| **198** | HPV 16 | E7 | 44-52 | QAEPDRAHY |
| **199** | HPV 16 | E7 | 44-62 | QAEPDRAHYNIVTFCCKCD |
| **200** | HPV 16 | E7 | 46-55 | EPDRAHYNIV |
| **201** | HPV 16 | E7 | 47-57 | PDRAHYNIVTF |
| **202** | HPV 16 | E7 | 48-57 | DRAHYNIVTF |
| **203** | HPV 16 | E7 | 48-62 | DRAHYNIVTFCCKCD |
| **204** | HPV 16 | E7 | 49-57 | RAHYNIVTF |
| **205** | HPV 16 | E7 | 50-57 | AHYNIVTF |
| **206** | HPV 16 | E7 | 50-60 | AHYNIVTFCCK |
| **207** | HPV 16 | E7 | 50-62 | AHYNIVTFCCKCD |
| **208** | HPV 16 | E7 | 51-58 | HYNIVTFC |
| **209** | HPV 16 | E7 | 51-59 | HYNIVTFCC |
| **210** | HPV 16 | E7 | 51-72 | HYNIVTFCCKCDSTLRLCVQST |
| **211** | HPV 16 | E7 | 52-60 | YNIVTFCCK |
| **212** | HPV 16 | E7 | 53-60 | NIVTFCCK |
| **213** | HPV 16 | E7 | 56-65 | TFCCKCDSTL |
| **214** | HPV 16 | E7 | 62-75 | DSTLRLCVQSTHVD |
| **215** | HPV 16 | E7 | 64-78 | TLRLCVQSTHVDIRT |
| **216** | HPV 16 | E7 | 66-74 | RLCVQSTHV |
| **217** | HPV 16 | E7 | 67-76 | LCVQSTHVDI |
| **218** | HPV 16 | E7 | 67-98 | LCVQSTHVDIRTLEDLLMGTLGIVCPICSQKP |
| **219** | HPV 16 | E7 | 69-76 | VQSTHVDI |
| **220** | HPV 16 | E7 | 69-86 | VQSTHVDIRTLEDLLMGT |
| **221** | HPV 16 | E7 | 71-85 | STHVDIRTLEDLLMG |
| **222** | HPV 16 | E7 | 72-97 | THVDIRTLEDLLMGTLGIVCPICSQK |
| **223** | HPV 16 | E7 | 73-84 | HVDIRTLEDLLM |
| **224** | HPV 16 | E7 | 76-86 | IRTLEDLLMGT |
| **225** | HPV 16 | E7 | 77-86 | RTLEDLLMGT |
| **226** | HPV 16 | E7 | 77-87 | RTLEDLLMGTL |
| **227** | HPV 16 | E7 | 78-86 | TLEDLLMGT |
| **228** | HPV 16 | E7 | 79-87 | LEDLLMGTL |
| **229** | HPV 16 | E7 | 80-90 | EDLLMGTLGIV |
| **230** | HPV 16 | E7 | 81-90 | DLLMGTLGIV |

(continued)

| SEQ ID NO | HPV type | protein / variant | amino acids | sequence |
|---|---|---|---|---|
| **231** | HPV 16 | E7 | 81-91 | DLLMGTLGIVC |
| **232** | HPV 16 | E7 | 82-89 | LLMGTLGI |
| **233** | HPV 16 | E7 | 82-90 | LLMGTLGIV |
| **234** | HPV 16 | E7 | 82-91 | LLMGTLGIVC |
| **235** | HPV 16 | E7 | 82-92 | LLMGTLGIVCP |
| **236** | HPV 16 | E7 | 83-93 | LMGTLGIVCPI |
| **237** | HPV 16 | E7 | 84-93 | MGTLGIVCPI |
| **238** | HPV 16 | E7 | 85-93 | GTLGIVCPI |
| **239** | HPV 16 | E7 | 86-93 | TLGIVCPI |
| **240** | HPV 16 | E7 | 86-94 | TLGIVCPIC |
| **241** | HPV 16 | E7 | 87-97 | LGIVCPICSQK |
| **242** | HPV 16 | E7 | 88-97 | GIVCPICSQK |
| **243** | HPV 16 | E7 | 89-97 | IVCPICSQK |
| **244** | HPV 16 | E7 | 98 | VCPICSQKP |
| **245** | HPV18 | E6 | 13-21 | KLPDLCTEL |
| **246** | HPV18 | E6 | 36-43 | KTVLELTE |
| **247** | HPV18 | E6 | 40-48 | ELTEVFEFA |
| **248** | HPV18 | E6 | 43-57 | EVFEFAFKDLFWYR |
| **249** | HPV18 | E6 | 51-72 | DLFVVYRDSIPHAACHKCIDFY |
| **250** | HPV18 | E6 | 71-92 | FYSRIRELRHYSDSVYGDTLEK |
| **251** | HPV18 | E6 | 125-135 | RRFHNIAGHYR |
| **252** | HPV18 | E7 | 1-32 | MHGPKATLQDIVLHLEPQNEIPVDLLCHEQL S |
| **253** | HPV18 | E7 | 5-16 | KATLQDIVLHLE |
| **254** | HPV18 | E7 | 7-15 | TLQDIVLHL |
| **255** | HPV18 | E7 | 21-42 | IPVDLLCHEQLSDSEEENDEID |
| **256** | HPV18 | E7 | 86-94 | FQQLFLNTL |
| **257** | HPV18 | E7 | 88-97 | QLFLNTLSFV |
| **258** | HPV16 | E6V R17I | 9-17 | FQDPQERPI |
| **259** | HPV16 | E6V R17I | 9-19 | FQDPQERPIKL |
| **260** | HPV16 | E6V R17T | 15-25 | RPTKLPQLCTE |
| **261** | HPV16 | E6V Q21 D | 18-28 | KLPDLCTELQT |
| **262** | HPV16 | E6V D32E | 25-33 | ELQTTIHEI |
| **263** | HPV16 | E6V D32E | 26-34 | LQTTIHEII |
| **264** | HPV16 | E6V D32E | 28-38 | TTIHEIILECV |
| **265** | HPV16 | E6V D32E, I34R | 28-38 | TTIHEIRLECV |
| **266** | HPV16 | E6V D32E | 29-38 | TIHEIILECV |
| **267** | HPV16 | E6V D32E, I34R | 29-38 | TIHEIRLECV |

(continued)

| SEQ ID NO | HPV type | protein / variant | amino acids | sequence |
|---|---|---|---|---|
| **268** | HPV16 | E6V D32E | 31-41 | HEIILECVYCK |
| **269** | HPV16 | E6V D32E, I34R | 31-41 | HEIRLECVYCK |
| **270** | HPV16 | E6V D32E | 32-41 | EIILECVYCK |
| **271** | HPV16 | E6V I34R | 34-41 | RLECVYCK |
| **272** | HPV16 | E6V A68G | 60-69 | VYRDGNPYGV |
| **273** | HPV16 | E6V H85Y | 79-89 | KISEYRYYCYS |
| **274** | HPV16 | E6V H85Y | 81-90 | SEYRYYCYSL |
| **275** | HPV16 | E6V H85Y, | 81-90 | SEYRYYCYSV |
| | | L90V | | |
| **276** | HPV16 | E6V L90V | 81-91 | SEYRHYCYSVY |
| **277** | HPV16 | E6V L90V | 82-90 | EYRHYCYSV |
| **278** | HPV16 | E6V H85Y | 83-90 | YRYYCYSL |
| **279** | HPV16 | E6V L90V | 83-90 | YRHYCYSV |
| **280** | HPV16 | E6V H85Y, L90V | 83-90 | YRYYCYSV |
| **281** | HPV16 | E6V L90V | 83-91 | YRHYCYSVY |
| **282** | HPV16 | E6V L90V | 84-91 | RHYCYSVY |
| **283** | HPV16 | E6V H85Y | 84-93 | RYYCYSLYGT |
| **284** | HPV16 | E6V L90V | 84-94 | RHYCYSVYGTT |
| **285** | HPV16 | E6V L90V | 86-95 | YCYSVYGTTL |
| **286** | HPV16 | E6V L90V | 87-95 | CYSVYGTTL |
| **287** | HPV16 | E6V L90V | 87-96 | CYSVYGTTLE |
| **288** | HPV16 | E6V L90V | 88-95 | YSVYGTTL |
| **289** | HPV16 | E6V L90V | 89-99 | SVYGTTLEQQY |
| **290** | HPV16 | E6V L90V | 90-97 | VYGTTLEQ |
| **291** | HPV16 | E6V L90V | 90-99 | VYGTTLEQQY |
| **292** | HPV16 | E7V L28F | 21-28 | DLYCYEQF |
| **293** | HPV16 | E7V S63F | 56-63 | TFCCKCDF |
| **294** | HPV16 | E7V S63F | 56-65 | TFCCKCDFTL |
| **295** | HPV16 | E7V S63F | 56-66 | TFCCKCDFTLR |

In a further embodiment of the present invention the immunogenic fragment is any immunogenic fragment that is at least 75% identical to an immunogenic fragment derived from the E6 and/or E7 proteins of HPV16 and/or HPV18. Examples of variants of the immunogenic fragment derived from the E6 (E6V) and/or E7 (E7V) proteins of HPV16 and/or HPV18 are shown in Table 1 (SEQ ID NOs: 258-295).

Interaction of CD8+ and CD4+ T cells with antigen-presenting MHC class I and MHC class II, respectively, plays a pivotal role in the induction and maintenance of cytotoxic T cell responses, and are also important for direct anti-tumor immunity. The term "cytotoxic T cell response" refers to the specific recognition of pathogenic antigens, epitopes or immunogenic fragments and the induction of apoptosis in infected cells by cytotoxic T cells.

The composition according to the present invention is characterized in that the immunogenic fragment is able to bind to MHC class I and/or MHC class II.

In a preferred embodiment of the invention the immunogenic fragment is able to induce a cytotoxic T cell response.

Immunogenic fragments derived from the E6 and/or E7 proteins of HPV16 and/or HPV18, respectively, with the ability of binding to MHC class I and MHC class II, respectively, thereby inducing a cytotoxic T cell response, can be identified, for instance, by mass spectrometry (MS) of human HPV16+ and HPV18+ tumor cells, Enzyme Linked Immuno Spot (ELISpot) assay, *in vitro* cytotoxicity assay, anti-tumor activity measurements in MHC-humanized animal models, and bioinformatic approaches, such as MHC class I binding prediction tools (e.g., MHCflurry, MSIntrinsic, MixMHCPred, NetMHC, NetMHCpan, NetMHCcons).

In a preferred embodiment of the invention the immunogenic fragment comprises 3 to 35 amino acids. In particular, an immunogenic fragment comprises at least 3 amino acids, preferably at least 5, at least 6, at least 7, or at least 8 amino acids. In a preferred embodiment the immunogenic fragment does not exceed a length of 35 amino acids, preferably, it does not exceed a length of 25 amino acids, more preferably, it does not exceed a length of 20 amino acids. In a most preferred embodiment the immunogenic fragment has a length from 8 to 20 amino acids.

In an alternatively preferred embodiment of the invention the nanoparticles are loaded with pharmaceutically acceptable compounds comprising one or more HPV-derived immunogenic fragments and polyinosinic:polycytidylic acid (poly(I:C)) or any derivatives thereof.

In a preferred embodiment of the invention the derivatives of poly(I:C) are poly(I:C) LMW (Low Molecular Weight Poly(I:C)) with an average size from 0.2 kb to 1 kb, poly(I:C) HMW (High Molecular Weight Poly(I:C)) with an average size from 1.5 kb to 8 kb, polyI:poly$C_{12}$U.

According to the present invention, the nanoparticle(s) comprise silicon dioxide ($SiO_2$), optional in mixture with another material. The material thus can also be admixed with further components, where silicon dioxide typically has the highest proportion in a multicomponent system. The nanoparticles of the invention can comprise at least 80% of silicon dioxide, preferably at least 90%.

In a particularly preferred embodiment of the nanoparticles according to the invention, the material comprises silicon dioxide which is essentially pure, i.e. only comprises the impurities to be expected in the course of the preparation process. In a more preferred embodiment of the invention, the nanoparticle material consists of silicon dioxide.

Examples of other materials are metals, a metal chalcogenide, a magnetic material, a magnetic alloy, a semiconductor material, metal oxides, polymers, organosilanes, other ceramics or glass. The metal is selected from the group Au, Ag, Cu, Pt, Pd, Fe, Co, Gd, Ru, Rh and Zn, or any combination thereof.

In a further embodiment of the present invention the nanoparticles have a coating which comprises silicon dioxide. The core may comprise any other material such as metals, polymers or ferromagnetic metals such as $Fe_2O_3$ or $Fe_3O_4$. The core can even be devoid of silicon dioxide.

Silicon dioxide nanoparticles according to the invention have been described in, for example, WO 2010/006753 A2, which is expressly incorporated herein by reference.

The silicon dioxide nanoparticles can be prepared using, inter alia, the classical Stöber synthesis, in which monodisperse nanoscale silicon dioxide of defined size can be prepared by hydrolysis of tetraethoxysilane (TEOS) in aqueous-alcoholic-ammonia medium (J. Colloid Interface Sci. 1968, 26, 62).

The process of the preparation of silicon dioxide nanoparticles is described in detail in EP 0216 278 B1 and WO 2005/085135 A1, and consequently these documents are incorporated in their totality into the disclosure content of the present invention by way of reference. At least one amine is preferably used in the medium.

The silicon dioxide matrix of the nanoparticles according to the invention can be either porous or non-porous. The porosity is essentially dependent on the production process. In the synthesis in accordance with EP 0 216 278 B1, non-porous particles, in particular, are obtained.

According to the present invention the nanoparticles contain functional groups on their surface. These functional groups are capable to carry and/or stabilize both negative and positive charges. These charges may belong to pharmaceutically acceptable compounds such as (HPV)-derived immunogenic fragment and TLR agonists such as poly(I:C) and its derivatives.

Preferred TLR agonists are TLR3 agonists selected from the group consisting of poly(I:C) (dsRNA, TLR3 agonist, as well as RIG-I agonist), polyI:poly$C_{12}$U (dsRNA, TLR3 agonist, trade name Ampligen®, INN: Rintatolimod) and NAB2 (Nucleic acid band 2, dsRNA isolated from yeast and identified as an agonist of the pattern-recognition receptors TLR3 and MDA-5) . More preferred is poly(I:C) LMW (Low Molecular Weight Poly(I:C) with an average size from 0.2 kb to 1 kb), poly(I:C) HMW (High Molecular Weight Poly(I:C) with an average size from 1.5 kb to 8 kb), polyI:poly$C_{12}$U or poly-ICLC. Most preferred is poly(I:C) LMW.

Functional groups which are capable to carry and/or stabilize both negative and positive charges are for example -SH, -COOH, $-NH_2$, -guanidino-group ($-NHC(=NH))NH_2$), $-PO_3H_2$, $-PO_2CH_3H$, $-SO_3H$, -OH, $-NR_3^+X^-$. Preferred functional groups are -COOH, -guanidino-group ($-NHC(=NH))NH_2$) and $-NH_2$. The functional groups may also be present in their salt form.

Compositions according to the invention comprise nanoparticles which have a surface loading density up to 0.5, preferably between 0.01 and 0.5, more preferred between 0.03 and 0.4, most preferred between 0.05 to 0.3, in relation to the total number of the pharmaceutically acceptable compounds with regard to the surface of the nanoparticle

in $nm^2$ [molecules/$nm^2$].

This surface loading density is calculated by assuming a perfect sphere and by the molar loading per particle. For example: a spherical particle with a diameter of 25 nm has a surface of 1964 $nm^2$ ($A=\pi*d^2$) and a weight of 1.64E-8 ng (with an assumed density of 2000 $kg/m^3$ for amorphous silica). The loading of such a particle with 5 % by weight with the peptide KKKV-Cit-YMLDLQPET (M=1,910.31 g/mol) equals to 4.283E-22 mol. This value multiplied by the Avogadro constant results in 258 single molecules of KKKV-Cit-YMLDLQPET attached to one particle with 1964 $nm^2$. This corresponds to a surface loading density of 0.13 molecules/$nm^2$.

The compositions according to the invention are formulated to have a pH between 6.0 and 8.0, preferably between 6.5 and 7.8 and more preferably between 6.8 and 7.5, even more preferred between 7.2 and 7.4. The pH of a composition can be maintained by the use of a buffer such as acetate, citrate, phosphate, succinate, TRIS (tris(hydroxymethyl)aminomethane) or histidine, typically employed in the range from about 1 mM to 50 mM. The pH of compositions can otherwise be adjusted by using physiologically acceptable acids or bases.

In one embodiment of the present invention the functional groups are $-PO_3H$ groups. Phosphorylated nanoparticles according to the invention could be for example prepared by reaction of (diethylphosphatoethyl)triethoxysilane with silica nanoparticles under addition of ammonia.

In a preferred embodiment of the present invention the functional groups are connected to the nanoparticle via a linker L. The linker can be connected to the nanoparticles e.g. by way of a covalent or adsorptive bond.

In a preferred embodiment of the present invention the linker compound L comprises at least one carboxyl (-COOH) or carboxylate ($-COO^-$) group as functional group.

In a more preferred embodiment such linker further comprises at least one guanidino-group ($-NHC(=NH)NH_2$ or $-NHC(=NH_2^+)NH_2$) or amino-group ($-NH_2$ or $-NH_3^+$) as functional group.

In a most preferred embodiment of the present invention the linker compound L contains at least a structural unit of the general formula (I)

$$*\text{-}(\text{-}O)_3Si\text{-}(CH_2)_nCH(COOX)\text{-}(CH_2)_p\text{-}C(O)\text{-}NH\text{-}CH(COOX)\text{-}(CH_2)_q\text{-}Y \qquad (I)$$

wherein

| | |
|---|---|
| X | is independently from each other H or a negative charge, |
| Y | is independently from each other $-NHC(=NH)NH_2$, $-NHC(=NH_2^+)NH_2$, $-NH_2$ or $-NH_3^+$, |
| n, p and q | are independently from each other 0 or a number from 1 to 25; and |
| *- | is the connection point to the nanoparticle. |

In a preferred embodiment the linker compounds L contain at least a structural unit of formula (I) wherein n is 3, p is 1 and q is 4 and Y is independently from each other a $-NH_2$ or $-NH_3^+$ group.

In a particularly preferred embodiment the linker compounds L contain at least a structural unit of formula (I) wherein n is 3, p is 1 and q is 3 and Y is independently from each other a $-NHC(=NH)NH_2$ or $-NHC(=NH_2^+)NH_2$ group.

Another aspect of the present invention is to provide a process of preparation of nanoparticles according to the invention wherein

- in a first step (i) hydrolytic polycondensation of tetraalkoxysilanes and/or organotrialkoxysilanes in a medium which comprises water, at least one solubilizer and at least one amine or ammonia take place, where firstly a sol of primary particles is produced, and the resultant nanoparticles are subsequently brought to the desired particle size in a range from 5 to 150 nm in such a way that further nucleation is limited by continuous metering-in of corresponding silane in a controlled manner corresponding to the extent of reaction, and
- in a second step (ii) the nanoparticles from step (i) are reacted with [(3-triethoxysilyl)propyl]succinic anhydride, which in a simultaneous reaction forms an amide with L-arginine or L-lysine.

It is preferred to use in step (ii) of the process L-arginine.

In an alternative embodiment the linker compound L could be also obtained by the reaction of L-arginine with N-(3-triethoxysilylpropyl)maleimide. The reaction is carried out preferably at pH values above 8.

In one embodiment of the present invention it also possible to produce the linker compound L by reaction of [(3-triethoxysilyl)propyl]succinic anhydride with agmatine, histamine, cadaverine or spermidine.

A further embodiment of the present invention are nanoparticles comprising a silicon dioxide based surface with a linker compound L covalently or adsorptive bonded to it, wherein the Linker L contains at least one guanidino-group ($-NHC(=NH)NH_2$ or - $NHC(=NH_2^+)NH_2$) or amino-group ($-NH_2$ or $-NH_3^+$) as a functional group. In a preferred embodiment the Linker L contains at least one guanidino-group ($-NHC(=NH)NH_2$ or - $NHC(=NH_2^+)NH_2$) or amino-group ($-NH_2$ or $-NH_3^+$) and at least one carboxyl (-COOH) or carboxylate ($-COO^-$) group as a functional group. In a more

preferred embodiment the Linker L contains at least one guanidino-group (-NHC(=NH)$NH_2$ or - NHC(=$NH_2^+$)$NH_2$) and at least one carboxyl (--COOH) or carboxylate (-$COO^-$) group as a functional group.

One embodiment of the nanoparticles according to the invention is illustrated in **Fig. 3.**

**Fig. 3** shows the schematic drawing of the cross section of a nanoparticle according to the invention. A represents the surface functionalization with the linker compound L, B represents the amorphous $SiO_2$ shell and C represents the core material, which could be void, water or any other material as well as amorphous $SiO_2$. The diameter d1 is between 0 and 149 nm and d2 is between 10 and 150 nm.

In one embodiment of the invention the pharmaceutically acceptable compound is conjugated to the nanoparticle by adsorptive or covalent attachment. The adsorptive attachment is preferred.

A further object of the present invention are nanoparticles having silicon dioxide and functional groups on the surface and a particle size below 150 nm, comprising a linker L which is covalently or adsorptive bonded to it, wherein the Linker L comprises at least one guanidino-group (-NHC(=NH)$NH_2$ or -NHC(=$NH_2^+$)$NH_2$) or amino-group (-$NH_2$ or -$NH_3^+$) group as a functional group.

A main advantage of the adsorptive binding of the pharmaceutically acceptable compound is that, in contrast to most covalent conjugations, no by-products are formed or remain in the "reaction" mixture. In case of a covalent attachment it could become necessary to chemically modify the molecule which should be attached by adding reactive functional groups to the molecule. This is a fundamental intervention in the structure of the peptide/antigen and constitutes a complex chemical modification in case of RNA- or DNA-based antigens or adjuvants. In addition, a covalent linkage of a known compound that has already been approved by the drug authorities creates a new, independent substance (New Chemical Entity, NCE) which, for regulatory reasons, requires a new approval.

In a preferred embodiment the linker compound L according to formula (I) is able to carry and/or stabilize by way of adsorption pharmaceutically acceptable compounds which have positive or negative charges.

In a particularly preferred embodiment the linker compound L according to formula (I) is able to carry and/or stabilize by way of adsorption pharmaceutically acceptable compounds which have negative charges.

In a more preferred embodiment the linker compound L according to formula (I) is able to carry and/or stabilize by way of adsorption pharmaceutically acceptable compounds which have phosphate or phosphonate groups.

It was surprisingly found that the electrostatic interaction between the arginine group of the linker compound L and the negative charge of the phosphate group of the pharmaceutically acceptable compound possesses a 'covalent-like' stability.

The term "attachment" here relates to any type of interaction between the surface functionality and the antigen, in particular covalent and non-covalent bonds, such as, for example, hydrophobic/hydrophilic interactions, van der Waals forces, ionic bonding, hydrogen bonds, ligand-receptor interactions, base pairing of nucleotides or interactions between epitope and antibody binding site.

In case where the pharmaceutically acceptable compound is hydrophobic, for example a hydrophobic peptide, it is advantageous to increase the hydrophilicity thereof. In case of a protein or peptide this is possible e.g. by an N- or C-terminal extension with polar amino acids. In a preferred embodiment of the invention the protein or peptide is extended with one or more polar amino acids selected from the group comprising aspartic acid, glutamic acid, histidine, lysine, arginine, serine, threonine or tyrosine, preferably lysine, arginine, glutamic acid and aspartic acid more preferred lysine.

The present invention is therefore particularly directed to a tripartite bioconjugate which comprises an N- or C-terminal extension with polar amino acids for enhancing the solubility and which is connected to the linker compound L by adsorptive linkage, a linker unit U and an antigen/epitope which is illustrated in **Fig. 4a.**

A linker unit U according to the invention is for example used in antibody-drug conjugates (ADCs) which contain various types of linkers. There are three main types of chemically cleavable linkers: acid cleavable, reducible disulfides and those cleavable by exogenous stimuli.

Examples of ADCs are Gemtuzumab ozogamicin (Mylotarg® by Pfizer, linker is 4-(4-acetylphenoxy)butanoic acid), Inotuzumab ozogamicin (Besponsa® by Pfizer, linker is condensation product of 4-(4'-acetylphenoxy)-butanoic acid (AcBut) and 3-methyl-3-mercaptobutane hydrazide (known as dimethylhydrazide), Trastuzumab emtansin (Kadcyla® by Roche, linker is 4-[N-Maleimidomethyl]cyclohexan-1-carboxylate) or Brentuximab vedotin (also known as SGN-035; Adcetris® by Seattle Genetics Inc., linker is the dipeptide valine-citrulline).

In a preferred embodiment of the present invention the peptides comprise as linker unit U comprising an N-terminal extension with an enzymatic cathepsin B-cleavable linker (catB-cleav-linker) to ensure the release of the native unmodified antigen for MHC class I or MHC class II. The enzymatic catB-cleav-linker comprises one of the cathepsin B sensitive dipeptides Val-Cit, Phe-Cit, Leu-Cit, Ile-Cit, Trp-Cit, Phe-Lys, Ala-Lys or Val-Lys, preferred dipeptides are Val-Cit or Trp-Cit, more preferred is Val-Cit.

In one embodiment the compositions according to the invention comprise phosphorylated nanoparticles and one or more peptides which are conjugated to the nanoparticles by adsorptive attachment, wherein the peptide comprises Val-Cit or Trp-Cit as cathepsin B sensitive dipeptide.

**Fig. 4b** shows a preferred tripartite bioconjugate according to the invention. The peptide used in **Fig. 4b** is an

extreme hydrophobic epitope derived from human NY-ESO-1 (SEQ ID NO: 297). For the N-terminal extension the enzymatic (cathepsin B) cleavable peptidic linker Val-Cit was used to ensure the release of the native unmodified antigen for MHC class I or MHC class II. For enhancing the solubility and the electrostatic attraction to the nanoparticle the extension part "Lys-Lys-Lys" was used. Also possible is the extension for example with Lys-Lys-Lys-Asp or $Arg_6$.

In a preferred embodiment of the present invention the HPV16 $E7_{82-90}$ epitope LLMGTLGIV (SEQ ID NO: 233) is enlarged on the N-terminal site with the enzymatic cleavable linker Val-Cit and the cationic solubilizing sequence Lys-Lys-Lys, leading to KKKV-Cit-LLMGTLGIV.

In another preferred embodiment of the present invention the HPV16 $E7_{11-19}$ epitope YMLDLQPET (SEQ ID NO: 174) is enlarged on the N-terminal site with the enzymatic cleavable linker Trp-Cit and the cationic solubilizing sequence Lys-Lys-Lys leading to KKKW-Cit-YMLDLQPET.

A further embodiment is a composition according to the invention comprising $SiO_2$-nanoparticles having linker compounds L on the surface, wherein the linker compound L comprises at least one guanidino-group ($-NHC(=NH)NH_2$ or $-NHC(=NH_2^+)NH_2$) and at least one carboxyl (-COOH) or carboxylate ($-COO^-$) group as functional groups and one or more peptides or antigens which are conjugated to the linker compound L by adsorptive attachment, wherein the peptide or antigen comprises a linker unit U and a hydrophilic elongation with one or more amino acids.

A preferred embodiment is a composition according to the invention comprising $SiO_2$-nanoparticles having linker compounds L on the surface, wherein the linker compound L comprises at least one guanidino-group ($-NHC(=NH)NH_2$ or $-NHC(=NH_2^+)NH_2$) and at least one carboxyl (-COOH) or carboxylate ($-COO^-$) group as functional groups and one or more peptides or antigens which are conjugated to the linker compound L by adsorptive attachment, wherein the peptide or antigen comprises a cathepsin B sensitive dipeptide and a hydrophilic elongation with Lys-Lys-Lys.

A more preferred embodiment is a composition according to the invention comprising $SiO_2$-nanoparticles having linker compounds L on the surface, wherein the linker compound L comprises at least one guanidino-group (--$NHC(=NH)NH_2$ or - $NHC(=NH_2^+)NH_2$) and at least one carboxyl (-COOH) or carboxylate ($-COO^-$) group as functional groups and one or more peptides or antigens which are conjugated to the linker compound L by adsorptive attachment, wherein the peptide or antigen comprises Val-Cit or Trp-Cit as cathepsin B sensitive dipeptide and a hydrophilic elongation with Lys-Lys-Lys.

Another embodiment is a composition according to the invention comprising $SiO_2$-nanoparticles having linker compounds L on the surface, wherein the linker compound L comprises at least one guanidino-group $-HC(=NH)NH_2$ or $-NHC(=NH_2^+)NH_2$) and at least one carboxyl (-COOH) or carboxylate ($-COO^-$) group as functional groups and the model peptide SIINFEKL which is conjugated to the linker compound L by adsorptive attachment, wherein the peptide comprises Val-Cit or Trp-Cit as cathepsin B sensitive dipeptide and a hydrophilic elongation with Lys-Lys-Lys.

However, this hydrophilic modification of the peptide could lead to a stronger binding to MHC class I thereby displacing the target epitope and could provoke an immune response against an epitope not present on the tumor cells. To avoid this effect, there could be the need of a spacer between the hydrophilizing sub-molecule and the epitope.

An optional embodiment of the present invention is the use of so-called self-immolative spacers (PABC, p-aminobenzylcarbamate). Examples of such self-immolative spacers are compounds which comprise the structural unit of 4-aminobenzyl alcohol, 2-aminobenzyl alcohol or 4-hydroxybenzyl alcohol, 2-hydroxybenzyl alcohol (EP-A 0 648 503, WO 2007/031734 A1, WO 2015/162291 A1, US 6,180,095 B1, US 6,214,345 B1).

A self-immolative spacer is defined as a molecular section which is chemically linked to at least two further molecular sections such that when one of the bonds to the molecular sections is released, the remaining bonds are split and the previously attached molecules are released.

If a self-immolative spacer is used, it is preferred that the spacer is placed between enzymatic catB-cleav-linker and the peptide. As an example the following bioconjugate could be produced: ($Arg_6$)-(Val-Cit)-(PABC)-(SIINFEKL), wherein SIINFEKL (SEQ ID NO: 296) is a model antigen.

It was surprisingly found that the enzymatic catB-cleav-linker systems can be used without the need of a self-immolative spacer.

The epitopes used for therapeutic vaccination typically consist of 8 to 11 amino acids for MHC class I and a maximum of 25 amino acids for MHCclass II. For those epitopes there is no need for using a self-immolative spacer, because of the small size of these epitopes in comparison to much larger drugs, inhibiting an enzymatic cleavage

A more preferred embodiment is a composition according to the invention comprising $SiO_2$-nanoparticles having linker compounds L on the surface, wherein the linker compound L comprises at least one guanidino-group ($-NHC(=NH)NH_2$ or - $NHC(=NH_2^+)NH_2$) and at least one carboxyl (-COOH) or carboxylate ($-COO^-$) group as functional groups and poly(I:C) or any derivatives thereof and one or more immunogenic fragments which are conjugated to the linker compound L by adsorptive attachment, wherein the an immunogenic fragment comprises a linker unit U and a hydrophilic elongation with one or more amino acids.

In specific embodiments, the immunogenic fragment can be selected from the group consisting of the following:

(a) peptides suitable to induce an immune response against infectious diseases;

(b) peptides suitable to induce an immune response against cancer cells.

In some embodiments, the immunogenic fragment is one that is useful for the prevention of infectious disease. Such treatment will be useful to treat a wide variety of infectious diseases affecting a wide range of hosts, preferably human, but including cow, sheep, pig, dog, cat, and other mammalian species and non-mammalian species.

Examples of cancers include, but are not limited to cervical cancer, anogential cancer, head and neck cancer or cytological abnormalities such as atypical squamous cells of undetermined significance (ASCUS) or any cancer caused by one or more HPV types. Another example of a disease which is caused by a HPV infection is cervical intraepithelial neoplasia grade 1 (CIN1), CIN2, CIN3. Cervical intraepithelial neoplasia (CIN), also known as cervical dysplasia, is the abnormal growth of cells on the surface of the cervix that could potentially lead to cervical cancer. More specifically, CIN refers to the potentially precancerous transformation of cells of the cervix.

One example of an infectious disease is a persistent infection with a high-risk HPV type.

HPV-derived immunogenic fragments are commonly used for immunoprophylaxis of HPV-related conditions, for instance, as vaccines or as immunostimulant.

The term "vaccine" or "immunostimulant" refers to a composition that comprises an immunogenic fragment capable of provoking an immune response in an individual, such as a human, wherein the composition optionally contains an adjuvant. A vaccine for HPV suitably elicits a protective immune response against incident infection, or persistent infection, or cytological abnormalities such as ASCUS, CIN1, CIN2, CIN3, or cancer caused by one or more HPV types.

Another object of the present invention is the composition comprising nanoparticles which are loaded with pharmaceutically acceptable compounds comprising one or more HPV-derived immunogenic fragments or a variant thereof according to the present invention for use as vaccines or as immunostimulant.

In one embodiment the compositions according to the invention are used as vaccines for personalized cancer treatment.

The compositions according to the invention are used as vaccines for the prevention of HPV infection or the treatment of HPV-positive humans or the treatment of HPV-positive tumors.

Also object of the present invention is a lyophilisate comprising nanoparticles according to the invention. The lyophilisate may comprise additives for example polymers (e.g. polyethylene glycol, polyvinyl pyrrolidone, hydroxyethyl starch, dextran and ficoll) and sugars, (e.g. trehalose, lactose, sucrose, glucose, galactose, maltose, mannose and fructose), polyhydroxy alcohols (e.g. mannitol, sorbitol and inositol), amino acids (e.g. glycine, alanine, proline and lysine) and methylamines (e.g. trimethylamine-N- oxide, betaine and sarcosine). Lyophilisates according to the invention could be resuspended with sterile water before vaccination.

A further object of the present invention is to provide a vaccine comprising compositions according to the invention.

Compositions according to the present invention are preferably stable dispersions.

The nanoparticles can be in dispersed form in any desired solvent, so long as the nanoparticles are neither chemically attacked nor physically modified by the solvent, and vice versa, so that the resultant nano-dispersion is stable, in particular pharmaceutically and physically stable. The dispersion is specifically characterized in that the nanoparticles are in monodisperse and non-aggregated form and have no tendency towards sedimentation, which results in sterile filterability.

A pharmaceutical composition according to the present invention is any composition which can be employed in the prophylaxis, therapy, control or post-treatment of patients who exhibit, at least temporarily, a pathogenic modification of the overall condition or the condition of individual parts of the patient organism, in particular as a consequence of infectious diseases, tumors or cancer. Thus, in particular, it is possible for the pharmaceutical composition in the sense of the invention to be a vaccine and/or an immunotherapeutic agent.

The compositions according to the invention may be formulated as pharmaceutical compositions that may be in the forms of solid or liquid compositions. Physiological saline solution or glycerol or glycols such as propylene glycol or polyethylene glycol may be included.

The compositions according to the present invention optionally may comprise other active ingredients or may comprise one or more of a pharmaceutically acceptable excipient, carrier, buffer, stabilizer, isotonic agent, preservative or anti-oxidant or other materials well known to those skilled in the art. Such materials should be non-toxic and should not interfere with the efficacy of the pharmaceutically acceptable compound. The precise nature of the carrier or other material may depend on the route of administration, e.g. orally or parenterally.

For intravenous, cutaneous or subcutaneous injection, or injection at the site of affliction, the composition according to the present invention will be in the form of a parenterally acceptable aqueous solution which is pyrogen-free and has suitable pH, isotonicity and stability.

Preservatives are generally included in compositions according to the invention to retard microbial growth, extending the shelf life of the compositions and allowing multiple use packaging. Examples of preservatives include phenol, meta-cresol, benzyl alcohol, para-hydroxybenzoic acid and its esters, methyl paraben, propyl paraben, benzalkonium chloride, 1-thioglycerol and benzethonium chloride.

The nanoparticle-containing compositions of the invention may be administered to patients by any number of different routes, including enteral or parenteral routes. Parenteral administration of the pharmaceutical composition is preferred. Parenteral administration includes administration by the following routes: cutaneous or subcutaneous, nasal, vaginal, rectal, intramuscular, intraocular, transepithelial, intraperitoneal, intracardiac, intraosseous, intradermal, intrathecal, intraperitoneal, transdermal, transmucosal, and inhalational and topical (including dermal, ocular, rectal, nasal, vaginal, inhalation and aerosol), and rectal systemic routes. In a preferred embodiment, the pharmaceutical composition is for local (e.g., mucosa, skin) applications.

Administration be performed e.g. by injection, or ballistically using a delivery gun to accelerate their transdermal passage through the outer layer of the epidermis. The nanoparticles can then be taken up, e.g. by dendritic cells, which mature as they migrate through the lymphatic system, resulting in modulation of the immune response and vaccination against the epitopic peptide and/or the antigen from which the epitopic peptide was derived or of which it forms a part. The nanoparticles may also be delivered in aerosols. This is made possible by the small size of the nanoparticles.

Particularly preferred is the injection an intradermal, subcutaneous, intramuscular injection. The administration can be carried out, for example, with the aid of so-called vaccination guns or by means of syringes. Mucosal administration, in particular mucosal vaccination, can be beneficial for cases were pathogens enter the body via the mucosal route. Mucosal delivery routes primarily include the oral and rectal route. Sometimes a pretreatment of the mucosa is necessary. It is also possible to prepare the substance as an aerosol, which is inhaled by the organism, preferably a human patient and taken up by the nasal and or bronchial mucosa. Other possible forms of mucosal administration are vaginal and rectal suppositories. These forms of administration are cost-effective (no consumables like syringes and needles), safe (very low risk of infection and operating errors), easy to apply and comprised with a high patients compliance (no needle fear). It also addresses the mucosal immune system (MALT) directly.

In one embodiment of the present invention the vaccines comprising the compositions according to the invention are used for a mucosal administration.

The exceptionally small size of the nanoparticles of the present invention is a great advantage for delivery to cells and tissues, as they can be taken up by cells even when linked to targeting or therapeutic molecules. Thus, the nanoparticles may be internalized by APCs, the immunogenic fragments processed and presented via MHC class I and MHC class II.

## PARTICULARLY PREFERRED EMBODIMENTS OF THE INVENTION

1. Composition comprising nanoparticles which are loaded with pharmaceutically acceptable compounds comprising one or more human papilloma virus (HPV)-derived immunogenic fragments or a variant thereof, having silicon dioxide and functional groups on the surface, wherein

    - the functional groups are capable to carry and/or stabilize both negative and positive charges of the pharmaceutically acceptable compounds,
    - the zeta potential of the composition has a value of at least ± 15 mV,
    - the nanoparticles have a particle size below 150 nm.

2. Composition according to embodiment 1 characterized in that the immunogenic fragment is derived from a high-risk HPV genotype, preferably selected from HPV types 16, 18, 31, 33, 35, 39, 45, 51, 52, 56, 58, 59, 66, 68, 73, and 82.

3. Composition according to embodiment 1 or 2 characterized in that the immunogenic fragment is derived from the E6 and/or E7 proteins of HPV16 and/or HPV18, preferably selected from SEQ ID NOs: 5-257.

4. Composition according to any of embodiments 1 to 3 characterized in that the immunogenic fragment is at least 75% identical to an immunogenic fragment derived from the E6 and/or E7 proteins of HPV16 and/or HPV18, preferably selected from SEQ ID NOs: 258-295.

5. Composition according to any of embodiments 1 to 4 characterized in that the immunogenic fragment is able to bind to major histocompatibility complex (MHC) class I and/or MHC class II.

6. Composition according to any of embodiments 1 to 5 characterized in that the immunogenic fragment is able to induce a cytotoxic T cell response.

7. Composition according to any of embodiments 1 to 6 characterized in that the immunogenic fragment has a length of 3 to 35 amino acids, more preferably of 5 to 25 amino acids, more preferably of 6 to 25 amino acids, more preferably of 7 to 25 amino acids, more preferably of 8 to 25 amino acids, most preferably of 8 to 20 amino acids.

8. Composition according to any of embodiments 1 to 7, wherein the nanoparticles are loaded with pharmaceutically acceptable compounds comprising one or more human papilloma virus (HPV)-derived immunogenic fragments and polyinosinic:polycytidylic acid (poly(I:C)) or any derivatives thereof.

9. Composition according to embodiment 8, wherein derivatives of poly(I:C) are poly(I:C) LMW (Low Molecular Weight Poly(I:C)) with an average size from 0.2 kb to 1 kb, poly(I:C) HMW (High Molecular Weight Poly(I:C)) with an average size from 1.5 kb to 8 kb, polyl:poly$C_{12}$U.

10. Composition according to embodiments 1 to 9, wherein the nanoparticles have a surface loading density up to 0.5, preferably between 0.01 and 0.5, more preferred between 0.03 and 0.4, most preferred between 0.05 to 0.3, in relation to the total number of the pharmaceutically acceptable compounds with regard to the surface of the nanoparticle in $nm^2$ [molecules/$nm^2$].

11. Composition according to embodiments 1 to 10, wherein the Polydispersity Index (PDI) of the composition is between 0 and 0.32, preferably between 0.1 and 0.3, more preferably between 0.1 and 0.2, most preferred less than 0.1.

12. Composition according to any of embodiments 1 to 11, wherein the pH value of the composition is between 6.0 and 8.0.

13. Composition according to any of the preceding embodiments, wherein the zeta potential of the composition has a value of at least ± 30 mV.

14. Composition according to any of the preceding embodiments, wherein the Z-average diameter of the nanoparticles is in the range of ≥5 and <150 nm, preferably ≥15 and ≤60nm, more preferably ≥20 and ≤50nm and still more preferably between 20 and 30nm.

15. Composition according to any of the preceding embodiments, wherein the net charge of the loaded nanoparticles in total is different to zero.

16. Composition according to any of the preceding embodiments, wherein the functional groups are connected to a linker L which is linked to the surface of the nanoparticles by way of a covalent or adsorptive bond.

17. Composition according to embodiment 16, wherein the linker compound L comprises at least one carboxyl (-COOH) or carboxylate ($-COO^-$) group as functional group.

18. Composition according to embodiment 16 or 17, wherein the linker compound L comprises at least one guanidino-group ($-NHC(=NH)NH_2$ or $-NHC(=NH_2^+)NH_2$) or amino-group ($-NH_2$ or $-NH_3^+$) group as a functional group.

19. Composition according to any of embodiments 16 to 18, wherein the linker L comprises at least one further functional group L', which is selected from the group -SH, -COOH, $-NH_2$, -guanidino-group ($-NHC(=NH))NH_2$), $-PO_3H_2$, $-PO_2CH_3H$, $-SO_3H$, -OH, - $NR_3^+X^-$.

20. Composition according to any of embodiments 10 to 13, wherein the linker compound L contains at least a structural unit of formula (I)

*- (-O)3Si-(CH2)n-CH(COOX)-(CH2)p-C(O)-NH-CH(COOX)-(CH2)q-Y (I)

wherein

| | |
|---|---|
| X | is independently from each other H or a negative charge, |
| Y | is independently from each other $-NHC(=NH)NH_2$, $--NHC(=NH_2^+)NH_2$, $-NH_2$ or $-NH_3^+$, |
| n, p and q | are independently from each other 0 or a number from 1 to 25; and |
| *- | is the connection point to the nanoparticle. |

21. Composition according to any of the preceding embodiments, wherein the pharmaceutically acceptable compound is conjugated to the nanoparticle by adsorptive attachment.

22. Composition according to any of the preceding embodiments, wherein the immunogenic fragment is N- or C-terminally extended with polar amino acids.

23. Composition according to embodiment 22, wherein the polar amino acids are selected from the group aspartic acid, glutamic acid, histidine, lysine, arginine, serine, threonine, tyrosine.

24. Composition according to embodiment 22 or 23, wherein the peptides comprise an N-terminal extension with an enzymatic cathepsin B-cleavable linker.

25. Composition according to embodiment 24, wherein the enzymatic cleavable linker comprises one of the cathepsin B sensitive dipeptides Val-Cit, Phe-Cit, Leu-Cit, Ile-Cit, Trp-Cit, Phe-Lys, Ala- Lys or Val-Lys.

26. Composition according to embodiment 24 or 25, wherein a spacer is placed between enzymatic cleavable linker and peptide.

27. Composition according to embodiment 26, wherein the spacer is a self-immolative spacer.

28. Composition according to embodiment 26 or 27, wherein the spacer comprises the structural unit of 4-aminobenzyl alcohol, 2-aminobenzyl alcohol or 4-hydroxybenzyl alcohol, 2-hydroxybenzyl alcohol.

29. Composition according to any of the preceding embodiments, wherein the nanoparticles comprise at least silicon dioxide ($SiO_2$), optional in mixture with another material.

30. Composition according to any of the preceding embodiments, wherein the nanoparticles consist of $SiO_2$.

31. Composition according to any of the preceding embodiments for use as vaccine or as immunostimulant.

32. Composition according to any of the preceding embodiments for personalized cancer treatment.

33. Composition according to any of the preceding embodiments for use as vaccine for the prevention of HPV infection or the treatment of HPV positive humans or the treatment of HPV positive tumors.

34. Lyophilisate comprising compositions according to any of the preceding embodiments.

35. Vaccine comprising compositions according to any of the preceding embodiments.

36. Composition according to any of the preceding embodiments, wherein the compositions are stable dispersions.

37. Composition according to any of the preceding embodiments, wherein the pH value of the composition is between 7.2 and 7.4.

38. Composition according to any of the preceding embodiments, wherein the composition is isotonic.

39. Composition according to any of the preceding embodiments, wherein the composition contains further pharmaceutically acceptable additives.

## EXAMPLES

### EXAMPLE 1

**Preparation of monodisperse Silicon Dioxide Nanoparticles with 25 nm Diameter**

500 mL ethanol absolute were taken from a 500 mL graduated measuring cylinder into a 1000 mL glass bottle with screw cap. 358 mL sterile DI water was added and the mixture was well shaken.

69.6 mL tetraethylorthosilicate (TEOS) were added to the bottle. The bottle was tightly closed and well shaken.

After 1 hour waiting time the clear colorless mixture got room temperature (22 °C). Then 9.5 mL ammonia water (25 %) was added quickly.

The bottle was shaken by hand very well for 10 seconds and stored at room temperature.

After 24 hours at room temperature additional 34.8 mL TEOS were added to the mixture (seed-growth process), in order to get a more narrow size distribution. After additional 24 hours at room temperature again 34.8 mL were added to the mixture (seed-growth process continued). After 24 hours at room temperature 50 μL of the reaction mixture were placed in a one-way PMMA cuvette (10 mm width) and diluted with 1.5 mL DI water (0.2 μm filtered). The cuvette was placed in a ZetaSizer Nano for particle size measurement (ZetaSizer Nano ZS, Malvern Instruments, UK).

Applied parameters:

| | |
|---|---|
| Measurement Angle: | 173° Backscatter (NIBS default) |
| Measurement Cell: | DTS1070 (used for particle size and zeta potential) |
| Refractive Index SiO2: | 1.460 |
| Absorption: | 0.010 |
| Dispersant: | water |
| Refractive Index: | 1.330 |
| Viscosity: | 0.8872 cP (= sample viscosity) |
| Temperature: | 25 °C |

>10 measurement runs for particle size

**Results:**

| | |
|---|---|
| Z-average (volume based): | 23.86 nm |
| PDI: | 0.108 |
| Intensity Peak: | 26.77 nm |
| pH: | 11.0 (freshly calibrated combination electrode) |

The nanoparticle suspension was transferred into a 2 liter round bottom flask and the ethanol as well as the ammonia gas was removed by a rotary evaporator with heated water bath. 400 mL sterile DI water was added in portions. The volume was reduced down to 198.15 g.

The solid content (pure nano dispersed silicon dioxide) of the suspension was determined in triplicate via vaporization of 250 μL and weigh out the residue.

**Result:**

| | |
|---|---|
| Solid content: | 163.3 mg/mL |
| pH: | 8.0 (freshly calibrated combination electrode) |

The distillation residue was diluted with 264.5 mL sterile DI water, in order to get a solid content of 70.0 mg/mL.

**EXAMPLE 2**

**Functionalization of the Nanoparticles with L-arginine to get Arginylated Silica Nanoparticles ($SiO_2$-Arg)**

462.65 g of the nanoparticle suspension obtained in Example 1 were placed into a 500 mL glass bottle. A magnetic stir was added and the bottle was placed onto a magnetic stirrer. 17.35 g L(+)-Arginine (CAS No. 74-79-3, PanReac AppliChem, Ph. Eur., USP. product code A1345.0500) were added to the nanoparticle suspension. When the

arginine was completely dissolved a pH value of 10.8 was obtained, then 5.34 mL 3-(Triethoxysilyl)propylsuccinic anhydride (CAS No. 93642-68-3, Gelest Corp. product code: SIT8192.6) were slowly added at room temperature. The reaction mixture got very turbid but cleared up within about 30 minutes. During this time two reactions took place simultaneously:
The ethoxy groups of the silane got hydrolyzed, due to the high pH value. The created silanol groups precipitated onto the silica nanoparticles building a surface coating on top of the nanoparticles. In parallel the amino group of the excess arginine reacts with the succininc anhydride group, forming an amide bond.
The pH value dropped to 9.8, due to the consumption of arginine and mainly due to the formation of a carboxylic group, by opening the cyclic anhydride.

After 24 hours of stirring the suspension was transferred into 20 falcons tubes with a 100 kDa membrane (Pall Corp. Macrosep® Advance, product code MAP100C38). The nanoparticle suspension was centrifuged for 10 minutes at 4,000 rpm (= 2,737 g at the used centrifuge) to a volume at least 5 less than the starting volume. After centrifugation the volume in the falcon tubes was restored with sterile DI water and the centrifugation was repeated 5 times. The centrifugation step is necessary to remove excess arginine and possible unbound reaction products.

After centrifugation the solid content of the collected supernatants was determined in triplicate. The method was the same as in Example 1.

**Result:**

| | |
|---|---|
| Yield: | 178.3 g |
| Solid content: | 97.16 mg/mL |

The nanoparticle suspension was diluted with 168.17 mL sterile DI water to get a final concentration of 50.0 mg/mL.

**EXAMPLE 3**

**Synthesis of Phosphorylated Silica Nanoparticles with 25 nm Diameter**

200 mL ethanol absolute were taken from a 250 mL graduated measuring cylinder into a 500 mL pressure-resistant glass bottle with screw cap 143.5 mL sterile DI water were added and the mixture was well shaken. 27.85 mL tetraethylorthosilicate (TEOS) were added to the bottle. The bottle was tightly closed and well shaken.

After 1 hour waiting time the clear colorless mixture got room temperature (22 °C). Then 3.95 mL ammonia water (25 %) was added quickly. The bottle was shaken by hand very well for 10 seconds and stored at room temperature. After 24 hours at room temperature additional 1.5 mL (Diethylphosphatoethyl)triethoxysilane (CAS No. 757-44-8, Gelest Corp. product code SID3412.0) were added to the mixture at room temperature. 10 mL ammonia water (25 %) was added too and the reaction mixture was placed in a water bath at 85 °C for 24 hours.

At this temperature two reactions took place in parallel: The ethoxy groups of the silane got hydrolyzed, due to the high pH value. The silanol groups precipitated onto the silica nanoparticles building a surface coating on top of the nanoparticles. In parallel, but much slower, the phosphoric acid ester got hydrolyzed to yield into the corresponding acid with ammonia as the counter ion. 50 µL of the reaction mixture were placed in a one-way PMMA cuvette (10 mm width) and diluted with 1.5 mL DI water (0.2 µm filtered). The cuvette was placed in a ZetaSizer Nano for particle size measurement.

**Results:**

| | |
|---|---|
| Z-average (volume based): | 21.3 nm |
| PDI: | 0.144 |
| Intensity Peak: | 27.7 nm |

The nanoparticle suspension was transferred into a 500 mL round bottom flask and the ethanol as well as the ammonia gas was removed by a rotary evaporator with heated water bath. 200 mL sterile DI water was added in portions.

The volume was reduced down to 120 mL. This volume was placed into 6 falcon tubes with a 100 kDa membrane (Pall Corp. Macrosep® Advance, product code MAP100C38). The nanoparticle suspension was centrifuged for 10 minutes at 4,000 rpm (= 2,737 g at the used centrifuge) to a volume at least 80 less than the starting volume. After centrifugation the volume in the falcon tubes was restored with sterile DI water and the centrifugation was repeated 5 times. The centrifugation step is necessary to remove excess possible unbound reaction products. After centrifugation the solid content of the collected supernatants was determined in triplicate. The method was the same as in Example 1.

**Result:**

| | |
|---|---|
| Yield: | 83.3 g |
| Solid content: | 104.0 mg/mL |

The nanoparticle suspension was diluted with 86.6 mL sterile DI water to get a final concentration of 50.0 mg/mL.

Reaction scheme 1:

Example 1 = a) and Example 2 = b)

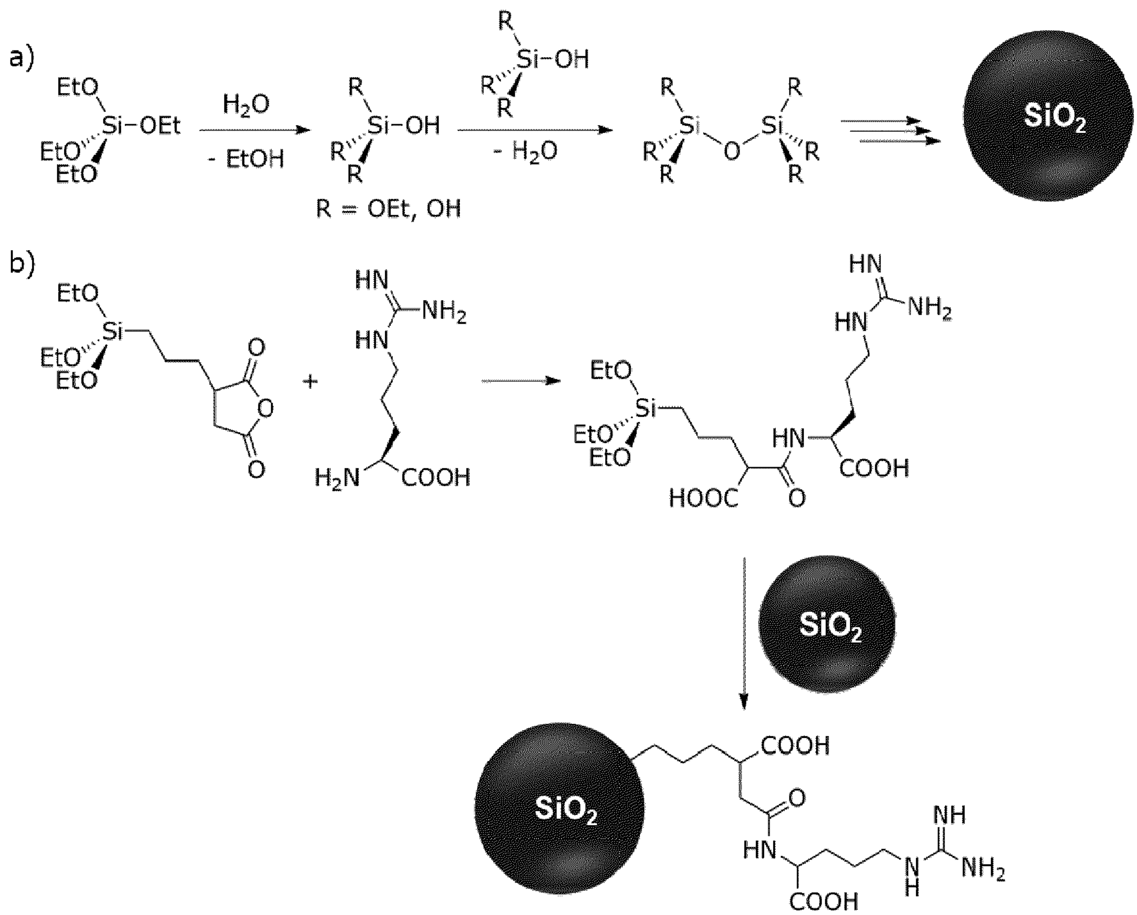

Reaction scheme 2:

Example 3

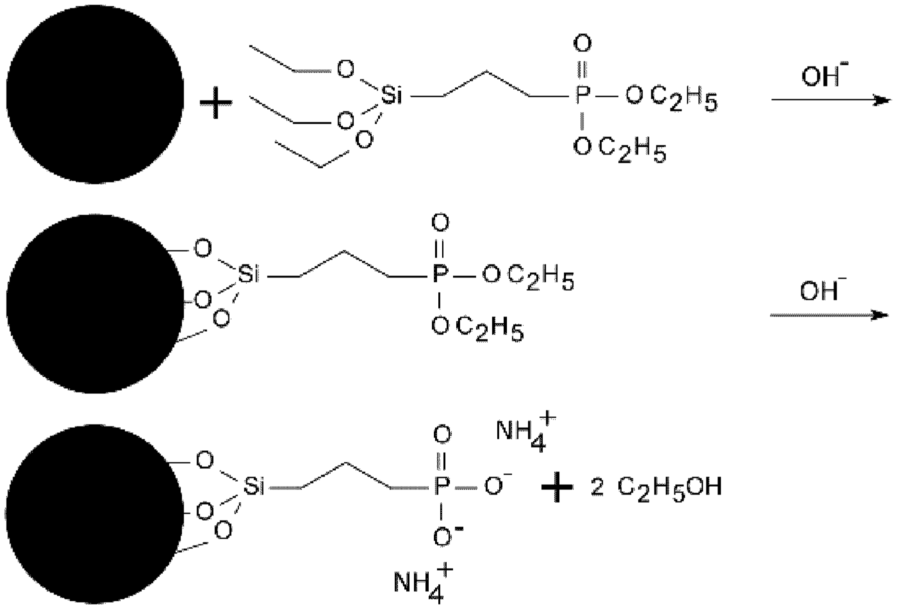


20

## EXAMPLE 4

**Preparation of poly(I:C)@ $SiO_2$-Arg by adsorptive binding of poly(I:C)**

A mixture of 200 mL ethanol, 143.5 mL sterile de-ionized water and 27.85 mL tetraethyl orthosilicate (TEOS) was prepared. 3.70 mL ammonia, 25 % ($NH_3$ in water) were added at room temperature. The reaction mixture was mixed vigorously by shaking for 10 seconds and left at room temperature for 24 hours without stirring. The next day another portion of 27.85 mL tetraethyl orthosilicate (TEOS) was added to the mixture. From the resulting mixture 50 $\mu$L were removed and measured by means of dynamic light scattering (DLS) on a ZetaSizer Nano ZS (Malvern). The following results were obtained:

| | |
|---|---|
| Z-average: | 25.53 nm |
| mean: | 20.36 nm |
| polydispersity index (PDI): | 0.136 |

A portion of 100 ml of the silicon dioxide particles produced before were subsequently concentrated to a volume of about 30 ml on a rotary evaporator and filled up again to 100 ml. This procedure was repeated three times to remove the ethanol and ammonia from the reaction solution. The resulting suspension was washed five times over a 100 kDa membrane with sterile deionized water. After the last washing step, the solids content of the suspension was determined gravimetrically with 7.9% $SiO_2$ and the suspension was adjusted to a solids content of 5.0% by adding the calculated amount of water.

500 mg L-arginine (CAS number 74-79-3; company abcr GmbH, Germany) (= 2.87 mmol) was added to 15 ml of the silicon dioxide particles produced in Example 1 (750 mg $SiO_2$) and stirred while the arginine was completely dissolved. A clear particle suspension was obtained and to this suspension 127 $\mu$L (3-triethoxysilylpropyl) succinic anhydride (CAS number: 93642-68-3) (= 0.45 mmol) were slowly added while stirring at room temperature.

2.00 ml of the "argininylated" silica nanoparticles obtained above were mixed with 3.00 ml of a low molecular weight solution of poly(I:C) (poly(I:C)-LMW from InvivoGen, Toulouse, France with a size of 0.2 to 1 kb, CAS number 31852-29-6) with a concentration of 0.833 mg poly(I:C)/mL. After thoroughly mixing with a vortex mixer for 15 seconds

and after one hour, the clear suspension was mixed with 250 mg of glucose. The formulation comprises:

| | |
|---|---|
| 100 mg | "argininylated" silica nanoparticles, $SiO_2$-Arg (c=20 mg/mL) |
| 2.5 mg | poly(I:C)-LMW (c = 0,5 mg/mL) |
| 250 mg | glucose (c = 50 mg/mL) (for isotonization of the formulation) |

The mixture was then filled into three sterile 2.0 mL HDPE vials using a 0.2 µm sterile filter. The obtained suspension is clear and completely transparent.
The poly(I:C) loaded particles pass easily a sterile filter. Two types of filters were used: Pall Life Sciences, Acrodisc Supor® Membrane (low protein binding) 0.2 µm, cat. no. PN4602 and VWR 0.2 µm Cellulose Acetate Membrane 0.2 µm, cat. no. 514-0061.
Even after adding larger amounts of poly(I:C), the suspension remains clear and completely transparent.

## EXAMPLE 5

### Peptide Loading Capacity of $SiO_2$-Arg Nanoparticles

In a peptide loading experiment 100 µL silica nanoparticles with a diameter of 25 nm (Z-average), a solid content of 20 mg/mL and an arginylated surface were loaded with different amounts of the model peptide KKKW-Cit-SIINFEKL. To simulate physiological conditions sodium chloride (NaCl) was added to get an isotonic suspension (0.9 % NaCl). Up to 10 % of peptide was added to the nanoparticles. KKKW-Cit-SIINFEKL has an iso-electric point of pH 10.24, indicating cationic properties determined by 4 basic amino acids (Lysine) and 1 acidic amino acid (Glutamic acid).
50 µL of the corresponding particle-peptide-NaCl mixture was diluted with 1.5 mL sterile filtered de-ionized water and measured via dynamic light scattering (DLS) in a ZetaSizer Nano (Malvern Instruments, UK). For every sample # 1 to 10 100 µL $SiO_2$-Arg stock solution was used. The obtained results are listed in Table 2.

Table 2: Results of the dynamic light scattering

| # | [mg/mL] $SiO_2$-Arg | [mg] peptide | [%] peptide | [µL] Peptide stock[2] | [mg] NaCl | [µL] NaCl stock[3] | z average [nm] | PDI |
|---|---|---|---|---|---|---|---|---|
| 0 | 20 | 0.00 | 0.00 | 0.0 | 0.90 | 9.00 | 23.62 | 0.07 |
| 1 | 20 | 0.02 | 1.00 | 5.1 | 0.95 | 9.45 | 24.00 | 0.10 |
| 2 | 20 | 0.04 | 2.00 | 10.2 | 0.99 | 9.92 | 24.48 | 0.10 |
| 3 | 20 | 0.06 | 3.00 | 15.5 | 1.04 | 10.39 | 26.03 | 0.14 |
| 4 | 20 | 0.08 | 4.00 | 20.8 | 1.09 | 10.87 | 26.91 | 0.14 |
| 5 | 20 | 0.11 | 5.00 | 26.3 | 1.14 | 11.37 | 28.,70 | 0.18 |
| 6 | 20 | 0.13 | 6.00 | 31.9 | 1.19 | 11.87 | 59.09 | 0.27 |
| 7 | 20 | 0.15 | 7.00 | 37.6 | 1.24 | 12.39 | 44.58 | 0.20 |
| 8 | 20 | 0.17 | 8.00 | 43.5 | 1.29 | 12.91 | 440.5 | 0.58 |
| 9 | 20 | 0.20 | 9.00 | 49.5 | 1.35 | 13.45 | 1074 | 0.31 |
| 10 | 20 | 0.22 | 10.00 | 55.6 | 1.40 | 14.00 | 2753 | 0.21 |

[1]Stock solution $SiO_2$-Arg: 20 mg/mL $SiO_2$-Arg
[2]Stock solution Peptide: 4 mg/mL Peptide (KKKW-Cit-SIINFEKL)
[3]Stock solution NaCl: 100 mg/mL NaCl

**Fig. 5a** illustrates the Z average versus peptide concentration and **Fig. 5b** the PDI versus peptide concentration.
As shown in **Fig. 5a** and **5b**, up to 5 % by weight of this peptide can be added to the nanoparticles, to keep a stable suspension without agglomeration or precipitation. Compared to bare particles the diameter is increasing slightly (+5 nm) and the PDI indicates a still quite narrow particle size distribution. This optical clear suspension is still passing a sterile filter.
The peptide loaded particles pass easily a sterile filter. Two types of filters were used: Pall Life Sciences, Acrodisc Supor® Membrane (low protein binding) 0.2 µm, cat. no. PN4602 and VWR 0.2 µm Cellulose Acetate Membrane 0.2

μm, cat. no. 514-0061.

With increased peptide loading the particle size increases, indicating the adsorptive peptide binding to the silica nanoparticles surface. At higher peptide concentrations - in the above example 6 % - the nano-suspension is collapsing, indicated by clouding and a measureable increased particle size due to agglomeration. These suspensions do not pass a sterile filter and are very unfavorable for parenteral administration.

## EXAMPLE 6

### Poly(I:C) Loading Capacity of $SiO_2$-Arg Nanoparticles

In a loading experiment 50 μL silica nanoparticles with a diameter of 25 nm (Z average), a solid content of 20 mg/mL and an arginylated surface were loaded with different amounts of poly(I:C) solution in RNase/DNase-free water by Gibco™. The poly(I:C) (LMW) was obtained from Invivogen Europe (cat. no. tlrl-picw). To simulate physiological conditions sodium chloride (NaCl) was added to get an isotonic suspension (0.9 % NaCl). Up to 8 % of poly(I:C) were added to the nanoparticles. The colloid remained stable: no precipitation or clouding was observable. The particle sizes and distributions, as well as the zeta potentials confirm the visual observations.

For the Zeta potential was measured according to the Smoluchowski calculation model under the following conditions:

| | |
|---|---|
| Measurement Temp.: | 25 °C |
| Equilibration Time: | 30 s |

10 to 100 runs per measurement, 5 measurements per sample (Zeta potential = mean of 5 measurements)

Table 3: Results of the dynamic light scattering

| [mg] poly(I:C) | [μL] poly(I:C ) stock | Vol [μL] | [mg] NaCl | [μL] NaCl stock | Z ave [nm] | PDI | Peak [nm] | int. Peak [nm] | Zeta potential |
|---|---|---|---|---|---|---|---|---|---|
| - | 50 | - | 0.45 | 4.50 | 33.83 | 0.557 | 12.69 | *) | - |
| 0.00 (0 %) | - | 50.0 | 0.45 | 4.50 | 22.79 | 0.077 | 20.23 | 24.47 | -27.4 |
| 0.01 (1 %) | 4.0 | 54.0 | 0.49 | 4.86 | 22.92 | 0.067 | 21.42 | 24.25 | -28.1 |
| 0.02 (2 %) | 8.2 | 58.2 | 0.52 | 5.23 | 23.12 | 0.075 | 19.96 | 23.97 | -28.7 |
| 0.04 (4 %) | 16.7 | 66.7 | 0.60 | 6.00 | 23.35 | 0.075 | 20.04 | 25.23 | -34.2 |
| 0.06 (6 %) | 25.5 | 75.5 | 0.68 | 6.80 | 23.45 | 0.08 | 19.96 | 34.93 | -36.2 |
| 0.09 (8 %) | 34.8 | 84.8 | 0.76 | 7.63 | 23.97 | 0.134 | **) | 32.15 | -37.2 |
| *) two peaks: 27.38 nm (67.8 %) and 362.6 nm (32.2 %)<br>**) two peaks: 7.691 nm (22.9 %) and 18.4 nm (77.1 %) | | | | | | | | | |

Up to a loading of 6 % poly(I:C) LMW the measured Z average values increases due to increase of particle diameter by adsorbed poly(I:C) (**Fig. 6b**). Also the low PDI (<0.1) indicates a smooth particle loading (**Fig. 6a**). At 8 % loading two peaks (at 7.691 and 18.4 nm) appear, also a strong peak broadening of the peak in direction to lower diameter values happens and the PDI value is getting worse (**Fig. 7d**). These data indicate particle "overloading": unbound poly(I:C) generates an additional peak, resp. broadens the measurement peak.

**Figures 7a** to 7d show the Number % in a display of a ZetaSizer. The Number % is the representation of the particle distribution according to its percentage frequency.

**Fig. 7a:** Poly(I:C) LMW without nanoparticles

**Fig. 7b:** $SiO_2$-Arg nanoparticles without poly(I:C)
**Fig. 7c:** $SiO_2$-Arg nanoparticles loaded with 6 % poly(I:C) by weight
**Fig. 7d:** $SiO_2$-Arg nanoparticles loaded with 8 % poly(I:C) by weight

## EXAMPLE 7

### Loading of $SiO_2$-Arg Nanoparticles with Peptides and Poly(I:C)

In a peptide and poly(I:C) loading experiment 100 μL silica nanoparticles with a diameter of 25 nm (Z average), a solid content of 20 mg/mL and an arginylated surface were loaded with different amounts of the model peptide KKKW-Cit-SIINFEKL and with 50 μg poly IC (LMW) each (20 μL of a poly (I:C) stock solution, having a concentration of 2.5 mg/mL).

To simulate physiological conditions sodium chloride (NaCl) was added to get an isotonic suspension (0.9 % NaCl). Up to 4 % of peptide were added to the nanoparticles. The colloid remained stable: no precipitation or clouding was observable. The particle sizes and distributions, as well as the zeta potentials confirm the visual observations.

Table 5: Results of the dynamic light scattering

| [mg] pept. * | [μL]pept.* stock | Vol [μL] | [mg] NaCl | [μL] NaCl stoc k | [μg] Poly( I: C) | z ave [nm] | PDI | int. Peak [nm] | Peak [nm] | Zeta potential |
|---|---|---|---|---|---|---|---|---|---|---|
| 0.04 (2 %) | 10.2 | 110.2 | 0.99 | 9.92 | 50 | 24.09 | 0.172 | 24.57 | 19.48 | -37.1 |
| 0.06 (3 %) | 15.5 | 115.5 | 1.04 | 10.39 | 50 | 27.87 | 0.279 | 25.7 | 21.31 | -27.6 |
| 0.08 (4%) | 20.8 | 120.8 | 1.09 | 10.87 | 50 | 28.47 | 0.305 | 25.41 | 20.76 | -25.9 |
| *(pept. = peptide) | | | | | | | | | | |

## EXAMPLE 8

### Loading of $SiO_2$-Arg Nanoparticles with ssRNA (poly(U))

In an ssRNA loading experiment 400 μL silica nanoparticles with a diameter of 25 nm (Z average), a solid content of 50 mg/mL and an arginylated surface (in total 20 mg silicon dioxide) were loaded with 500 μL poly(U) solution with a concentration of 1.0 mg/mL (in total 0.5 mg poly(U) and 100 μL sodium chloride (NaCl) with a concentration of 90 mg/mL was added to get an isotonic suspension (0.9 % NaCl). The loading of poly(U) on silica nanoparticles in this case is 2.44 % by weight. 50 μL of this sample were measure by DLS. The poly(U) was obtained from InvivoGen Europe, Toulouse, France (Cat. Code: tlrl-sspu).

Table 5: Results of the dynamic light scattering

| [mg] poly (U) | [mg] $SiO_2$-Arg | [mg] NaCl | [μL] total volume | Z ave [nm] | PDI | Peak [nm] | int. Peak [nm] | Zeta potent ial |
|---|---|---|---|---|---|---|---|---|
| 0.5 (2 %) | 20.0 | 90 | 1000 | 23.19 | 0.065 | 20.21 | 24.93 | -31.6 |

## EXAMPLE 9

### Loading of $SiO_2$-Arg Nanoparticles with unmethylated DNA (CpG ODN)

In a DNA loading experiment 40 μL silica nanoparticles with a diameter of 25 nm (Z average), a solid content of 50 mg/mL and an arginylated surface (in total 2 mg silicon dioxide) were loaded with 50 μL ODN 2395 solution with a concentration of 1.0 mg/mL (in total 0.5 mg ODN 2395 and 10 μL sodium chloride (NaCl) with a concentration of 90 mg/mL was added to get an isotonic suspension (0.9 % NaCl). The loading of on silica nanoparticles in this case is 2.44 % by weight. 50 μL of this sample were measure by DLS.

ODN 2395 was obtained from InvivoGen, France (cat. code: tlrl-2395). It is a 22mer with the structure: 5'-tcgtcgttttcggcgc:gcgccg-3' (bases are phosphorothioate (nuclease resistant), palindrome is underlined)

Table 6: Results of the dynamic light scattering

| [mg] CPG ODN | [mg] $SiO_2$-Arg | [mg] NaCl | [μL] total volume | Z ave [nm] | PDI | Peak [nm] | int. Peak [nm] | Zeta potential |
|---|---|---|---|---|---|---|---|---|
| 0.05 (2.44 %) | 2.0 | 9 | 100 | 23.88 | 0.083 | 20.76 | 25.19 | -28.5 |

**EXAMPLE 10**

**Example for Solubility Enhancement of Epitopes**

On the N-terminal site of the enzymatically cleaved linker the addition of polar amino acids can be used to enhance the solubility of the whole peptide. The HLA-A:02 immunogenic HPV 16 E782-90 epitope LLMGTLGIV (SEQ ID NO: 233), for example, is extremely non-polar and has a very bad solubility in water. The use in a human vaccine is difficult. In animal studies researchers dissolve it in DMSO. The solubility after e.g. subcutaneous injection is questionable: dilution might result in precipitation of large peptide particles, which is very unfavorable for transport to lymphnodes.

According to the invention the epitope LLMGTLGIV was enlarged on the N-terminal site with the enzymatic cleavable linker Val-Cit and the cationic solubilizing sequence Lys-Lys-Lys, leading to

**KKKV-Cit-LLM GTLGIV.**

The complete synthesis was done by automated microwave supported solid phase peptide synthesis (SPPS) in one run.

This peptide has an excellent solubility in water. After endosomal and/or cytosolic cleavage by cathepsin B the native HPV 16 E782-90 is released. Also the remaining KKKV-Cit is not immunogenic, due to the fact it's too short to be presented at any MHC class I or MHC class II.

**EXAMPLE 11**

**Human TLR-induced Cytokines in the Primary Human Macrophage Model (THP-1)**

Production of cytokines after stimulation of differentiated THP-1 cells with poly(I:C), $SiO_2$-Arg and poly(I:C)@ $SiO_2$-Arg were performed with Multi-Analyte ELISArray from Qiagen. Nanoparticle diameter is 22.1 nm (Z-average), PDI 0.08.

All experiments were performed with low molecular weight poly(I:C)-LMW from InvivoGen Europe, Toulouse, France.

The screening of cytokines showed significant production of cytokines:

**Fig. 8:** Cytokine expression (arbitrary units) for different types of cytokines after 72 h stimulation

TNF-α, IL8 (CXCL8), MCP-1 (CCL2), RANTES (CCL5), IP-10 (CXCL10), MIG (CXCL9) were highly expressed after 72 h stimulation with poly(I:C) @$SiO_2$-Arg. High cytokine release of IL8 (CXCL8), MCP-1 (CCL2), RANTES (CCL5), IP-10 (CXCL10), MIG (CXCL9) was also noticed for $SiO_2$-Arg nanoparticles.

**Fig. 9:** Cytokine expression (arbitrary units) for different types of cytokines after 96 h stimulation

After 96 h stimulation with poly(I:C) @$SiO_2$-Arg, the expression rate of cytokines IL 1-β, IL12, IL17A, TARC, IFN-α is increased. High cytokine release of IL8 (CXCL8), MCP-1 (CCL2), RANTES (CCL5), IP-10 (CXCL10), MIG (CXCL9) was also noticed for $SiO_2$-Arg nanoparticles.

To investigate whether the combination of the TLR3 agonist poly(I:C)-LMW and $SiO_2$-Arg is able to stimulate cytokine release, the differentiated human macrophage-like THP-1 cells were incubated with poly(I:C)-LMW adsorptively bound to $SiO_2$-Arg (poly(I:C)@ $SiO_2$-Arg) or with the individual compounds and then subjected to cytokine-specific enzyme-linked immunosorbent assay (ELISA). The supernatant of the THP-1 cells was analyzed for interleukin 8 (IL-8) and tumor necrosis factor α (TNF-α) at several time points (**Fig. 10**). IL-8 and TNF-α are important mediators of the innate immune system response, regulating the activity of various immune cells. The release of these two cytokines is proof of a successful stimulation of the immune system.

**Fig. 10a and 10b:** Cytokine release at different time points after stimulation of differentiated THP-1 cells with poly(I:C) [12.5 μg/ml], $SiO_2$-Arg [0.5 mg/ml] or the novel adjuvant (poly(I:C) [12.5 μg/ml] bounded on $SiO_2$-Arg [0.5 mg/ml]).

**Fig. 10a:** Quantification of IL-8 release was performed using ELISA MAXTMDeluxe Set Human IL-8 from

BioLegend, USA.

**Fig. 10b:** Quantification of TNF-α release was performed using ELISA MAXTMDeluxe Set Human TNF-α from BioLegend, USA.

**EXAMPLE 12**

**Experiments in the influenza A model of the mouse**

For the determination of the immunostimulatory potency of TLR3 agonists, a suspension is prepared according to Example 6 was tested in an in vivo study together with other active compounds as immunostimulators with regard to its prophylactic effect against a five-fold LD50 dose of the influenza A virus PR8/34.

The following active compounds were tested:

- Placebo: glucose solution (5 %)
- free poly(I:C) LMW: Low Molecular Weight poly(I:C) comprises short strands of inosinic acid poly(I) homopolymer annealed to strands of cytidinic acid poly(C) homopolymer. The average size of poly(I:C) LMW is from 0.2 kb to 1 kb (InvivoGen, France) in glucose solution (5 %)
- ZelNate®: FDA approved immunostimulant that aids in the prophylaxis of Bovine Respiratory Disease (BRD) due to *Mannheimia haemolytica* (Bayer AG, Germany)
- Poly(I:C)@$SiO_2$-arginylated prepared according to Example 6
- Silicon nanoparticles unmodified with a diameter of 25 nm in 5 % glucose solution

Table 7: Overview of prophylaxis trials in mice, every group consists of ten mice

| Group No. | active compound | composition | H1N1-dose; admin. |
|---|---|---|---|
| A | Placebo | - | 50 $TCID_{50}$ (5× $LD_{50}$); i.n. |
| B | Zelnate® | 100 μL | 50 $TCID_{50}$ (5× $LD_{50}$); i.n. |
| C | poly(I:C) (LMW) | 50 μg in 100 μL | 50 $TCID_{50}$ (5× $LD_{50}$); i.n. |
| D | $SiO_2$ (25nm) | 2mg $SiO_2$; (unmodified) | 50 $TCID_{50}$ (5× $LD_{50}$); i.n. |
| E | poly(I:C)@$SiO_2$-Arg (25 nm) | 2 mg $SiO_2$-"argynilated" and 50 μg poly(I:C) (LMW) in 100 μL | 50 $TCID_{50}$ (5× $LD_{50}$); i.n. |

In all cases the injection volume was 100 μL.

Each animal group (A to E), consisting of ten C57BL/6 mice, was treated 24 hours before administration of the influenza A virus subcutaneously with the respective active compound or placebo. The virus was administered intranasally.

In this study the body weight was used as a reliable and easy-to-measure marker for the animal health. Sick animals eat less and lose weight very quickly. For ethical reasons, the study defined a body weight loss of 25%, based on the body weight on the day of the virus administration, as the termination criterion. In contrast, in many publications from older studies, the "termination criterion" is the death of the animals due to the viral disease.

In this study, the formulation of poly(I:C) prepared according to Example 6, which is adsorptively bound to silica nanoparticles with an "argininylated" surface, showed a surprisingly strong immunostimulatory effect, which resulted in a statistically significantly longer survival rate of the animals of group E and to a significantly less weight loss.

The free poly(I:C), which is not bound to nanoparticles (**group C**), with the same concentration of active ingredient, does not show any statistically significant improvement compared to the placebo group (**group A**). The unmodified

silica nanoparticles (**group D**) also showed no statistically significant improvement.

From the comparative experiments above, it could be shown that a TLR3 agonist which is adsorptively bound to silica nanoparticles (**group E**), is clearly superior over the free TLR3 agonist (**group C**) which was applied in the same concentration and under identical test conditions. The immune booster ZelNate® (**group B**) also showed no significant improvement in this study compared to the placebo group (**group A**). This also applies to the unmodified silica nanoparticles of **group D**, where no significant effect could be observed (**Fig. 11a and 11b**).

**Fig. 12** shows the **clinical scores.** Data are presented as mean clinical score (maximum score = 5; death of animals, abort of experiment) ± SEM (n=10) in relation to days after challenge.

## EXAMPLE 13

### Generation of HPV16 $E7_{11-19}$ specific CD8 T cell immune responses in tumor-free A2.DR1 mice

The immunogenicity of human HPV16 E6/E7-derived, HLA-A2-binding epitopes can only be studied in genetically modified mice. Therefore the in vivo studies were performed using the HLA-humanized A2.DR1 BL6 mice. A2.DR1 mice are a highly sophisticated mouse model since they underwent a multitude of genetic alterations to exhibit the HLA-A2+/HLA-DR1+, H-2-phenotype and shown to assemble functional $CD4^+$ and $CD8^+$ T cell responses against multiple epitopes restricted by HLA-A2 and HLA-DR1. *(*Pajot, A. et al., A mouse model of human adaptive immune functions: HLA-A2.1 -/HLA-DR1-transgenic H-2 class I-/class II-knockout mice, European Journal of Immunology, 2004, Vol. 34, p. 3060-3069*)*.

The efficacy of various formulations was examined for their ability to induce high frequencies of immunogenic fragment-specific $CD8^+$ T cells after three weekly, *subcutaneous* immunizations (Day 0: Prime, Day 7: Boost, Day 14: Boost, Day 21: Spleen Sampling).

Used substances:

- poly(I:C)-HMW: The average size of poly(I:C)-HMW is 1.5 to 8 kb, InvivoGen, France
- $SiO_2$-$PO_3H_2$ according to Example 3

  - *Preparation* of *KKKW-Cit-$E7_{11-19}$* + *poly(I:C)-HMW* @ $SiO_2$-*Arg*;

2.72 mg (1.48 μmol) KKKW-Cit-$E7_{11-19}$ are dissolved in 592 μL DNase/RNase free distilled water under sterile conditions. The solution is added under vortexing to 2.37 ml of a 50 mg/mL stock solution of $SiO_2$-Arg. 296 mg solid glucose are added and the solution is mixed until the solid has completely dissolved. Finally, 2.96 mL poly(I:C)-HMW is added as a 1.0 mg/mL stock solution while the sample is vortexed. The vaccine is filtered through a 0.45 μm filter. 1.8 mL each are filled into 3 separate vials (1 vial per immunization) and stored at 4°C.

The influence of the N-terminal peptide elongation of the immunogenic fragment HPV16 $E7_{11-19}$ (SEQ ID NO: 174 = YMLDLQPET) as well as the influence of the nanoparticles surface modification were investigated. To evaluate the vaccine-induced HPV16 $E7_{11-19}$-specific $CD8^+$ T cells, the ex vivo restimulated splenocytes were assessed in an IFN-γ intracellular cytokine staining (ICS) followed by flow cytometry (**Fig. 13**).

**Fig. 13:**

Y-axis:

HPV-001: RW-Cit-$E7_{11-19}$ [50nmol] + poly(I:C)-HMW [50 μg]
HPV-002: RW-Cit-$E7_{11-19}$ [50nmol] + poly(I:C)-HMW [50 μg] @ $SiO_2$-Arg [2.25 mg]
HPV-003: RW-Cit-$E7_{11-19}$ [50nmol] + poly(I:C)-HMW [50 μg] @ $SiO_2$-$PO_3H_2$ [2.25 mg]
HPV-004: KKKW-Cit-$E7_{11-19}$ [50nmol] + poly(I:C)-HMW [50 μg] @ $SiO_2$-Arg [2.25 mg]

X-axis:

Percentage of IFN-γ+-CD8+ T cells (taken from mouse spleens)

Frequency of IFN-γ positive $E7_{11-19}$ specific $CD8^+$ T cells after ex vivo stimulation of splenocytes with $E7_{11-19}$

in the presence of Golgi apparatus-transport-inhibitors. After subsequent IFN-γ ICS, IFN-γ positive $E7_{11-19}$ specific T cells were determined by flow cytometry. Data are represented as the mean +/- SEM. Each dot represents one mouse.

As shown in **Fig. 13**, three immunizations with nanoparticles conjugated to epitope were able to induce antigen-specific splenic IFN-$\gamma^+$ $CD8^+$ T cells at higher frequencies in comparison to free epitope injections. Furthermore, the direct comparison of two surface modifications of nanoparticles (HPV-002 vs. HPV-003) demonstrated a better performance when the epitope was conjugated to $SiO_2$-Arg (HPV-002).

Moreover, the influence of the N-terminal peptide elongation (HPV-002 vs. HPV-004) was analyzed, where the "KKKW-Cit-"modification (HPV-004) led to higher frequencies of epitope-specific $CD8^+$ T cells.

This experiment successfully demonstrated the ability of the HPV16 vaccines according to the invention to induce a high frequency of epitope specific $CD8^+$ T cells. In summary, $SiO_2$-Arg as well as the "KKKW-Cit-" peptide elongation have shown their beneficial properties over $SiO_2$-$PO_3H_2$ nanoparticles and the "RW-Cit-" peptide elongation. Therefore, the combination of both was used for assessing therapeutic efficacy in a tumor study.

**EXAMPLE 14**

**Therapeutic efficacy in the PAP-A2-HPV16 tumor model**

The final study goal is the development of a therapeutic anti-HPV16 vaccine, which would be given to patients diagnosed with either a precursor lesion or an established cancer. Therefore, the ability of the novel vaccines was tested to induce control of tumor growth in a therapeutic vaccination experiment.

Immunization schedule for early therapeutic treatment study in the PAP-A2-HPV16 tumor model: (Kruse, S. Therapeutic vaccination against HPV-positive tumors in a MHC-humanized mouse model, Ruperto Carola University Heidelberg, 2019*, Dissertation;* Kruse et al., Therapeutic vaccination using minimal HPV16 epitopes in a novel MHC-humanized, Oncoimmunology, 2019, Vol. 8, 1, p. e1524694):

To evaluate the therapeutic efficacy of $SiO_2$-Arg conjugated with HPV16 E7-derived immunogenic fragment, the HPV16 $E6^+$/$E7^+$ PAP-A2 tumor model in HLA-humanized A2.DR1 BL6 mice was used. $1.5 \cdot 10^6$ PAP-A2 cells were injected subcutaneously, which should result in large tumors within 2 to 3 weeks. Starting with day 4 after tumor inoculation, the tumor-bearing mice were treated weekly (3 immunizations total, Prime-Boost-Boost) with the complete vaccine or with the individual compounds as controls, until the ethical endpoint (tumor volume 1000 $mm^3$) was reached.

**Fig. 14** shows the survival rate of mice, either receiving the free antigen HPV16 E7 YMLDLQPET(SEQ ID NO: 174) + poly(I:C) (HMW, high molecular weight), shown as "free antigen + TLR agonist" or KKKW-Cit-YMLDLQPET + poly(I:C) (HMW) both adsorptively bound to arginylated silica nanoparticles having a diameter of 23 nm, shown as "HPV16Nano".

As shown in **Fig. 14**, the treatment of tumor-bearing mice with KKKW-Cit-YMLDLQPET + poly(I:C) (HMW) both adsorptively bound to arginylated silica nanoparticles (HPV16Nano) resulted in complete tumor regression (CR) in 5 out of 9 mice with overall survival rate of 55%. In contrast, 90% of carrier control (free antigen + TLR3 agonist) mice had to be eliminated due to excessive tumor growth. **Fig. 15a** and **Fig. 15b** show the individual tumor growth of both groups.

Taking together the results from therapeutic vaccinations with single compounds and with $SiO_2$-Arg conjugated with epitope, it can be concluded that the best therapeutic anti-tumor results were achieved with the triple surface-arginylated nanoparticles conjugated with KKKW-Cit-YMLDLQPET vaccination.

**EXAMPLE 15**

**Cross-Presentation Experiments**

*MHC class I cross presentation of* ***$OVA_{257-264}$ after intracellular processing in DC2.4 cells***

To demonstrate the functionality of the enzymatic cleavage (W-Cit) site attached to an immunogenic epitope, experiments on cross-presentation of the model antigen $OVA_{257-264}$ (=SIINFEKL in one letter code for amino acids) in murine dendritic cells (DC2.4 cells, immortalized murine dendritic cells) were performed. Therefore, the expression of SIINFEKL on MHC class I after incubation with various OVA-derived constructs, with and without nanoparticles, was determined by antibody (25-D1.16, PE/Cy7 anti-mouse H-2Kb bound to SIINFEKL Antibody, Biolegend, Inc., USA) specific detection of the native epitope presented on MHC class I. Free, not MHC class I bound epitope or elongated/modified or other epitopes on MHC class I are not recognized by the antibody, which is highly selective to SIINFEKL presented on the MHC class I molecule H-2Kb.

In the experiment, the $OVA_{257-264}$ presentation efficacy after incubation of $5.10^4$ DC2.4 cells with 5 μM solutions of full length protein (OVA = Ovalbumin), N- and C-terminal elongated epitope ($OVA_{247-264}A_5K$, a so called synthetic long peptide (SLP)), N-terminal elongated epitope with a Cathepsin B cleavable sequence (exemplary shown RW-Cit-

$OVA_{257-264}$) or native epitope ($OVA_{257-264}$), each with and without nanoparticles, was compared. Surface phosphorylated silica nanoparticles ($SiO_2$-$PO_3H_2$) with an average diameter of 25 nm (comparable size to $SiO_2$-Arg used in other experiments) were used as carrier. After an incubation time of 6 h, the test substances were removed by washing with phosphate-buffered saline (PBS). In the next step, the cells were incubated with CD16/CD32 antibody in order to block the non-specific binding of the detection antibody to the Fc (Fragment, crystallizable) receptor of the cells. After incubation with 25-D1.16 antibody, the amount of cross-presented $OVA_{257-264}$ was quantified by flow cytometry.

Fig. 16: $OVA_{257-264}$ MHC class I presentation after 6 h incubation of H2-Kb positive cells with 5 μM solution of native or elongated $OVA_{257-264}$ epitope or full length OVA protein with or without $SiO_2$-$PO_3H_2$. Quantification was carried out by flow cytometry after labeling with 25-D1.16 detection antibody.

As shown in **Fig. 16**, only the native epitope and the elongated epitope with an enzymatic cleavage site are presented in significant amounts on the MHC. The uptake and thus the amount of presented epitope can be increased slightly by incubation with peptides adsorptively bound to nanoparticles. While the native epitope does not necessarily have to be internalized into the cell, since it can also be loaded exogenously onto the MHC molecule, the N-terminal elongated epitope must be internalized to release the native sequence. The detection of the native epitope on MHC class I after incubation of the cells with RW-Cit-$OVA_{257-264}$ confirms a proof of the functionality of the enzymatic cleavage site.

## EXAMPLE 16

### Stability Measurement in Human Serum

HEK-Blue™ hTLR3 Cells are designed to measure the stimulation of human TLR3 by monitoring the activation of NF-kB. HEK-Blue™ hTLR3Cells were obtained by co-transfection of the hTLR3 gene and an optimized secreted embryonic alkaline phosphatase (SEAP) reporter gene placed under the control of an NF-kB and AP-1-inducible promoter intoHEK293 cells. Stimulation with a TLR3 ligand activates NF-kB and AP-1 which induce the production of SEAP. Levels of SEAP can be easily determined with HEK-Blue™ Detection, a cell culture medium that allows for real-time detection of SEAP. The hydrolysis of the substrate by SEAP produces a purple/blue color that can be easily detected with the naked eye or measured with a 96 well plate reader. (Invivogen, see https://www.invivogen.com/hek-blue-htlr3).

Poly(I:C) and poly(I:C)@$SiO_2$-Arg were exposed for 60 minutes at 37 °C to human serum (HS). The serum was used in concentrations of 5, 10 and 20 %. A serum concentration of 20 % corresponds quite well to the composition of peripheral lymph.

Poly(I:C) and poly(I:C)@$SiO_2$-Arg were added separately to human serum to get a concentration of 1 μg poly(I:C)/mL. In case of poly(I:C)@$SiO_2$-Arg the $SiO_2$ concentration was 40 μg/mL. The poly(I:C) payload at $SiO_2$-Arg in this case was about 2.5 %. After exposure to HS 20 μL of the medium were taken and added to 180 μL HEK-Blue™hTLR3 cells in HEK-Blue™ Detection medium. This mixture was incubated for 13 hours at 37 °C and the plate was analyzed in a 96 well plate reader (Tecan Reader, Type: Infinite M200 Pro).

The results are shown in **Fig. 17.**

The calculated half-life of free poly(I:C) in 20 % human serum under the chosen conditions is 24.2 minutes (18 % of initial concentration after 60 minutes). Half-life calculation:

$$t_{½} = \ln 2 / (\ln 100 / \ln 18) * 60 \qquad [min]$$

The half-life for poly(I:C)@$SiO_2$-Arg under the same conditions is calculated to 395 minutes (90 % of initial concentration after 60 minutes)

$$t_{½} = \ln 2 / (\ln 100 / \ln 90) * 60 \qquad [min]$$

So the attachment of poly(I:C) to arginylated silica nanoparticles increases the half-life by a factor of 16 (=395 / 24.2).

## EXAMPLE 17

### Stability Measurement in Bovine Serum

HEK-Blue™ hTLR3 Cells are designed to measure the stimulation of human TLR3 by human TLR3 by monitoring the activation of NF-kB. HEK-Blue™hTLR3Cells were obtained by co-transfection of the hTLR3 gene and an optimized

secreted embryonic alkaline phosphatase (SEAP) reporter gene placed under the control of an NF-kB and AP-1-inducible promoter intoHEK293 cells. Stimulation with a TLR3 ligand activates NF-kB and AP-1 which induce the production of SEAP. Levels of SEAP can be easily determined with HEK-Blue™ Detection, a cell culture medium that allows for real-time detection of SEAP. The hydrolysis of the substrate by SEAP produces a purple/blue color that can be easily detected with the naked eye or measured with a 96 well plate reader (Invivogen).

Poly(I:C) and poly(I:C)@$SiO_2$-Arg were exposed for 60 minutes at 37 °C to fetal bovine serum (FBS). The serum was used in concentrations of 5, 10 and 20 %. A serum concentration of 20 % corresponds quite well to the composition of peripheral lymph.

Poly(I:C) and poly(I:C)@$SiO_2$-Arg were added separately to bovine serum to get a concentration of 1 μg poly(I:C)/mL. In case of poly(I:C)@$SiO_2$-Arg the $SiO_2$ concentration was 40 μg/mL. The poly(I:C) payload at $SiO_2$-Arg in this case was about 2.5 %. After exposure to FBS 20 μL of the medium were taken and added to 180 μL HEK-Blue™ hTLR3 cells in HEK-Blue™ Detection medium. This mixture was incubated for 13 hours at 37 °C and the plate was analyzed in a 96 well plate reader (Tecan Reader, Type: Infinite M200 Pro).

The results can be seen in **Fig. 18:**

While the free poly(I:C) shows clear signs of degradation at higher serum concentrations, poly(I:C)@$SiO_2$-Arg shows markedly higher stability. Half-life calculation for poly(I:C)@$SiO_2$-Arg is useless, due to a very low decay rate.

**Claims**

1. Composition comprising nanoparticles which are loaded with pharmaceutically acceptable compounds comprising one or more human papilloma virus (HPV)-derived immunogenic fragments or a variant thereof, having silicon dioxide and functional groups on the surface, wherein

    - the functional groups are capable to carry and/or stabilize both negative and positive charges of the pharmaceutically acceptable compounds,
    - the zeta potential of the composition has a value of at least ± 15 mV,
    - the nanoparticles have a particle size below 150 nm.

2. Composition according to claim 1 **characterized in that** the immunogenic fragment is derived from the E6 and/or E7 proteins of HPV16 and/or HPV18, preferably selected from SEQ ID NOs: 5-257.

3. Composition according to any of claims 1 or 2 **characterized in that** the immunogenic fragment is at least 75% identical to an immunogenic fragment derived from the E6 and/or E7 proteins of HPV16 and/or HPV18, preferably selected from SEQ ID NOs: 258-295.

4. Composition according to any of claims 1 to 3 **characterized in that** the immunogenic fragment is able to bind to major histocompatibility complex (MHC) class I and/or MHC class II.

5. Composition according to any of claims 1 to 4 **characterized in that** the immunogenic fragment is able to induce a cytotoxic T cell response.

6. Composition according to any of claims 1 to 5, wherein the nanoparticles are loaded with pharmaceutically acceptable compounds comprising one or more human papilloma virus (HPV)-derived immunogenic fragments and polyinosinic:polycytidylic acid (poly(I:C)) or any derivatives thereof.

7. Composition according to claims 1 to 6, wherein the nanoparticles have a surface loading density up to 0.5, preferably between 0.01 and 0.5, more preferred between 0.03 and 0.4, most preferred between 0.05 to 0.3, in relation to the total number of the pharmaceutically acceptable compounds with regard to the surface of the nanoparticle in $nm^2$ [molecules/$nm^2$].

8. Composition according to any of the preceding claims, wherein the net charge of the loaded nanoparticles in total is different to zero.

9. Composition according to any of the preceding claims, wherein the functional groups are connected to a linker L which is linked to the surface of the nanoparticles by way of a covalent or adsorptive bond.

10. Composition according to claim 9, wherein the linker compound L comprises at least one carboxyl (-COOH) or carboxylate ($-COO^-$) group as functional group.

11. Composition according to claim 9 or 10, wherein the linker compound L comprises at least one guanidino-group ($-NHC(=NH)NH_2$ or $-NHC(=NH_2^+)NH_2$) or amino-group ($-NH_2$ or $-NH_3^+$) group as a functional group.

12. Composition according to any of the preceding claims, wherein the pharmaceutically acceptable compound is conjugated to the nanoparticle by adsorptive attachment.

13. Composition according to any of the preceding claims for use as vaccines or as immunostimulant.

14. Composition according to any of the preceding claims for use as vaccines for the prevention of HPV infection or the treatment of HPV positive humans or the treatment of HPV positive tumors.

15. Vaccine comprising compositions according to any of the preceding claims.

Figure 1a

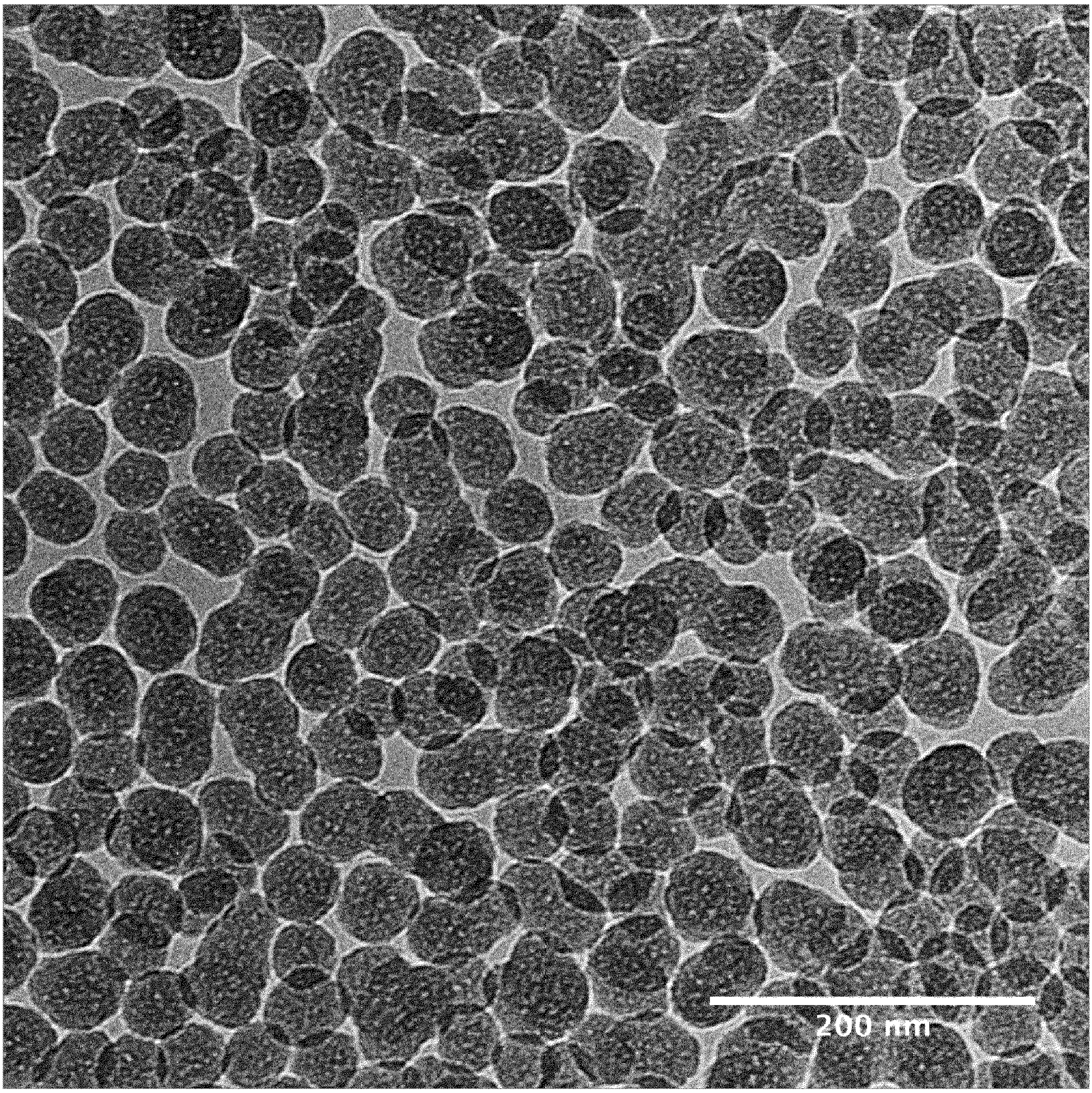

Figure 1b

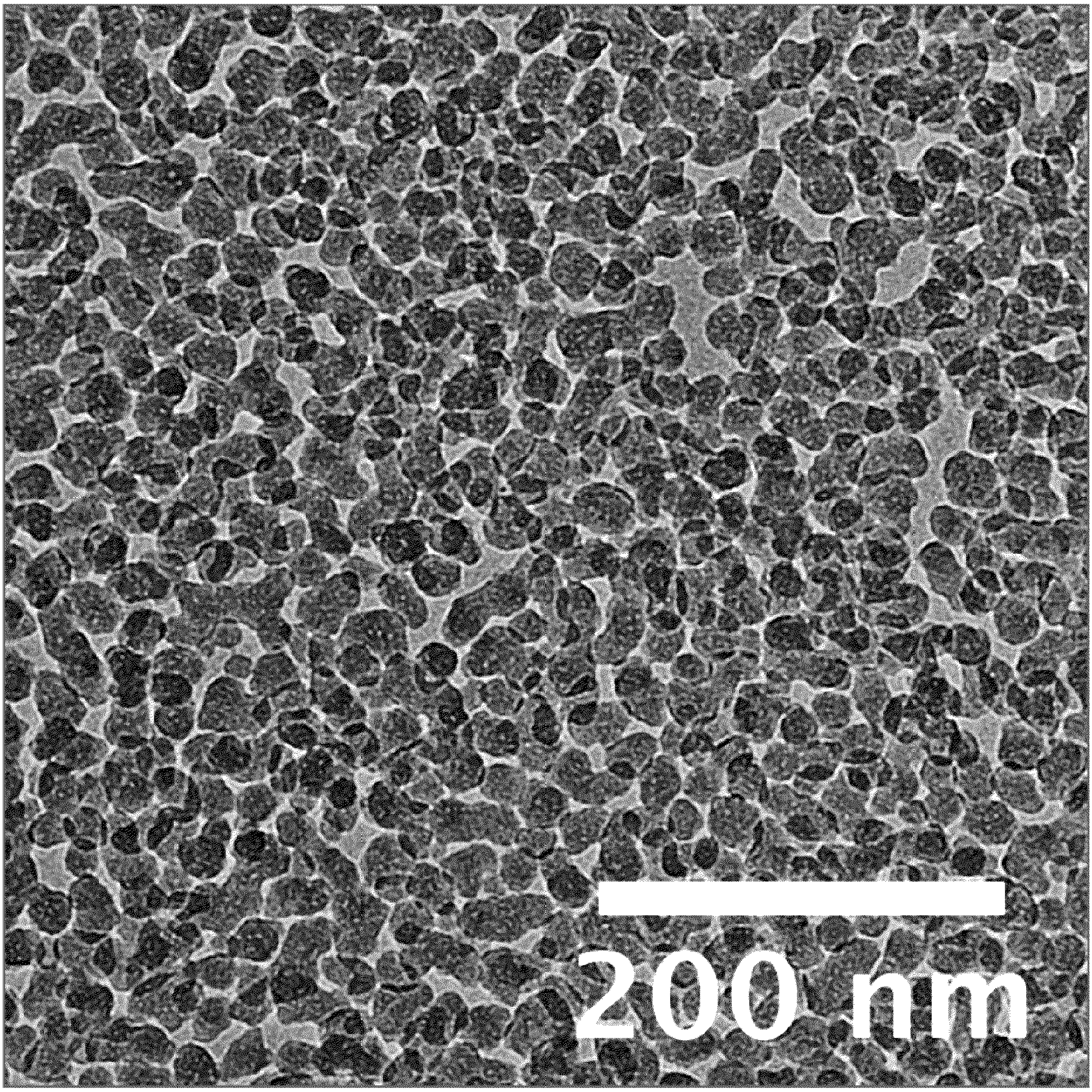

Figure 1c

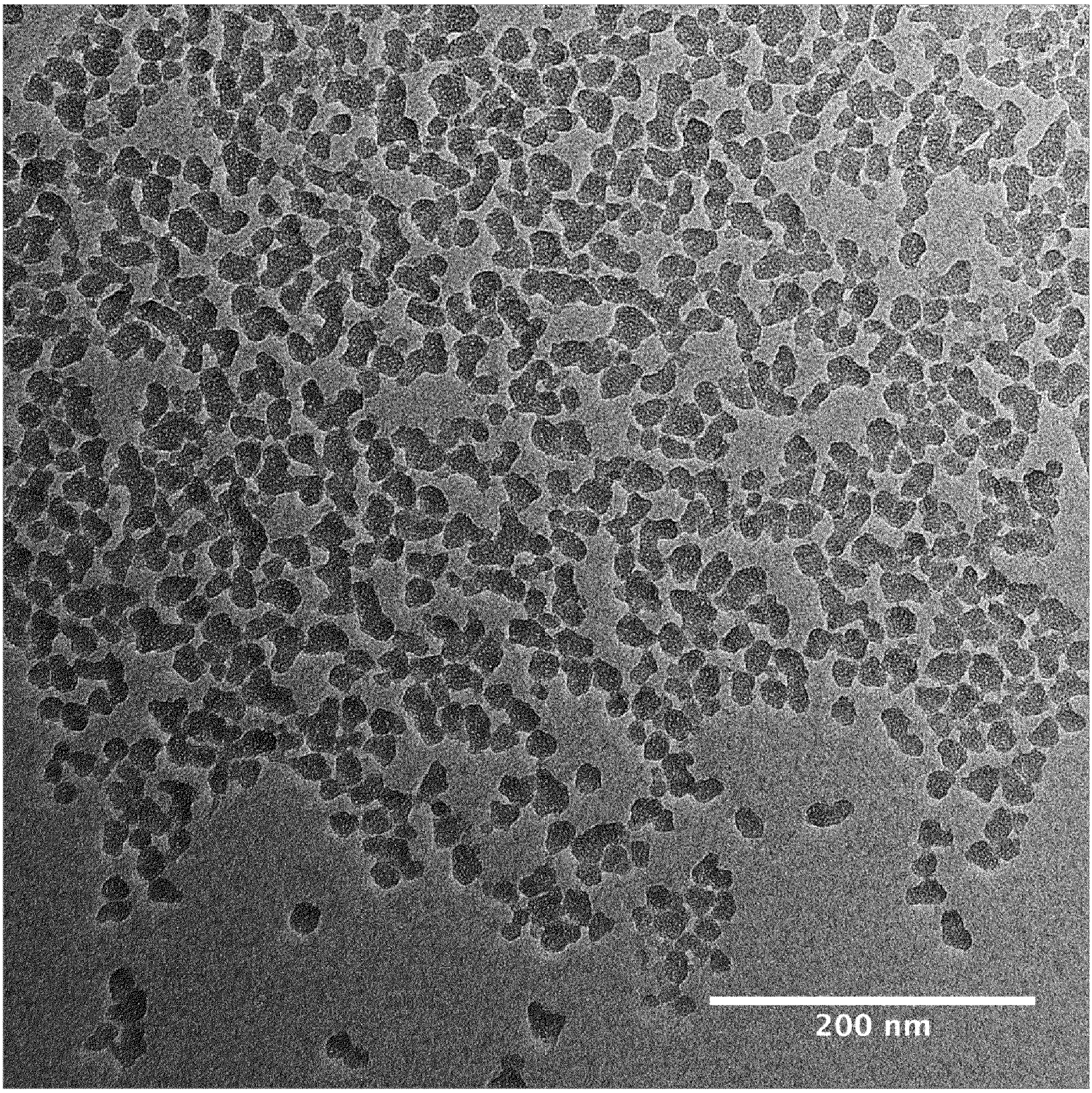

Figure 1d

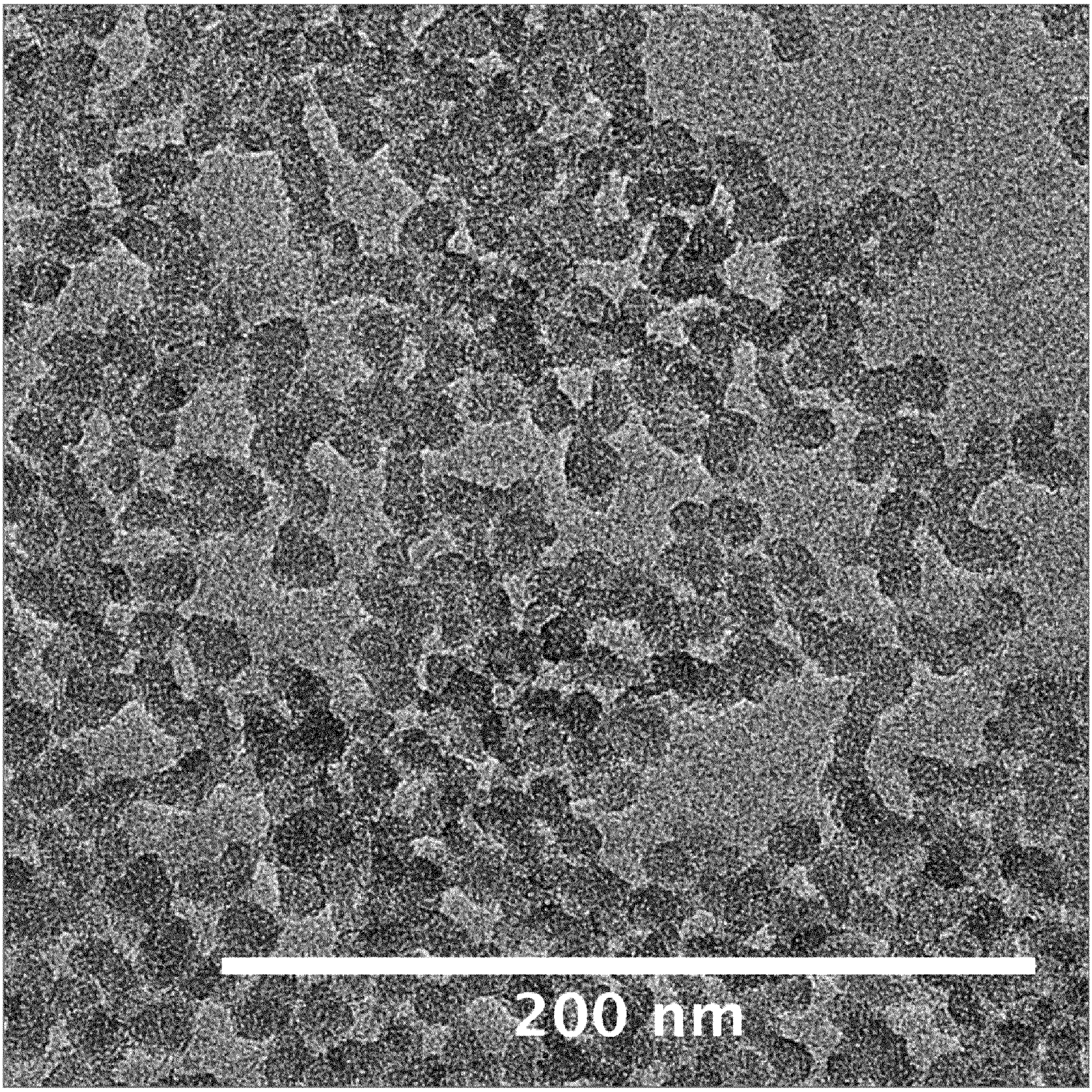

Figure 2

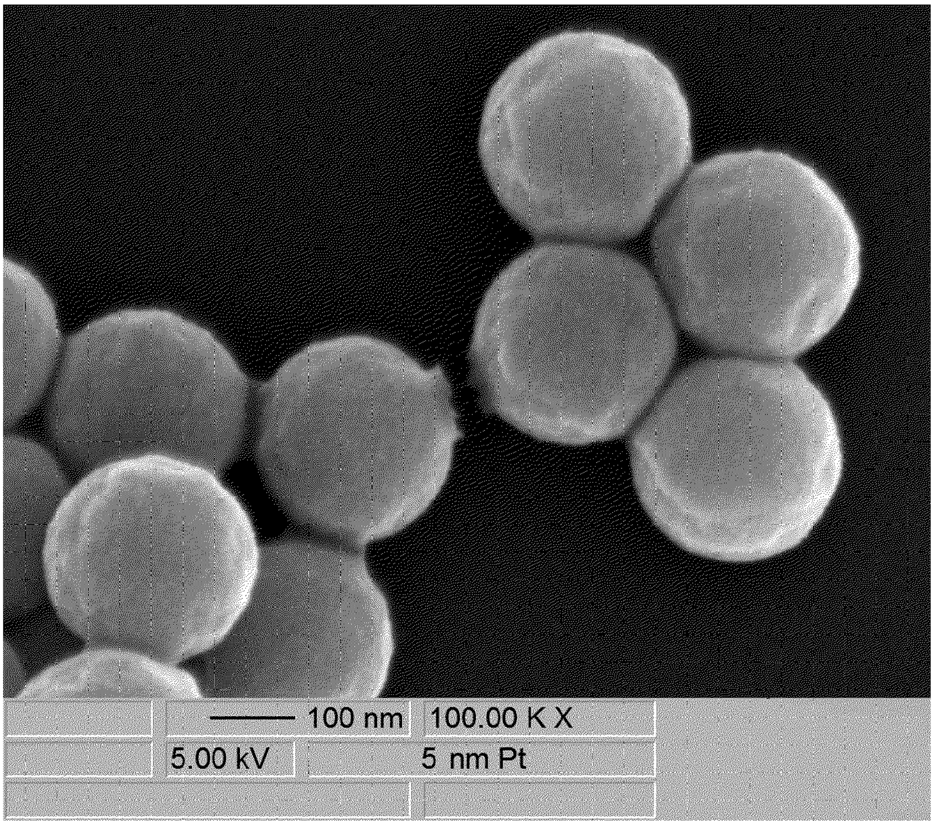


Figure 3

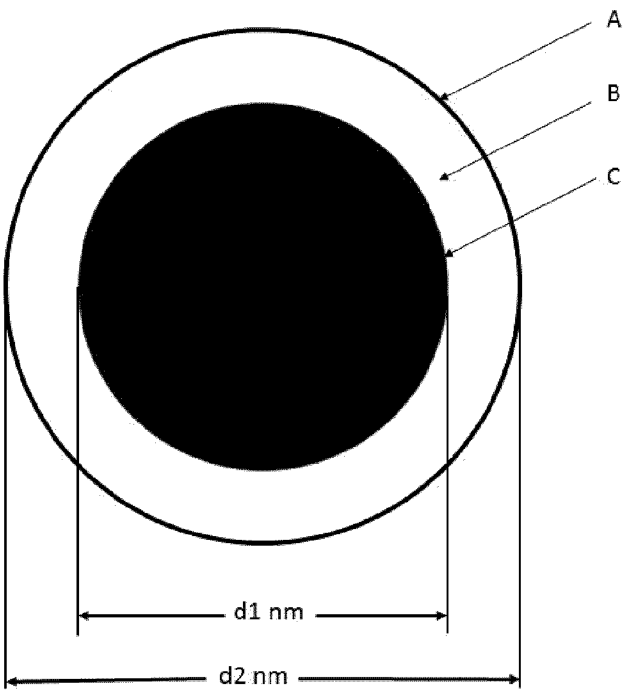

**Figure 4a**

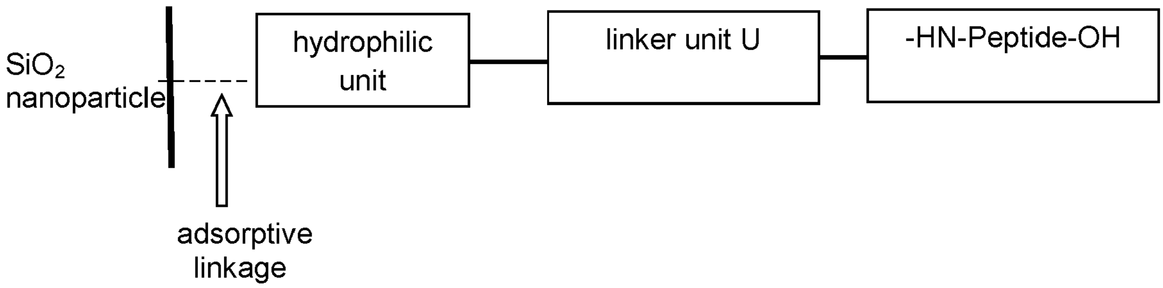


**Figure 4b**

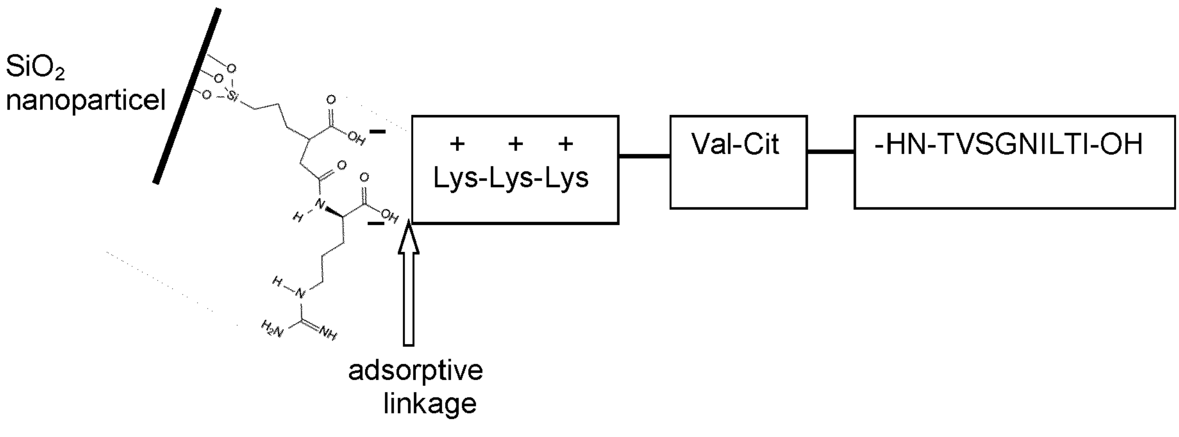

## Figure 5a

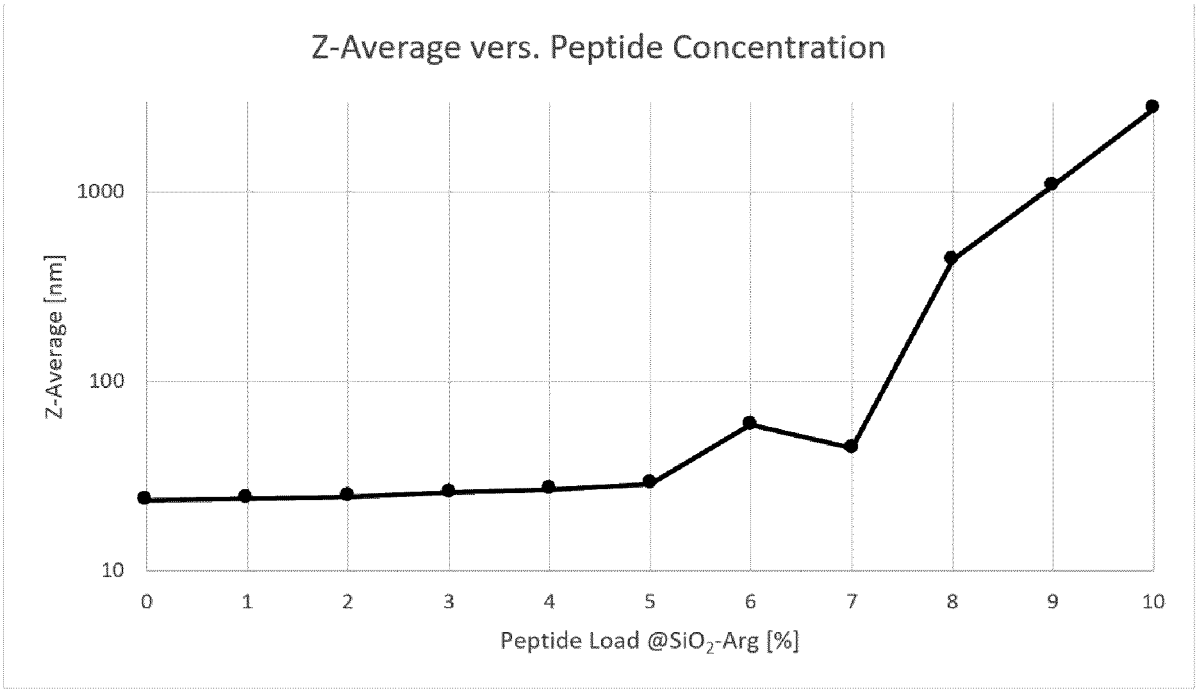


## Figure 5b

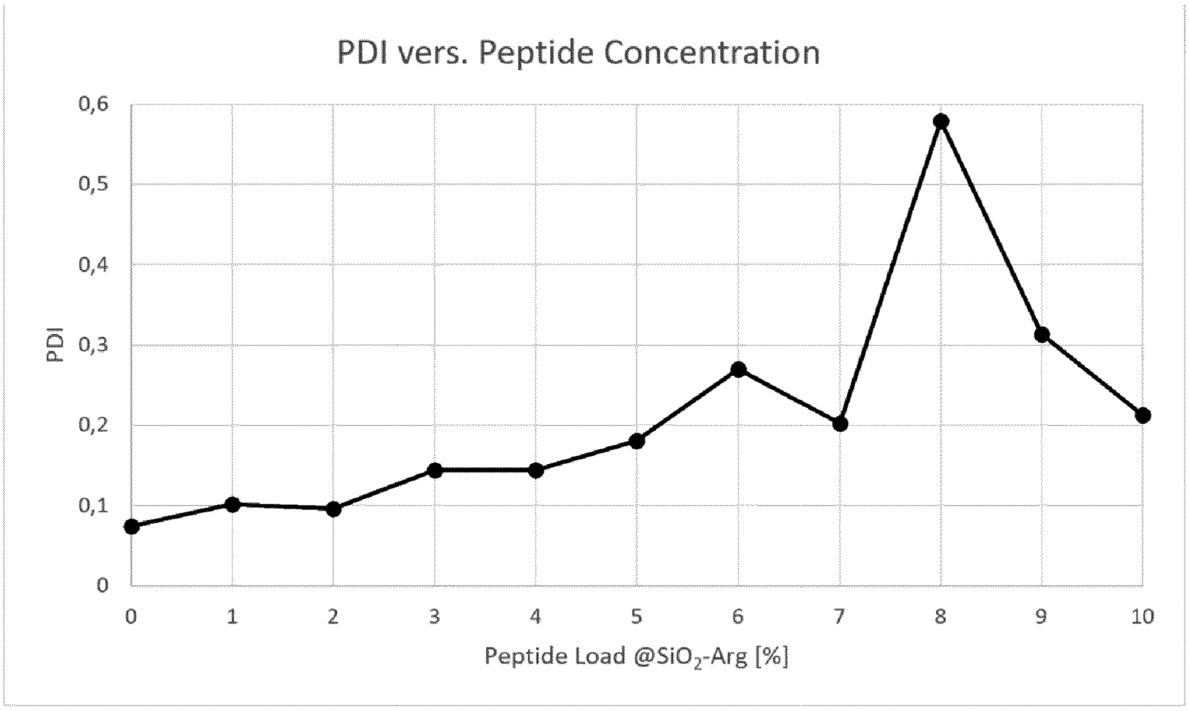

## Figure 6a

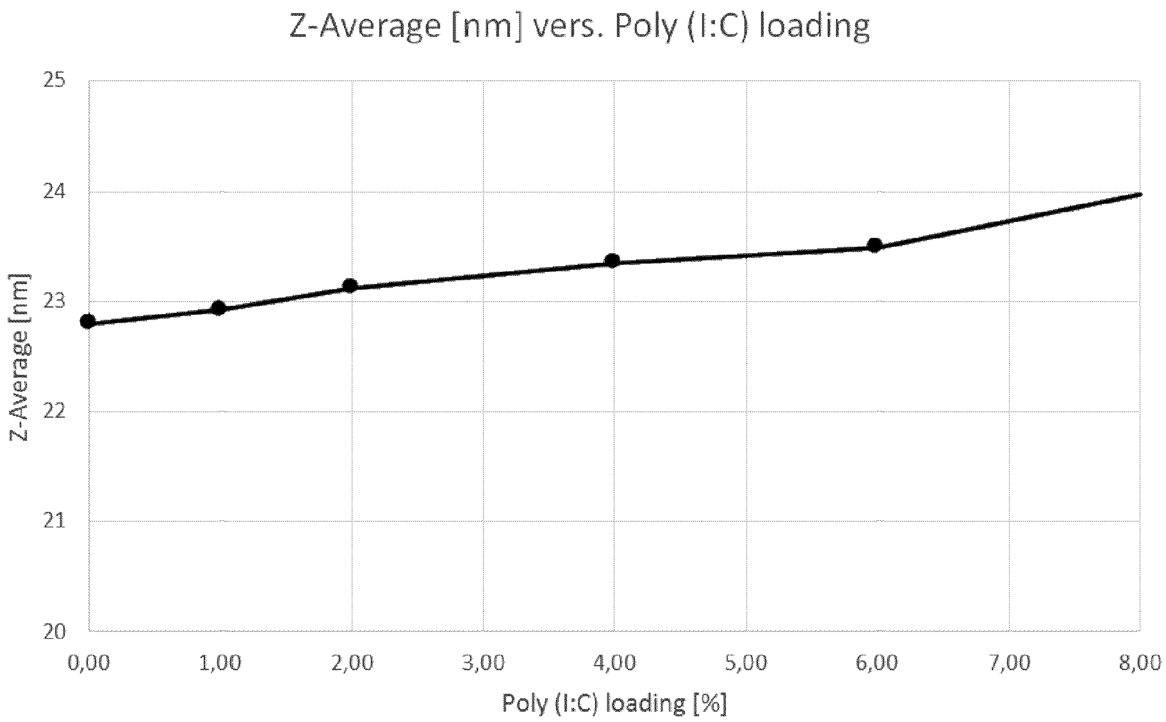


## Figure 6b

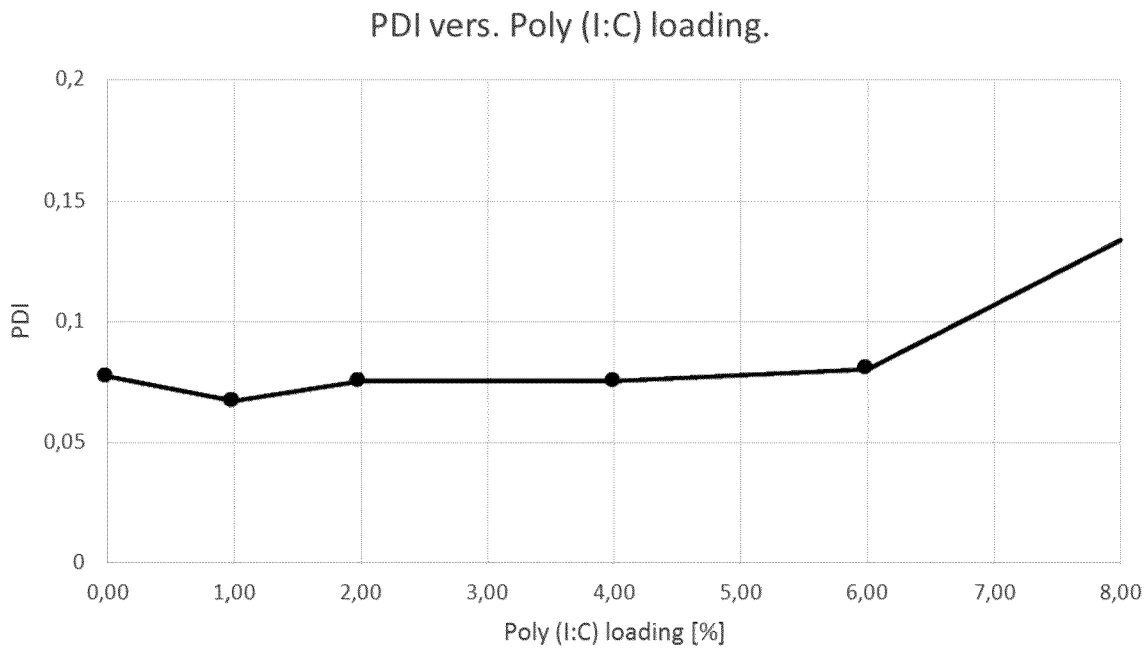

## Figure 7a

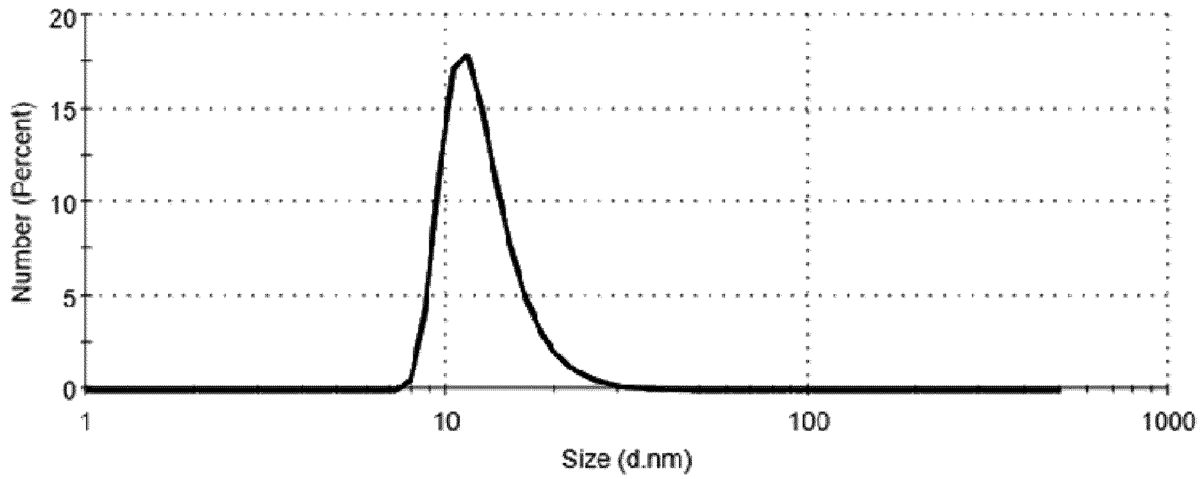


## Figure 7b

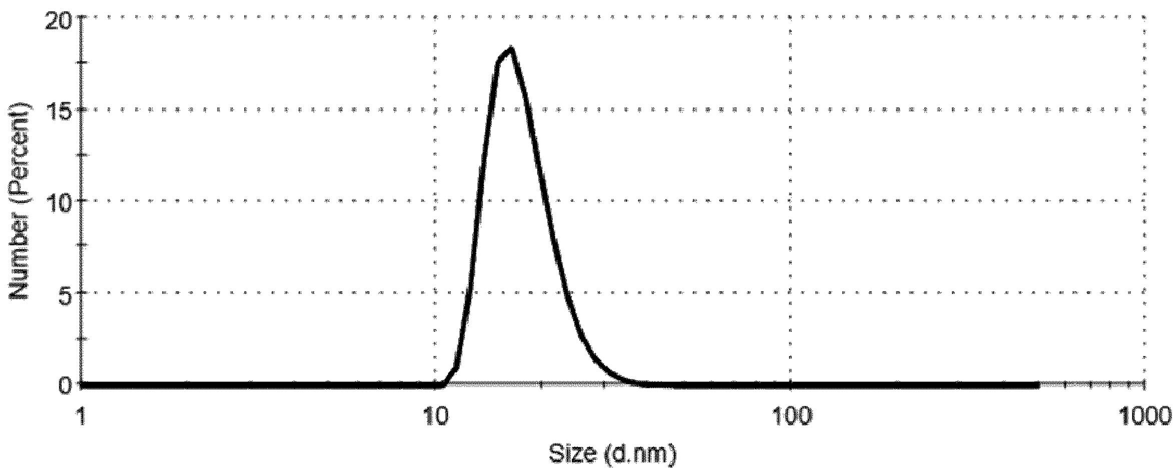

# Figure 7c

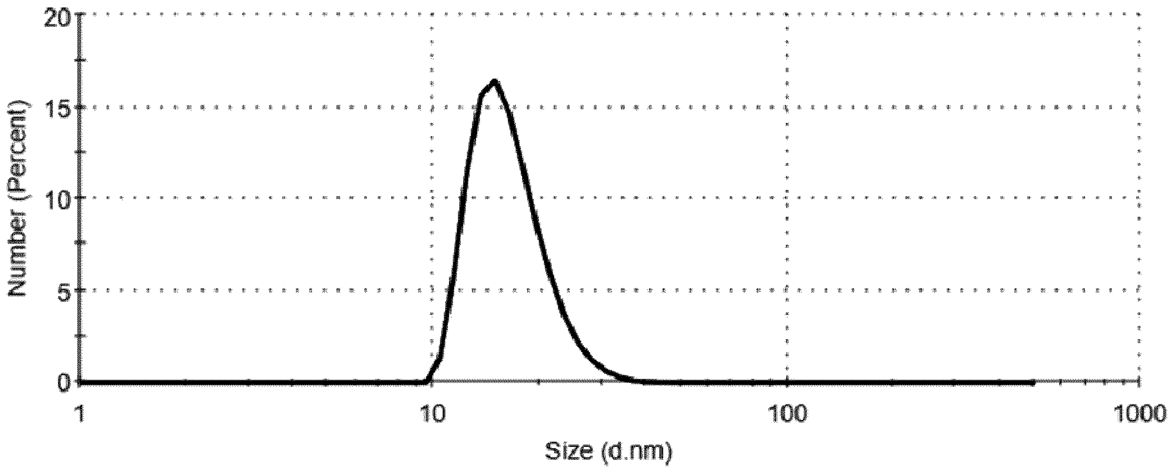


# Figure 7d

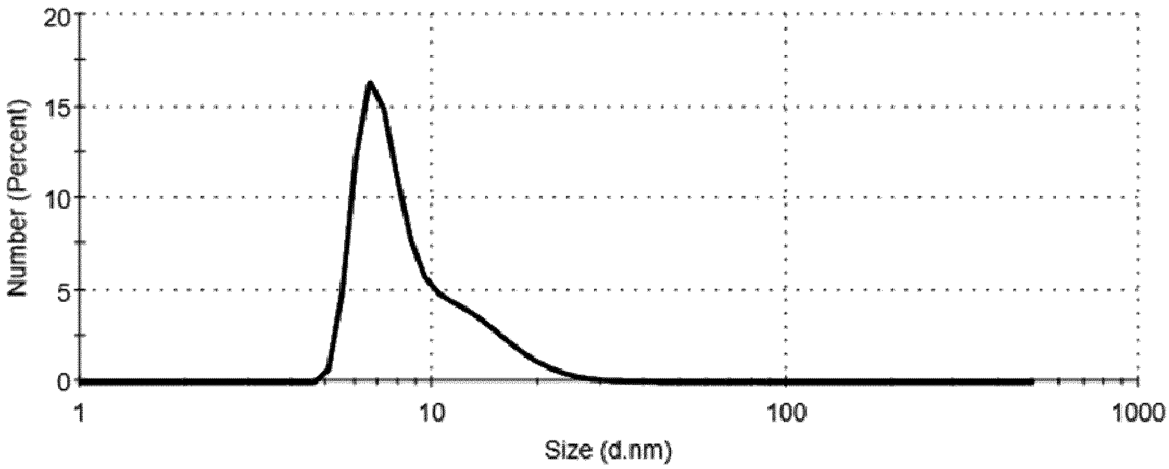

# Figure 8

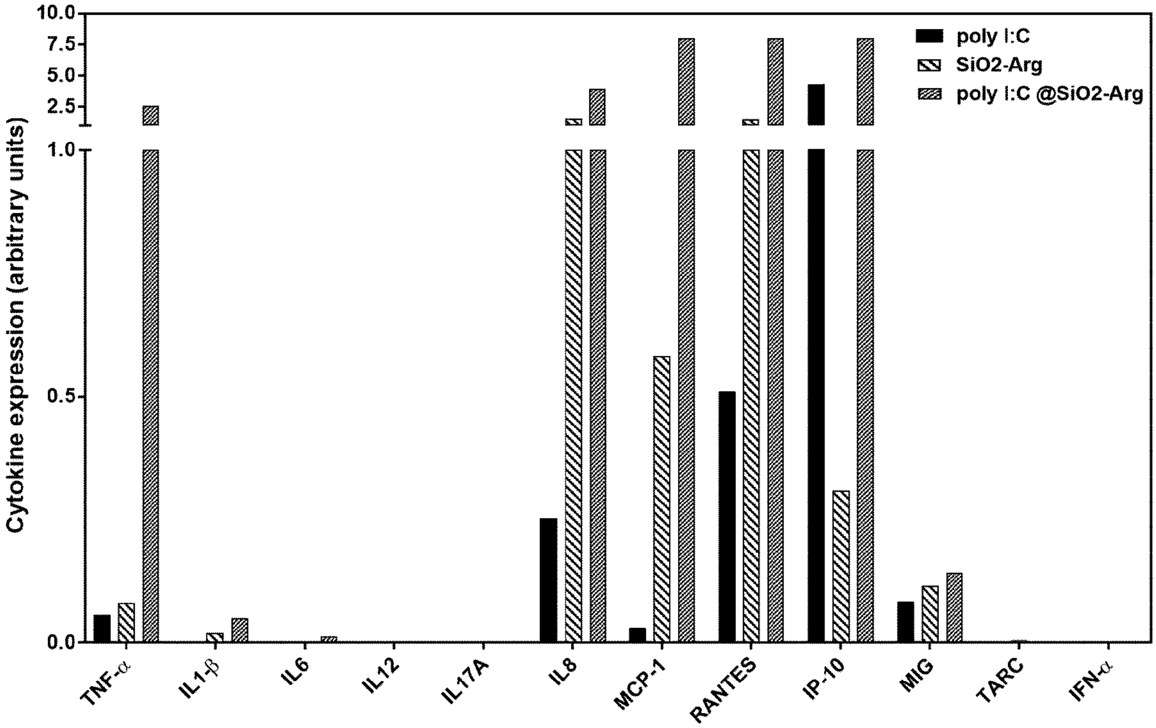


# Figure 9

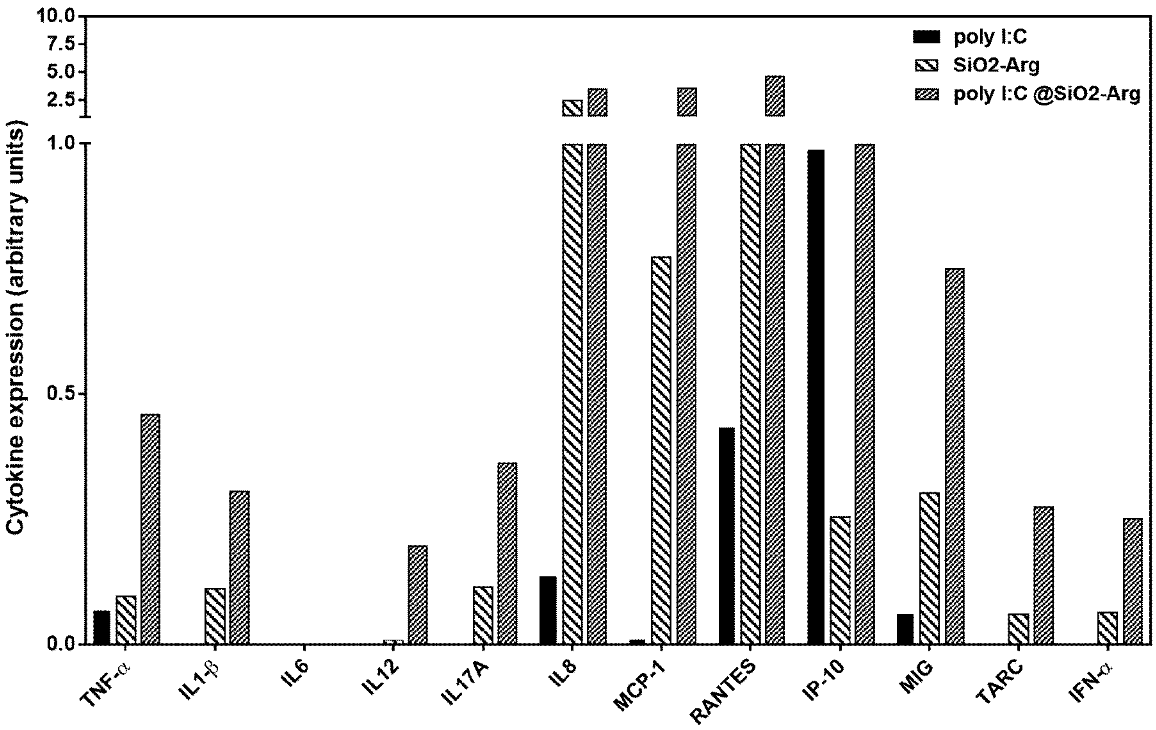

**Figure 10a**

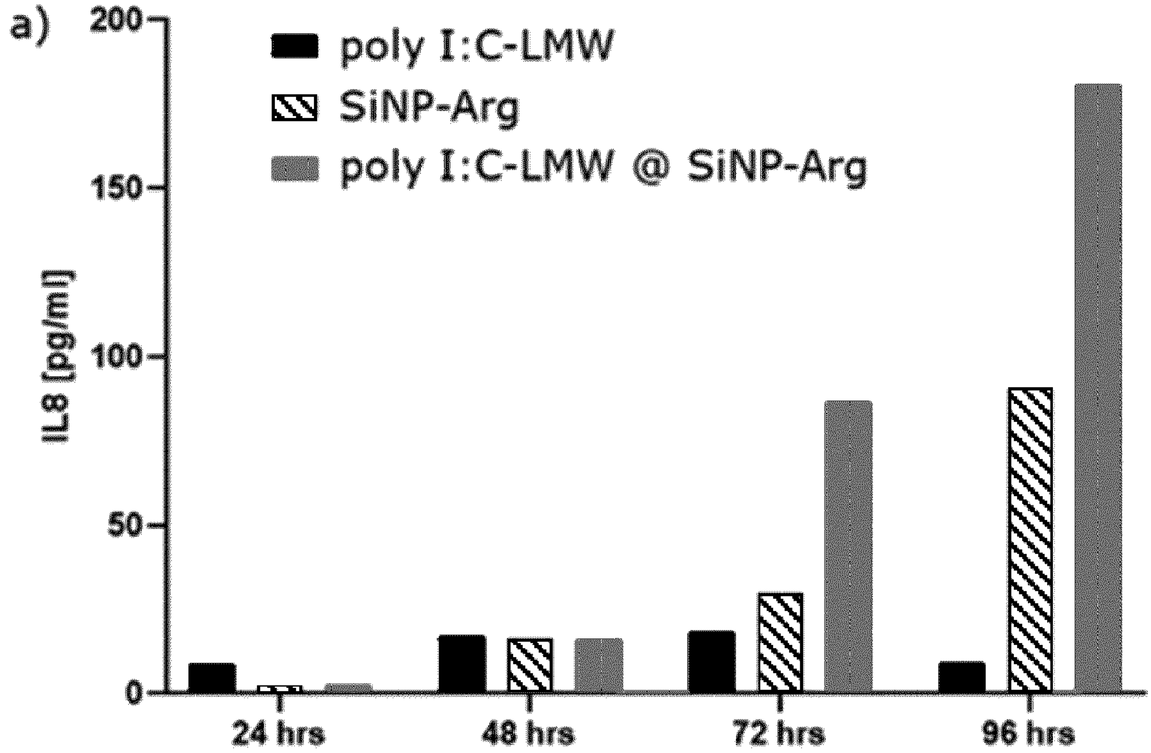


**Figure 10b**

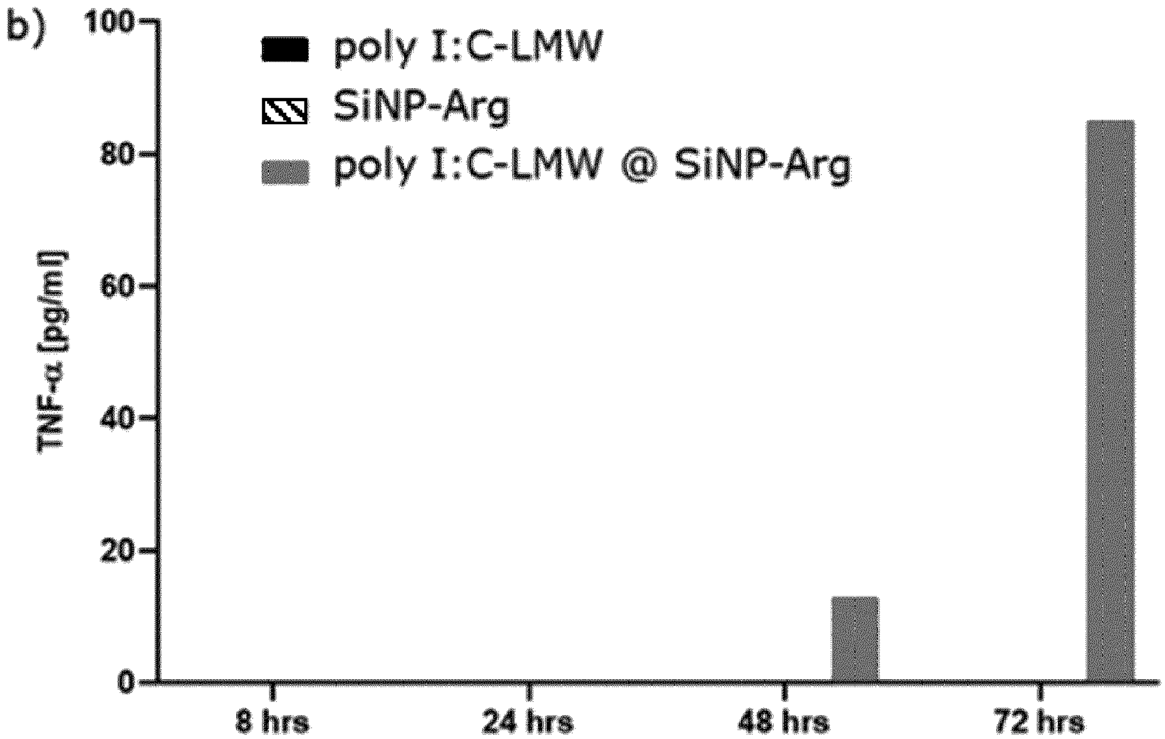

**Figure 11a**

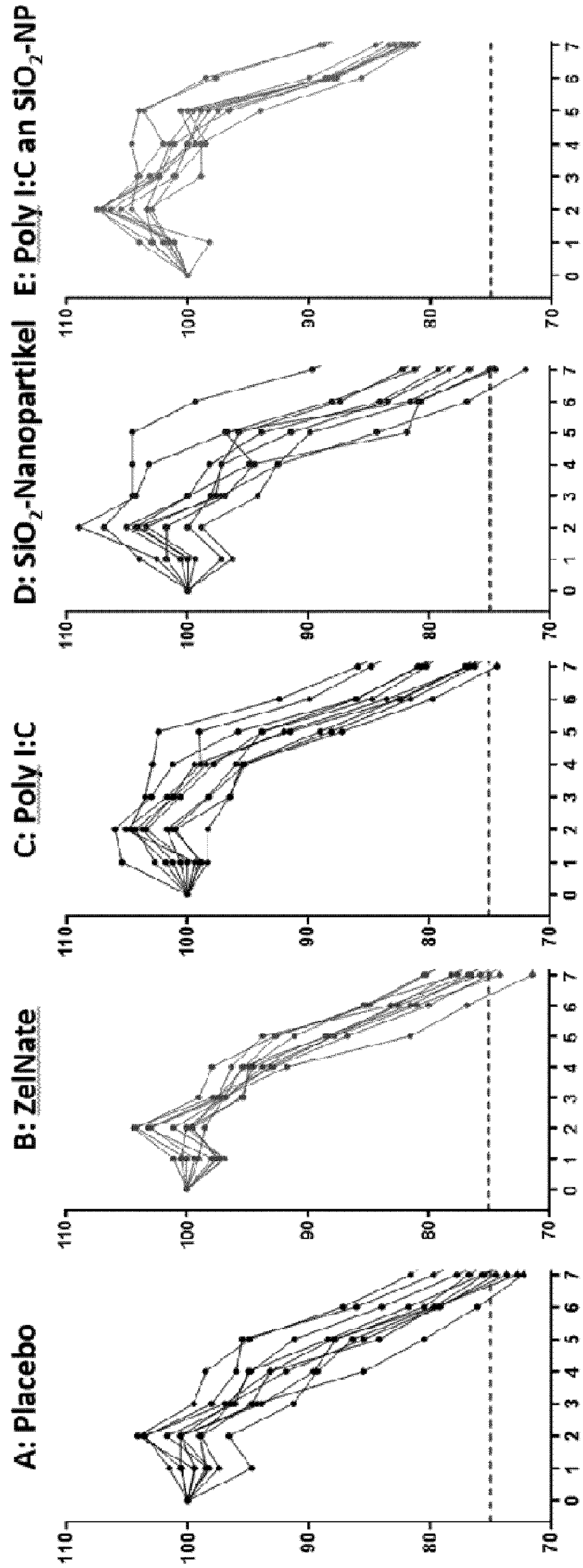
A: Placebo
B: ZelNate
C: Poly I:C
D: $SiO_2$-Nanopartikel
E: Poly I:C an $SiO_2$-NP
110
100
90
80
70
0 1 2 3 4 5 6 7

# Figure 11b

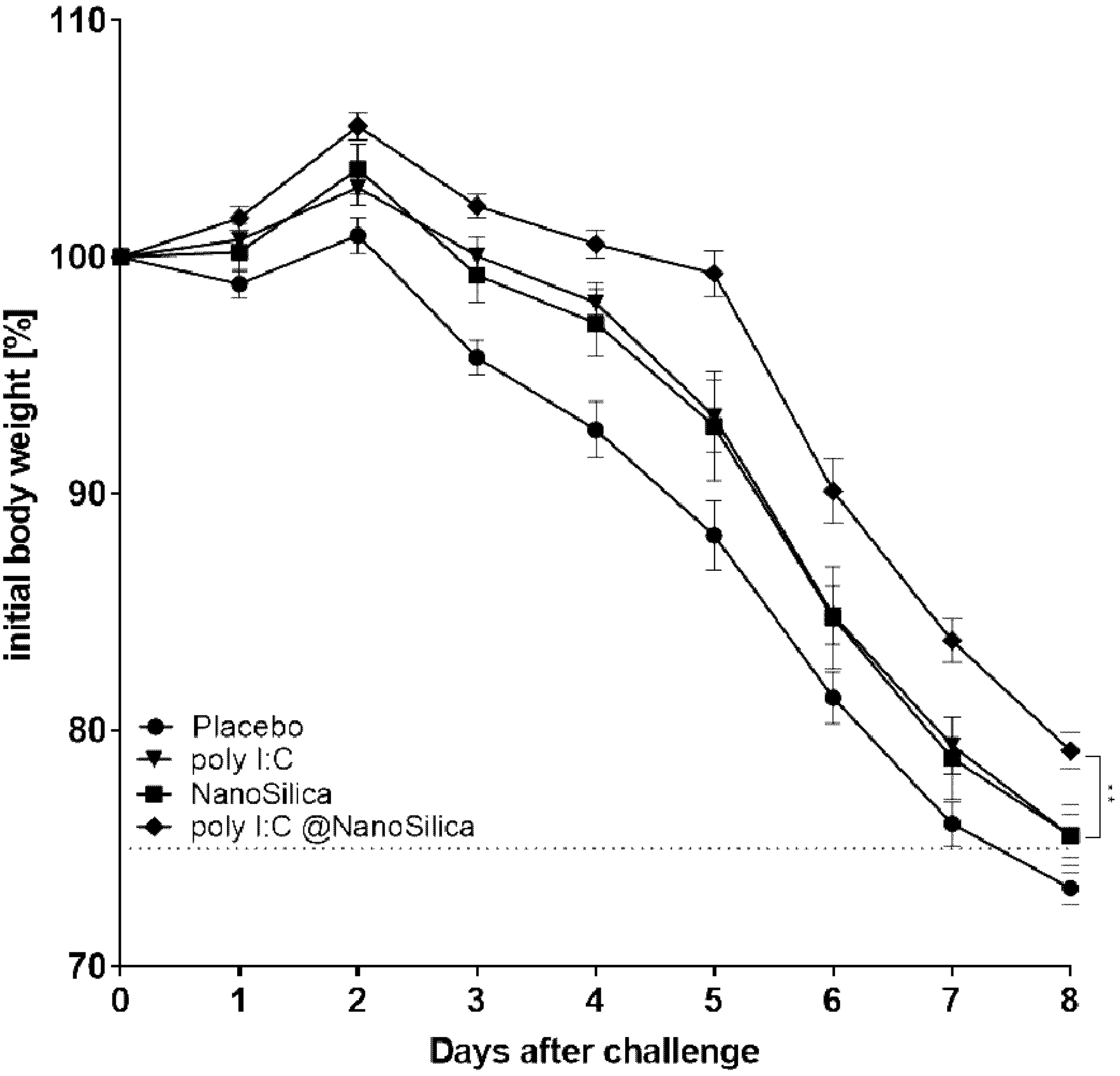
110
100
90
80
70
initial body weight [%]
0
1
2
3
4
5
6
7
8
Days after challenge
Placebo
poly I:C
NanoSilica
poly I:C @NanoSilica
**

# Figure 12

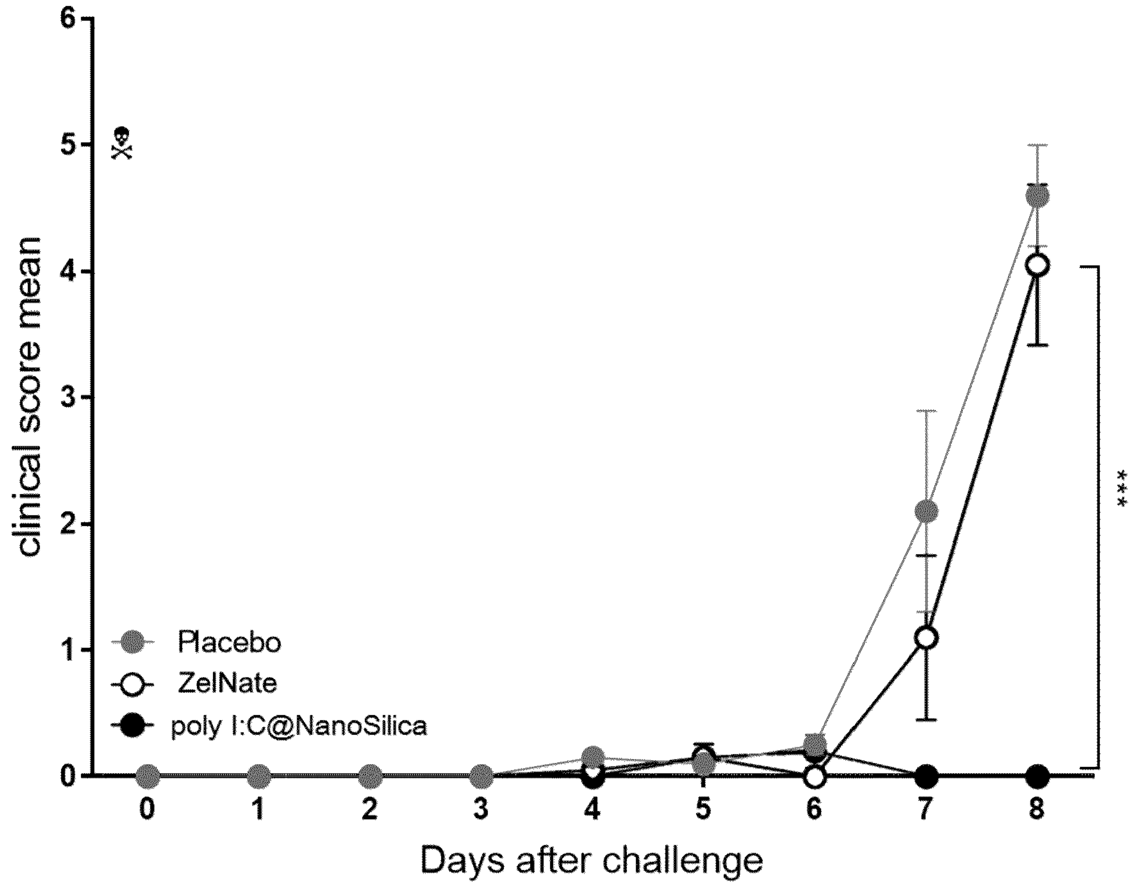
6
5
4
3
2
1
0
clinical score mean
Placebo
ZelNate
poly I:C@NanoSilica
0
1
2
3
4
5
6
7
8
Days after challenge
***

Figure 13

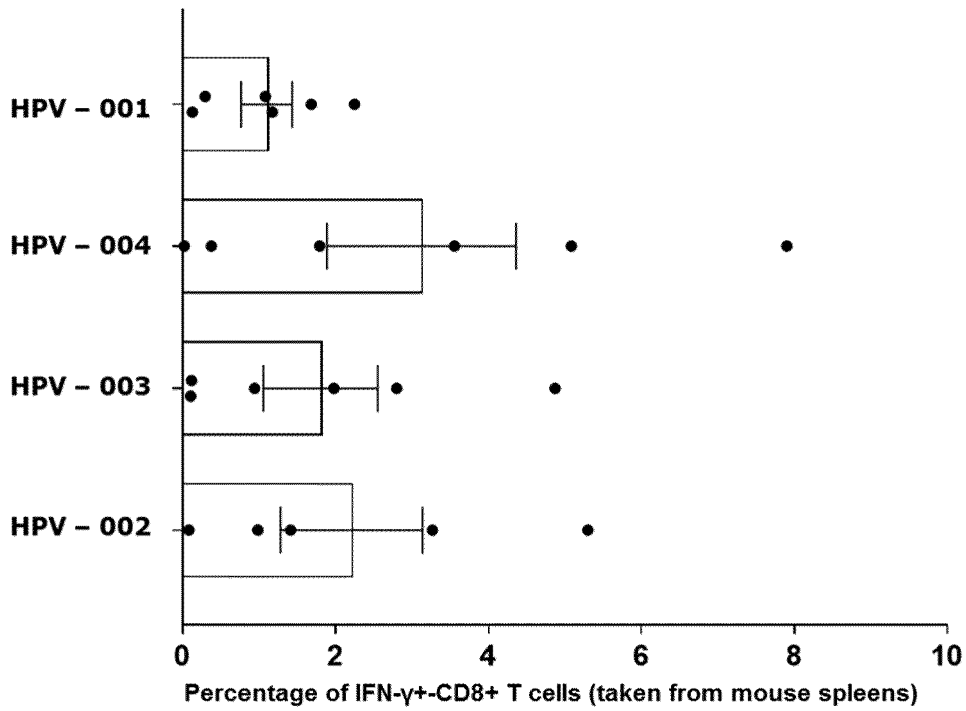
HPV – 001
HPV – 004
HPV – 003
HPV – 002
0
2
4
6
8
10
Percentage of IFN-γ+-CD8+ T cells (taken from mouse spleens)

# Figure 14

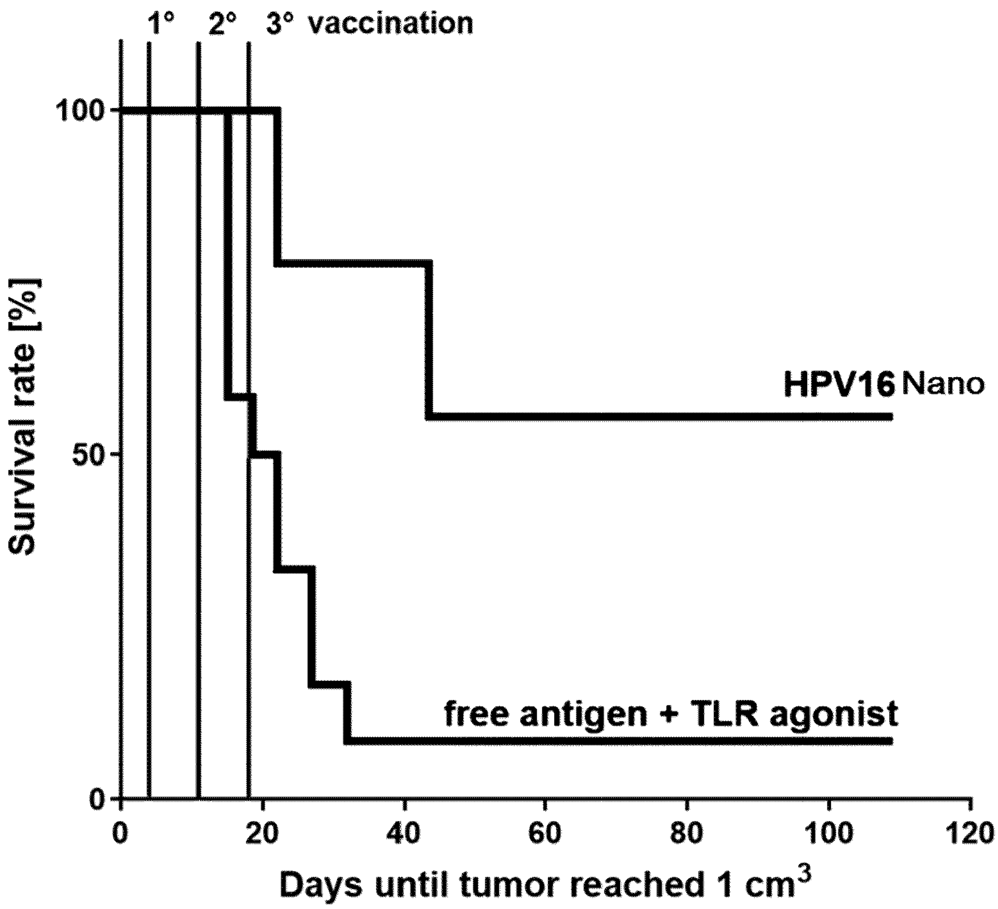
1°
2°
3° vaccination
100
50
0
Survival rate [%]
HPV16 Nano
free antigen + TLR agonist
0
20
40
60
80
100
120
Days until tumor reached 1 cm3

Figure 15a

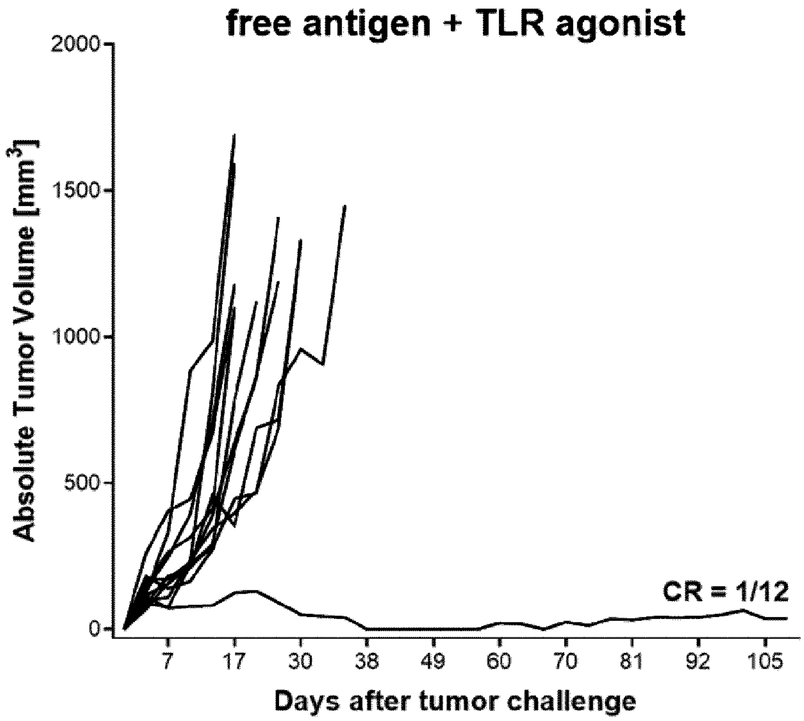


Figure 15b

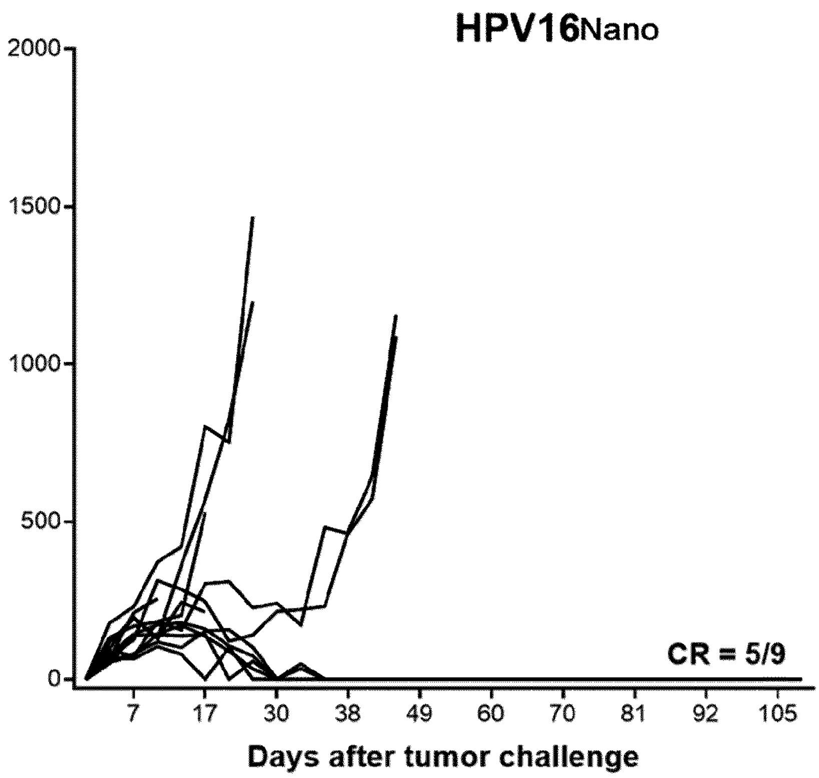

## Figure 16

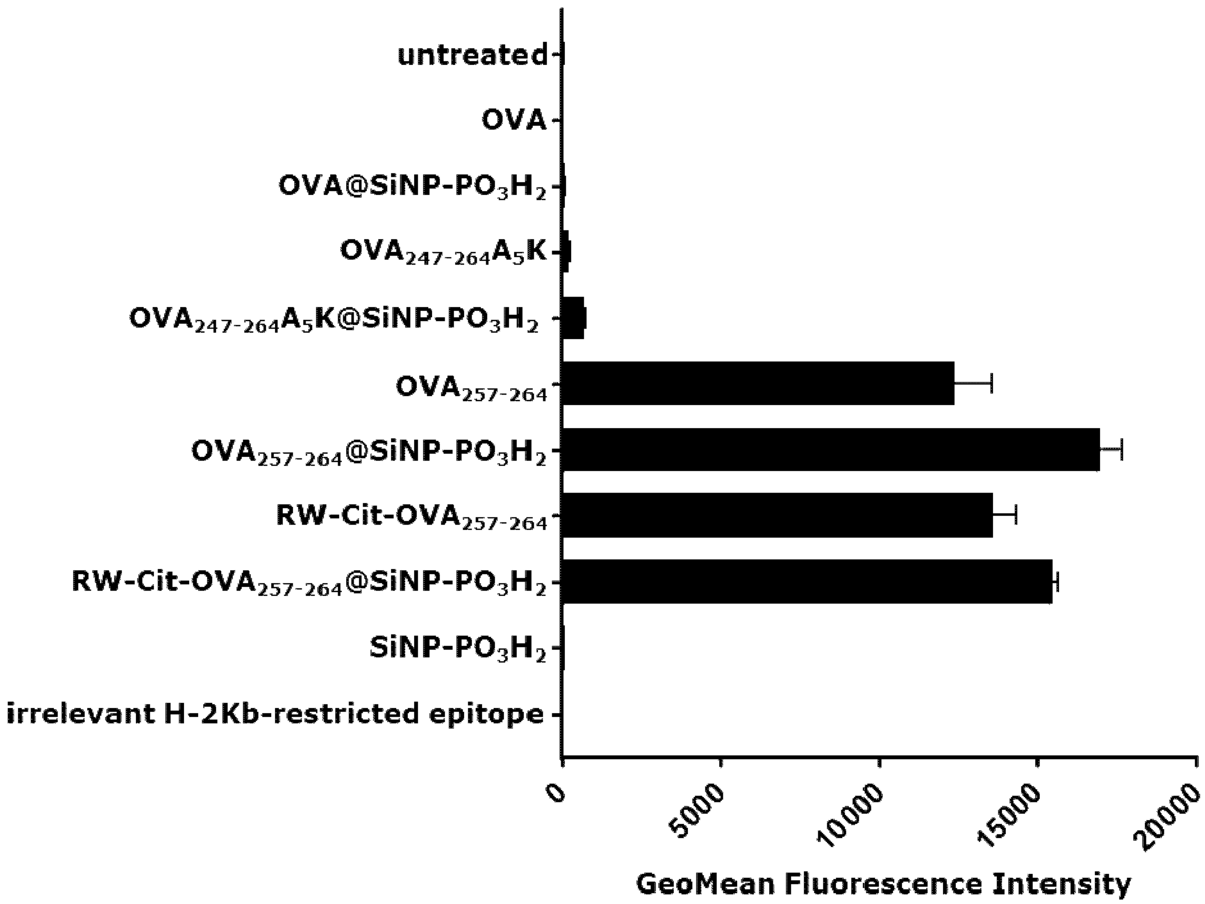
untreated
OVA
OVA@SiNP-PO3H2
OVA247-264A5K
OVA247-264A5K@SiNP-PO3H2
OVA257-264
OVA257-264@SiNP-PO3H2
RW-Cit-OVA257-264
RW-Cit-OVA257-264@SiNP-PO3H2
SiNP-PO3H2
irrelevant H-2Kb-restricted epitope
0
5000
10000
15000
20000
GeoMean Fluorescence Intensity

Figure 17

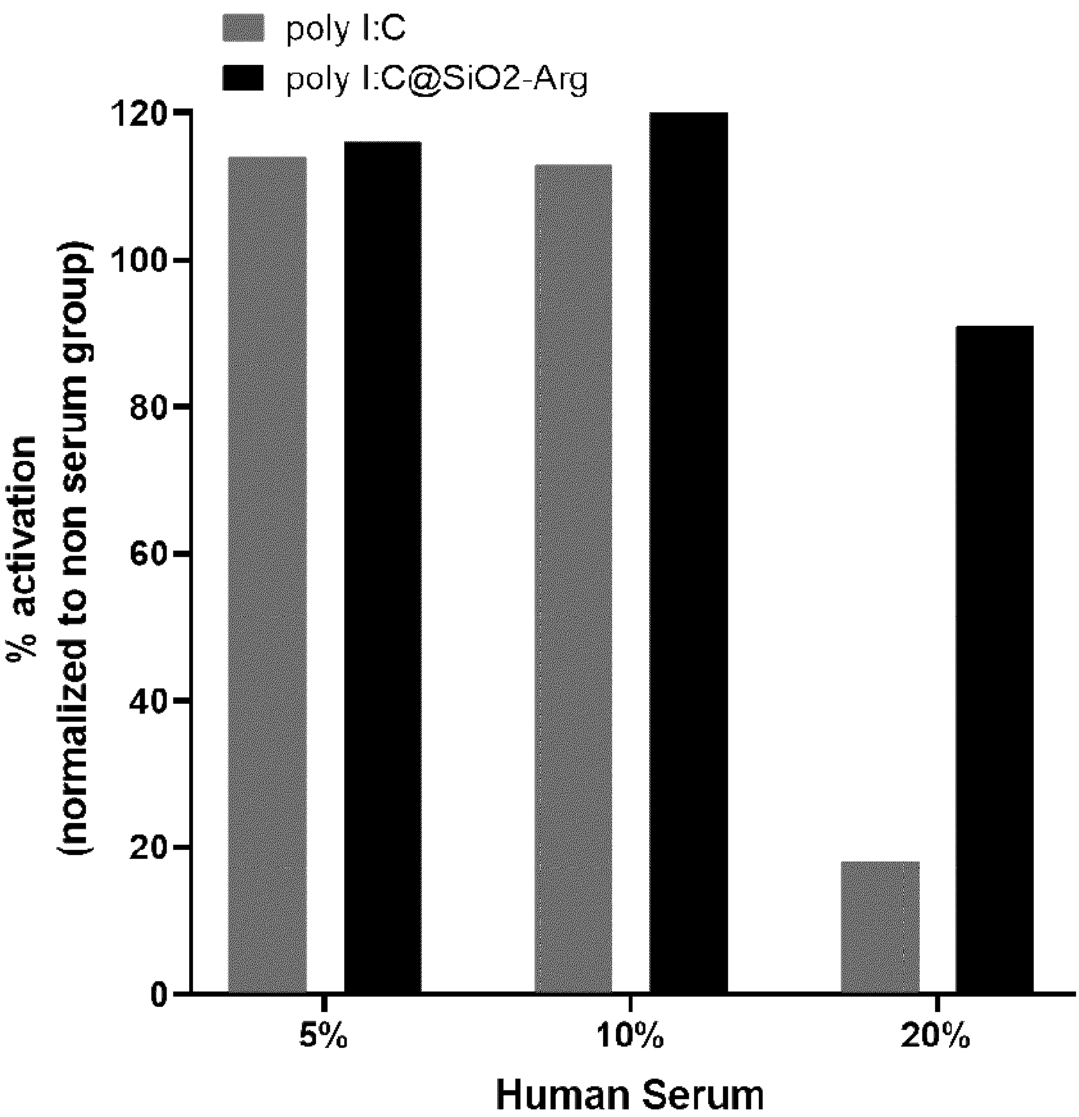
poly I:C
poly I:C@SiO2-Arg
120
100
80
60
40
20
0
% activation
(normalized to non serum group)
5%
10%
20%
Human Serum

Figure 18

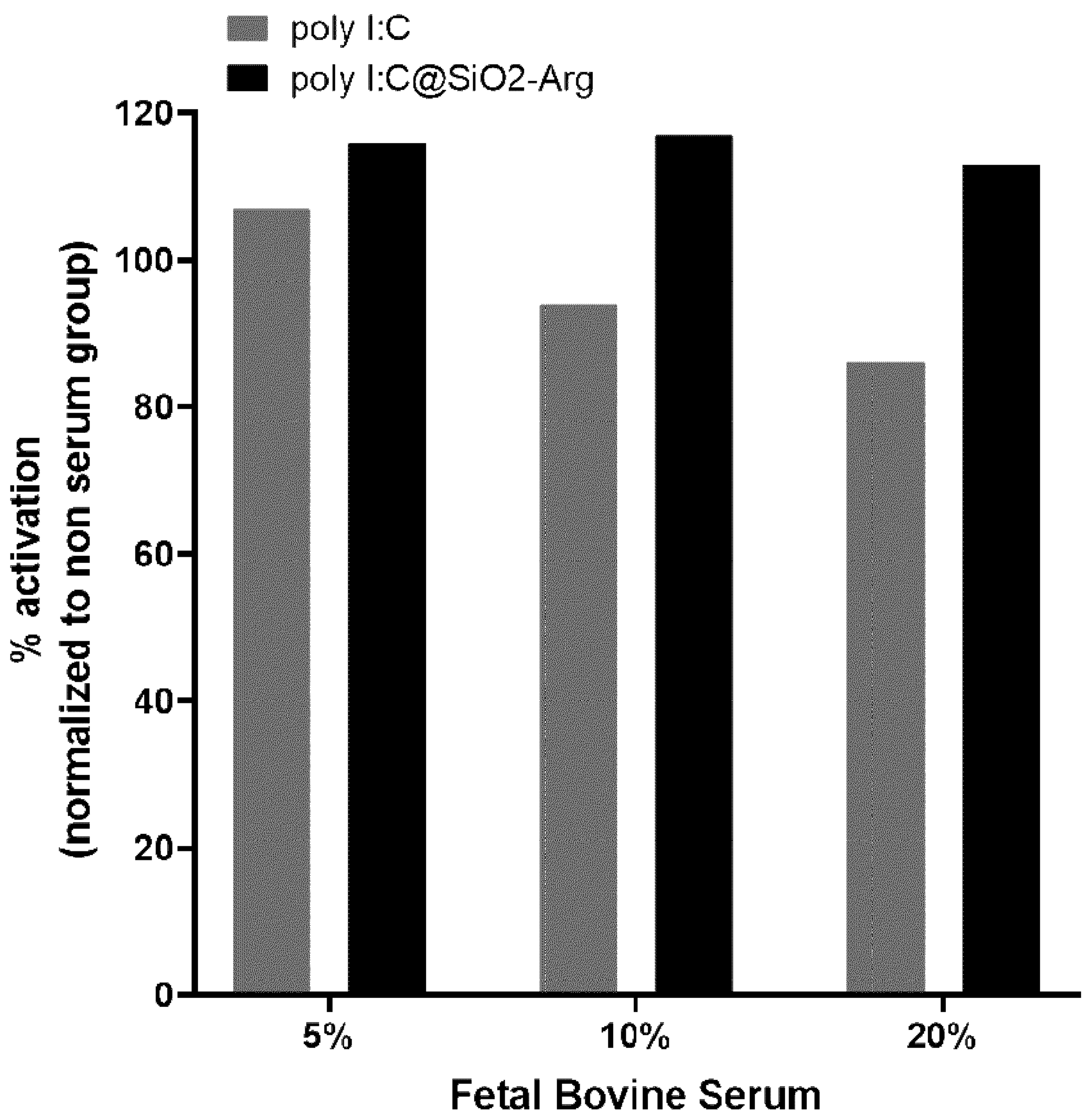
poly I:C
poly I:C@SiO2-Arg
% activation
(normalized to non serum group)
120
100
80
60
40
20
0
5%
10%
20%
Fetal Bovine Serum

Europäisches Patentamt
European Patent Office
Office européen des brevets

# EUROPEAN SEARCH REPORT

Application Number
EP 20 19 6027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2016/090603 A1 (CARNES ERIC CHRISTOPHER [US] ET AL) 31 March 2016 (2016-03-31)<br>* figure 1B *<br>* example 4; tables 2,3 *<br>----- | 1-15 | INV.<br>A61K47/69<br>A61K39/12<br>A61K39/00<br>A61P35/00 |
| Y | SEONG SOO A. AN ET AL: "Analysis of SiO2 nanoparticles binding proteins in rat blood and brain homogenate",<br>INTERNATIONAL JOURNAL OF NANOMEDICINE,<br>vol. 9, no. Suppl.2,<br>15 December 2014 (2014-12-15), pages 207-215, XP055749347,<br>ISSN: 1176-9114, DOI: 10.2147/IJN.S58203<br>* abstract *<br>* page 208, right-hand column *<br>* tables 1-3 *<br>* figures 1-2 *<br>* page 214, left-hand column, paragraph Conclusion *<br>----- | 1-15 | |
| Y | DE 10 2011 018499 A1 (EMC MICROCOLLECTIONS GMBH [DE]) 25 October 2012 (2012-10-25)<br>* examples *<br>* figures *<br>----- | 1-15 | TECHNICAL FIELDS SEARCHED (IPC)<br>A61K<br>A61P |
| Y | WO 2019/097226 A1 (N4 PHARMA UK LTD [GB]) 23 May 2019 (2019-05-23)<br>* examples 2,3 *<br>----- | 1-15 | |
| Y | ES 2 366 841 A1 (CONSEJO SUPERIOR INVESTIGACION [ES]; UNIV VALENCIA POLITECNICA ET AL.)<br>26 October 2011 (2011-10-26)<br>* figure 1; examples *<br>-----<br>-/-- | 1-15 | |

The present search report has been drawn up for all claims

| Place of search | Date of completion of the search | Examiner |
|---|---|---|
| The Hague | 29 April 2021 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 6

Europäisches Patentamt
European Patent Office
Office européen des brevets

# EUROPEAN SEARCH REPORT

Application Number
EP 20 19 6027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2006/017476 A2 (UNIV NEW YORK STATE RES FOUND [US]; PRASAD PARAS N [US] ET AL.) 16 February 2006 (2006-02-16) * examples * * figures * | 1-15 | |
| Y | SHAKIBA SHAHABI ET AL: "Utilizing the protein corona around silica nanoparticles for dual drug loading and release", NANOSCALE, vol. 7, no. 39, 2015, pages 16251-16265, XP055749759, ISSN: 2040-3364, DOI: 10.1039/C5NR04726A * abstract * * figures; table 1 * * page 16264, left-hand column, paragraph Conclusions * | 1-15 | |
| Y | WO 00/56288 A1 (INST NEUE MAT GEMEIN GMBH [DE] ET AL.) 28 September 2000 (2000-09-28) * examples * * claims * | 1-15 | TECHNICAL FIELDS SEARCHED (IPC) |
| Y | CHRISTINA GRAF ET AL: "Surface Functionalization of Silica Nanoparticles Supports Colloidal Stability in Physiological Media and Facilitates Internalization in Cells", LANGMUIR, vol. 28, no. 20, 22 May 2012 (2012-05-22), pages 7598-7613, XP055749340, ISSN: 0743-7463, DOI: 10.1021/la204913t * abstract * * figures; tables * | 1-15 | |

-/--

The present search report has been drawn up for all claims

| Place of search | Date of completion of the search | Examiner |
|---|---|---|
| The Hague | 29 April 2021 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 6

Europäisches Patentamt
European Patent Office
Office européen des brevets

# EUROPEAN SEARCH REPORT

Application Number
EP 20 19 6027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WANG HANQI ET AL: "Amino acid-based anti-fouling functionalization of silica nanoparticles using divinyl sulfone", ACTA BIOMATERIALIA, vol. 40, 23 March 2016 (2016-03-23), pages 273-281, XP029652904, ISSN: 1742-7061, DOI: 10.1016/J.ACTBIO.2016.03.035 * abstract * * page 276 * * figures; tables * ----- | 1-15 | |
| Y | AMARIA ET AL: "Coating of L-Arginine Modified Silica on Magnetite through Two Different Sol-Gel Routes", INDONESIAN JOURNAL OF CHEMISTRY, vol. 17, no. 2, April 2017 (2017-04), pages 256-263, XP055749820, ISSN: 1411-9420, DOI: 10.22146/ijc.22521 * abstract * * figures; tables * * page 261, right-hand column * ----- | 1-15 | TECHNICAL FIELDS SEARCHED (IPC) |
| Y | SEONG SOO A. AN ET AL: "Surface treatment of silica nanoparticles for stable and charge-controlled colloidal silica", INTERNATIONAL JOURNAL OF NANOMEDICINE, December 2014 (2014-12), page 29, XP055261799, DOI: 10.2147/IJN.S57922 * abstract * * figure 6 * ----- | 1-15 | |
| Y | WO 2017/223085 A2 (UNIV MICHIGAN REGENTS [US]) 28 December 2017 (2017-12-28) * examples * ----- | 1-15 | |

-/--

The present search report has been drawn up for all claims

| Place of search | Date of completion of the search | Examiner |
|---|---|---|
| The Hague | 29 April 2021 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 6

Europäisches Patentamt
European Patent Office
Office européen des brevets

# EUROPEAN SEARCH REPORT

Application Number
EP 20 19 6027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DAFTARIAN PIROUZ M ET AL: "Rejection of large HPV-16 expressing tumors in aged mice by a single immunization of VacciMax encapsulated CTL/T helper peptides", JOURNAL OF TRANSLATIONAL MEDICINE, vol. 5, no. 1, 7 June 2007 (2007-06-07), pages 26-1-26-9, XP021030172, ISSN: 1479-5876, DOI: 10.1186/1479-5876-5-26<br>* abstract *<br>* page 2 *<br>* page 6, paragraph Discussion - page 7 *<br>* figures *<br>----- | 1-15 | |
| Y | JUNKO MATSUZAKI ET AL: "Recognition of naturally processed and ovarian cancer reactive CD8+ T cell epitopes within a promiscuous HLA class II T-helper region of NY-ESO-1", CANCER IMMUNOLOGY, IMMUNOTHERAPY, vol. 57, no. 8, 6 February 2008 (2008-02-06), pages 1185-1195, XP019624374, ISSN: 1432-0851<br>* abstract *<br>* figures *<br>* page 1192, right-hand column - page 1194 *<br>-----<br>-/-- | 1-15 | TECHNICAL FIELDS SEARCHED (IPC) |

The present search report has been drawn up for all claims

| Place of search | Date of completion of the search | Examiner |
|---|---|---|
| The Hague | 29 April 2021 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 4 of 6

Europäisches Patentamt
European Patent Office
Office européen des brevets

# EUROPEAN SEARCH REPORT

Application Number
EP 20 19 6027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ANGELIKA B. RIEMER ET AL: "A Conserved E7-derived Cytotoxic T Lymphocyte Epitope Expressed on Human Papillomavirus 16-transformed HLA-A2 + Epithelial Cancers", JOURNAL OF BIOLOGICAL CHEMISTRY, vol. 285, no. 38, 17 September 2010 (2010-09-17), pages 29608-29622, XP055207597, ISSN: 0021-9258, DOI: 10.1074/jbc.M110.126722 * abstract * * figures; tables * ----- | 1-15 | |
| Y | TSANG KWONG Y ET AL: "Identification and characterization of enhancer agonist human cytotoxic T-cell epitopes of the human papillomavirus type 16 (HPV16) E6/E7", VACCINE, vol. 35, no. 19, 4 April 2017 (2017-04-04), pages 2605-2611, XP029971906, ISSN: 0264-410X, DOI: 10.1016/J.VACCINE.2017.03.025 * abstract * * figure 1; tables * * page 2608, paragraph Discussion - page 2610 * ----- | 1-15 | TECHNICAL FIELDS SEARCHED (IPC) |
| | -/-- | | |

The present search report has been drawn up for all claims

| Place of search | Date of completion of the search | Examiner |
|---|---|---|
| The Hague | 29 April 2021 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 5 of 6

Europäisches Patentamt
European Patent Office
Office européen des brevets

# EUROPEAN SEARCH REPORT

Application Number
EP 20 19 6027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | XU M ET AL: "Ii-Key/HPV16 E7 hybrid peptide immunotherapy for HPV16+ cancers", VACCINE, vol. 27, no. 34, 23 July 2009 (2009-07-23), pages 4641-4647, XP026266831, ISSN: 0264-410X, DOI: 10.1016/J.VACCINE.2009.05.054 [retrieved on 2009-06-09]<br>* abstract *<br>* figures; table 1 *<br>* page 4644, paragraph Discussion - page 4646 *<br>----- | 1-15 | |

TECHNICAL FIELDS SEARCHED (IPC)

The present search report has been drawn up for all claims

| Place of search | Date of completion of the search | Examiner |
|---|---|---|
| The Hague | 29 April 2021 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 6 of 6

# ANNEX TO THE EUROPEAN SEARCH REPORT ON EUROPEAN PATENT APPLICATION NO.

EP 20 19 6027

This annex lists the patent family members relating to the patent documents cited in the above-mentioned European search report. The members are as contained in the European Patent Office EDP file on
The European Patent Office is in no way liable for these particulars which are merely given for the purpose of information.

29-04-2021

| Patent document cited in search report | Publication date | Patent family member(s) | Publication date |
|---|---|---|---|
| US 2016090603 A1 | 31-03-2016 | US 2016090603 A1 | 31-03-2016 |
| | | US 2018028686 A1 | 01-02-2018 |
| | | US 2018049984 A1 | 22-02-2018 |
| | | WO 2016054225 A1 | 07-04-2016 |
| DE 102011018499 A1 | 25-10-2012 | DE 102011018499 A1 | 25-10-2012 |
| | | EP 2701740 A1 | 05-03-2014 |
| | | WO 2012146364 A1 | 01-11-2012 |
| WO 2019097226 A1 | 23-05-2019 | AU 2018366384 A1 | 04-06-2020 |
| | | CA 3082682 A1 | 23-05-2019 |
| | | CN 111601771 A | 28-08-2020 |
| | | EP 3710404 A1 | 23-09-2020 |
| | | JP 2021509393 A | 25-03-2021 |
| | | US 2020392005 A1 | 17-12-2020 |
| | | WO 2019097226 A1 | 23-05-2019 |
| ES 2366841 A1 | 26-10-2011 | ES 2366841 A1 | 26-10-2011 |
| | | WO 2011124739 A1 | 13-10-2011 |
| WO 2006017476 A2 | 16-02-2006 | US 2006088599 A1 | 27-04-2006 |
| | | WO 2006017476 A2 | 16-02-2006 |
| WO 0056288 A1 | 28-09-2000 | AT 297716 T | 15-07-2005 |
| | | DE 19912502 A1 | 21-09-2000 |
| | | EP 1162955 A1 | 19-12-2001 |
| | | WO 0056288 A1 | 28-09-2000 |
| WO 2017223085 A2 | 28-12-2017 | CA 3028721 A1 | 28-12-2017 |
| | | EP 3471778 A2 | 24-04-2019 |
| | | US 2021106538 A1 | 15-04-2021 |
| | | WO 2017223085 A2 | 28-12-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015086354 A2
- WO 2018085751 A1
- WO 2006037979 A2
- WO 2013034741 A1
- WO 2013034726 A1
- WO 2011154711 A1
- WO 2010006753 A2
- EP 0216278 B1
- WO 2005085135 A1
- EP 0648503 A
- WO 2007031734 A1
- WO 2015162291 A1
- US 6180095 B1
- US 6214345 B1


### Non-patent literature cited in the description


- **ZUR HAUSEN H.** *Appl Pathol,* 1987, vol. 5, 19-24
- **BOSCH FX et al.** *J Clin Pathol,* vol. 55, 244-65
- **GILLISON ML et al.** *Vaccine,* 2012, vol. 30 (5), F34-541, 3
- **STANLEY MA.** *J Reprod Immunol,* 2001, vol. 52, 45-59
- **WELTERS MJ et al.** *Cancer Res,* 2003, vol. 63, 636-4
- **NAKAGAWA M et al.** *J Infect Dis,* 2000, vol. 182, 595-8
- **VAN DER BURG SH et al.** *Virus Res,* 2002, vol. 89, 275-84
- **ZUR HAUSEN H.** *J Natl Cancer Inst,* 2000, vol. 92, 690-8
- **TAN S. et al.** *Curr Cancer Drug Targets,* 2012, vol. 12, 170-84
- **ROBINSON J et al.** *Nucleic Acids Res,* 2009, vol. 37, D1013-D1017
- *Sci. Rep.,* 12 December 2017, vol. 7 (1), 17479
- *J. Colloid Interface Sci.,* 1968, vol. 26, 62
- *CHEMICAL ABSTRACTS,* 74-79-3
- *CHEMICAL ABSTRACTS,* 93642-68-3
- *CHEMICAL ABSTRACTS,* 757-44-8
- **PAJOT, A. et al.** *A mouse model of human adaptive immune functions: HLA-A2.1 -/HLA-DR1-transgenic H-2 class I-/class II-knockout mice, European Journal of Immunology,* 2004, vol. 34, 3060-3069
- **KRUSE, S.** Therapeutic vaccination against HPV-positive tumors in a MHC-humanized mouse model. Ruperto Carola University Heidelberg, 2019
- **KRUSE et al.** Therapeutic vaccination using minimal HPV16 epitopes in a novel MHC-humanized. *Oncoimmunology,* 2019, vol. 8 (1), e1524694

SEQUENCE LISTING

<110> Life Science Inkubator Betriebs GmbH & Co. KG
Deutsches Krebsforschungszentrum Stiftung des Öffentlichen Rechtes

<120> Composition of nanoparticles as carrier for HPV-derived immunogenic fragments

<130> Nanoparticles as HPV-vaccine

<150> EP20174156.8
<151> 2020-05-12

<160> 297

<170> BiSSAP 1.3.6

<210> 1
<211> 158
<212> PRT
<213> Human papillomavirus type 16

<220>
<223> E6 protein

<400> 1
```
Met His Gln Lys Arg Thr Ala Met Phe Gln Asp Pro Gln Glu Arg Pro
1               5                   10                  15
Arg Lys Leu Pro Gln Leu Cys Thr Glu Leu Gln Thr Thr Ile His Asp
            20                  25                  30
Ile Ile Leu Glu Cys Val Tyr Cys Lys Gln Gln Leu Leu Arg Arg Glu
        35                  40                  45
Val Tyr Asp Phe Ala Phe Arg Asp Leu Cys Ile Val Tyr Arg Asp Gly
    50                  55                  60
Asn Pro Tyr Ala Val Cys Asp Lys Cys Leu Lys Phe Tyr Ser Lys Ile
65                  70                  75                  80
Ser Glu Tyr Arg His Tyr Cys Tyr Ser Leu Tyr Gly Thr Thr Leu Glu
                85                  90                  95
Gln Gln Tyr Asn Lys Pro Leu Cys Asp Leu Leu Ile Arg Cys Ile Asn
            100                 105                 110
Cys Gln Lys Pro Leu Cys Pro Glu Glu Lys Gln Arg His Leu Asp Lys
        115                 120                 125
Lys Gln Arg Phe His Asn Ile Arg Gly Arg Trp Thr Gly Arg Cys Met
    130                 135                 140
Ser Cys Cys Arg Ser Ser Arg Thr Arg Arg Glu Thr Gln Leu
145                 150                 155
```

<210> 2
<211> 98
<212> PRT
<213> Human papillomavirus type 16

<220>
<223> E7 protein

<400> 2
```
Met His Gly Asp Thr Pro Thr Leu His Glu Tyr Met Leu Asp Leu Gln
1               5                   10                  15
Pro Glu Thr Thr Asp Leu Tyr Cys Tyr Glu Gln Leu Asn Asp Ser Ser
            20                  25                  30
```

```
Glu Glu Glu Asp Glu Ile Asp Gly Pro Ala Gly Gln Ala Glu Pro Asp
        35                  40                  45
Arg Ala His Tyr Asn Ile Val Thr Phe Cys Cys Lys Cys Asp Ser Thr
    50                  55                  60
Leu Arg Leu Cys Val Gln Ser Thr His Val Asp Ile Arg Thr Leu Glu
65                  70                  75                  80
Asp Leu Leu Met Gly Thr Leu Gly Ile Val Cys Pro Ile Cys Ser Gln
                85                  90                  95
Lys Pro
```

<210> 3
<211> 158
<212> PRT
<213> Human papillomavirus type 18

<220>
<223> E6 protein

<400> 3

```
Met Ala Arg Phe Glu Asp Pro Thr Arg Arg Pro Tyr Lys Leu Pro Asp
1               5                   10                  15
Leu Cys Thr Glu Leu Asn Thr Ser Leu Gln Asp Ile Glu Ile Thr Cys
            20                  25                  30
Val Tyr Cys Lys Thr Val Leu Glu Leu Thr Glu Val Phe Glu Phe Ala
        35                  40                  45
Phe Lys Asp Leu Phe Val Val Tyr Arg Asp Ser Ile Pro His Ala Ala
    50                  55                  60
Cys His Lys Cys Ile Asp Phe Tyr Ser Arg Ile Arg Glu Leu Arg His
65                  70                  75                  80
Tyr Ser Asp Ser Val Tyr Gly Asp Thr Leu Glu Lys Leu Thr Asn Thr
                85                  90                  95
Gly Leu Tyr Asn Leu Leu Ile Arg Cys Leu Arg Cys Gln Lys Pro Leu
            100                 105                 110
Asn Pro Ala Glu Lys Leu Arg His Leu Asn Glu Lys Arg Arg Phe His
        115                 120                 125
Asn Ile Ala Gly His Tyr Arg Gly Gln Cys His Ser Cys Cys Asn Arg
    130                 135                 140
Ala Arg Gln Glu Arg Leu Gln Arg Arg Arg Glu Thr Gln Val
145                 150                 155
```

<210> 4
<211> 105
<212> PRT
<213> Human papillomavirus type 18

<220>
<223> E7 protein

<400> 4

```
Met His Gly Pro Lys Ala Thr Leu Gln Asp Ile Val Leu His Leu Glu
1               5                   10                  15
Pro Gln Asn Glu Ile Pro Val Asp Leu Leu Cys His Glu Gln Leu Ser
            20                  25                  30
Asp Ser Glu Glu Glu Asn Asp Glu Ile Asp Gly Val Asn His Gln His
        35                  40                  45
Leu Pro Ala Arg Arg Ala Glu Pro Gln Arg His Thr Met Leu Cys Met
    50                  55                  60
Cys Cys Lys Cys Glu Ala Arg Ile Lys Leu Val Val Glu Ser Ser Ala
65                  70                  75                  80
Asp Asp Leu Arg Ala Phe Gln Gln Leu Phe Leu Asn Thr Leu Ser Phe
```

85 90 95
Val Cys Pro Trp Cys Ala Ser Gln Gln
100 105

<210> 5
<211> 30
<212> PRT
<213> Human papillomavirus type 16

<220>
<223> E6 protein aa 1-30

<400> 5
Met His Gln Lys Arg Thr Ala Met Phe Gln Asp Pro Gln Glu Arg Pro
1 5 10 15
Arg Lys Leu Pro Gln Leu Cys Thr Glu Leu Gln Thr Thr Ile
20 25 30

<210> 6
<211> 9
<212> PRT
<213> Human papillomavirus type 16

<220>
<223> E6 protein aa 7-15

<400> 6
Ala Met Phe Gln Asp Pro Gln Glu Arg
1 5

<210> 7
<211> 11
<212> PRT
<213> Human papillomavirus type 16

<220>
<223> E6 protein aa 8-18

<400> 7
Met Phe Gln Asp Pro Gln Glu Arg Pro Arg Lys
1 5 10

<210> 8
<211> 10
<212> PRT
<213> Human papillomavirus type 16

<220>
<223> E6 protein aa 9-18

<400> 8
Phe Gln Asp Pro Gln Glu Arg Pro Arg Lys
1 5 10

<210> 9
<211> 9
<212> PRT
<213> Human papillomavirus type 16

<220>
<223> E6 protein aa 11-19

<400> 9
Asp Pro Gln Glu Arg Pro Arg Lys Leu
1               5

<210> 10
<211> 22
<212> PRT
<213> Human papillomavirus type 16

<220>
<223> E6 protein aa 11-32

<400> 10
Asp Pro Gln Glu Arg Pro Arg Lys Leu Pro Gln Leu Cys Thr Glu Leu
1               5                   10                  15
Gln Thr Thr Ile His Asp
            20

<210> 11
<211> 10
<212> PRT
<213> Human papillomavirus type 16

<220>
<223> E6 protein aa 13-22

<400> 11
Gln Glu Arg Pro Arg Lys Leu Pro Gln Leu
1               5                   10

<210> 12
<211> 8
<212> PRT
<213> Human papillomavirus type 16

<220>
<223> E6 protein aa 15-22

<400> 12
Arg Pro Arg Lys Leu Pro Gln Leu
1               5

<210> 13
<211> 9
<212> PRT
<213> Human papillomavirus type 16

<220>
<223> E6 protein aa 15-23

<400> 13
Arg Pro Arg Lys Leu Pro Gln Leu Cys
1               5

<210> 14

<211> 10
<212> PRT
<213> Human papillomavirus type 16

<220>
<223> E6 protein aa 15-24

<400> 14
Arg Pro Arg Lys Leu Pro Gln Leu Cys Thr
1 5 10

<210> 15
<211> 11
<212> PRT
<213> Human papillomavirus type 16

<220>
<223> E6 protein aa 15-25

<400> 15
Arg Pro Arg Lys Leu Pro Gln Leu Cys Thr Glu
1 5 10

<210> 16
<211> 9
<212> PRT
<213> Human papillomavirus type 16

<220>
<223> E6 protein aa 18-26

<400> 16
Lys Leu Pro Gln Leu Cys Thr Glu Leu
1 5

<210> 17
<211> 11
<212> PRT
<213> Human papillomavirus type 16

<220>
<223> E6 protein aa 18-28

<400> 17
Lys Leu Pro Gln Leu Cys Thr Glu Leu Gln Thr
1 5 10

<210> 18
<211> 8
<212> PRT
<213> Human papillomavirus type 16

<220>
<223> E6 protein aa 19-26

<400> 18
Leu Pro Gln Leu Cys Thr Glu Leu
1 5

<210> 19
<211> 10
<212> PRT
<213> Human papillomavirus type 16

<220>
<223> E6 protein aa 19-28

<400> 19
Leu Pro Gln Leu Cys Thr Glu Leu Gln Thr
1 5 10

<210> 20
<211> 9
<212> PRT
<213> Human papillomavirus type 16

<220>
<223> E6 protein aa 23-31

<400> 20
Cys Thr Glu Leu Gln Thr Thr Ile His
1 5

<210> 21
<211> 9
<212> PRT
<213> Human papillomavirus type 16

<220>
<223> E6 protein aa 25-33

<400> 21
Glu Leu Gln Thr Thr Ile His Asp Ile
1 5

<210> 22
<211> 9
<212> PRT
<213> Human papillomavirus type 16

<220>
<223> E6 protein aa 26-34

<400> 22
Leu Gln Thr Thr Ile His Asp Ile Ile
1 5

<210> 23
<211> 11
<212> PRT
<213> Human papillomavirus type 16

<220>
<223> E6 protein aa 28-38

<400> 23

Thr Thr Ile His Asp Ile Ile Leu Glu Cys Val
1 5 10

<210> 24
<211> 9
<212> PRT
<213> Human papillomavirus type 16

<220>
<223> E6 protein aa 29-37

<400> 24
Thr Ile His Asp Ile Ile Leu Glu Cys
1 5

<210> 25
<211> 10
<212> PRT
<213> Human papillomavirus type 16

<220>
<223> E6 protein aa 29-38

<400> 25
Thr Ile His Asp Ile Ile Leu Glu Cys Val
1 5 10

<210> 26
<211> 11
<212> PRT
<213> Human papillomavirus type 16

<220>
<223> E6 protein aa 29-39

<400> 26
Thr Ile His Asp Ile Ile Leu Glu Cys Val Tyr
1 5 10

<210> 27
<211> 10
<212> PRT
<213> Human papillomavirus type 16

<220>
<223> E6 protein aa 30-39

<400> 27
Ile His Asp Ile Ile Leu Glu Cys Val Tyr
1 5 10

<210> 28
<211> 8
<212> PRT
<213> Human papillomavirus type 16

<220>
<223> E6 protein aa 31-38

<400> 28
His Asp Ile Ile Leu Glu Cys Val
1 5

<210> 29
<211> 9
<212> PRT
<213> Human papillomavirus type 16

<220>
<223> E6 protein aa 31-39

<400> 29
His Asp Ile Ile Leu Glu Cys Val Tyr
1 5

<210> 30
<211> 11
<212> PRT
<213> Human papillomavirus type 16

<220>
<223> E6 protein aa 31-41

<400> 30
His Asp Ile Ile Leu Glu Cys Val Tyr Cys Lys
1 5 10

<210> 31
<211> 10
<212> PRT
<213> Human papillomavirus type 16

<220>
<223> E6 protein aa 32-41

<400> 31
Asp Ile Ile Leu Glu Cys Val Tyr Cys Lys
1 5 10

<210> 32
<211> 9
<212> PRT
<213> Human papillomavirus type 16

<220>
<223> E6 protein aa 33-41

<400> 32
Ile Ile Leu Glu Cys Val Tyr Cys Lys
1 5

<210> 33
<211> 8
<212> PRT
<213> Human papillomavirus type 16

<220>
<223> E6 protein aa 34-41

<400> 33
Ile Leu Glu Cys Val Tyr Cys Lys
1 5

<210> 34
<211> 11
<212> PRT
<213> Human papillomavirus type 16

<220>
<223> E6 protein aa 34-44

<400> 34
Ile Leu Glu Cys Val Tyr Cys Lys Gln Gln Leu
1 5 10

<210> 35
<211> 10
<212> PRT
<213> Human papillomavirus type 16

<220>
<223> E6 protein aa 35-44

<400> 35
Leu Glu Cys Val Tyr Cys Lys Gln Gln Leu
1 5 10

<210> 36
<211> 10
<212> PRT
<213> Human papillomavirus type 16

<220>
<223> E6 protein aa 37-46

<400> 36
Cys Val Tyr Cys Lys Gln Gln Leu Leu Arg
1 5 10

<210> 37
<211> 18
<212> PRT
<213> Human papillomavirus type 16

<220>
<223> E6 protein aa 37-54

<400> 37
Cys Val Tyr Cys Lys Gln Gln Leu Leu Arg Arg Glu Val Tyr Asp Phe
1 5 10 15
Ala Phe

<210> 38
<211> 32

<212> PRT
<213> Human papillomavirus type 16

<220>
<223> E6 protein aa 37-68

<400> 38
Cys Val Tyr Cys Lys Gln Gln Leu Leu Arg Arg Glu Val Tyr Asp Phe
1 5 10 15
Ala Phe Arg Asp Leu Cys Ile Val Tyr Arg Asp Gly Asn Pro Tyr Ala
20 25 30

<210> 39
<211> 8
<212> PRT
<213> Human papillomavirus type 16

<220>
<223> E6 protein aa 38-45

<400> 39
Val Tyr Cys Lys Gln Gln Leu Leu
1 5

<210> 40
<211> 9
<212> PRT
<213> Human papillomavirus type 16

<220>
<223> E6 protein aa 38-46

<400> 40
Val Tyr Cys Lys Gln Gln Leu Leu Arg
1 5

<210> 41
<211> 10
<212> PRT
<213> Human papillomavirus type 16

<220>
<223> E6 protein aa 38-47

<400> 41
Val Tyr Cys Lys Gln Gln Leu Leu Arg Arg
1 5 10

<210> 42
<211> 11
<212> PRT
<213> Human papillomavirus type 16

<220>
<223> E6 protein aa 40-50

<400> 42
Cys Lys Gln Gln Leu Leu Arg Arg Glu Val Tyr
1 5 10

<210> 43
<211> 10
<212> PRT
<213> Human papillomavirus type 16

<220>
<223> E6 protein aa 41-50

<400> 43
Lys Gln Gln Leu Leu Arg Arg Glu Val Tyr
1 5 10

<210> 44
<211> 9
<212> PRT
<213> Human papillomavirus type 16

<220>
<223> E6 protein aa 42-50

<400> 44
Gln Gln Leu Leu Arg Arg Glu Val Tyr
1 5

<210> 45
<211> 11
<212> PRT
<213> Human papillomavirus type 16

<220>
<223> E6 protein aa 42-52

<400> 45
Gln Gln Leu Leu Arg Arg Glu Val Tyr Asp Phe
1 5 10

<210> 46
<211> 10
<212> PRT
<213> Human papillomavirus type 16

<220>
<223> E6 protein aa 43-52

<400> 46
Gln Leu Leu Arg Arg Glu Val Tyr Asp Phe
1 5 10

<210> 47
<211> 15
<212> PRT
<213> Human papillomavirus type 16

<220>

<223> E6 protein aa 43-57

<400> 47
Gln Leu Leu Arg Arg Glu Val Tyr Asp Phe Ala Phe Arg Asp Leu
1 5 10 15

<210> 48
<211> 9
<212> PRT
<213> Human papillomavirus type 16

<220>
<223> E6 protein aa 44-52

<400> 48
Leu Leu Arg Arg Glu Val Tyr Asp Phe
1 5

<210> 49
<211> 11
<212> PRT
<213> Human papillomavirus type 16

<220>
<223> E6 protein aa 44-54

<400> 49
Leu Leu Arg Arg Glu Val Tyr Asp Phe Ala Phe
1 5 10

<210> 50
<211> 24
<212> PRT
<213> Human papillomavirus type 16

<220>
<223> E6 protein aa 45-68

<400> 50
Leu Arg Arg Glu Val Tyr Asp Phe Ala Phe Arg Asp Leu Cys Ile Val
1 5 10 15
Tyr Arg Asp Gly Asn Pro Tyr Ala
20

<210> 51
<211> 8
<212> PRT
<213> Human papillomavirus type 16

<220>
<223> E6 protein aa 47-54

<400> 51
Arg Glu Val Tyr Asp Phe Ala Phe
1 5

<210> 52
<211> 8
<212> PRT

<213> Human papillomavirus type 16

<220>
<223> E6 protein aa 48-55

<400> 52
Glu Val Tyr Asp Phe Ala Phe Arg
1               5

<210> 53
<211> 10
<212> PRT
<213> Human papillomavirus type 16

<220>
<223> E6 protein aa 48-57

<400> 53
Glu Val Tyr Asp Phe Ala Phe Arg Asp Leu
1               5                   10

<210> 54
<211> 9
<212> PRT
<213> Human papillomavirus type 16

<220>
<223> E6 protein aa 49-57

<400> 54
Val Tyr Asp Phe Ala Phe Arg Asp Leu
1               5

<210> 55
<211> 10
<212> PRT
<213> Human papillomavirus type 16

<220>
<223> E6 protein aa 49-58

<400> 55
Val Tyr Asp Phe Ala Phe Arg Asp Leu Cys
1               5                   10

<210> 56
<211> 11
<212> PRT
<213> Human papillomavirus type 16

<220>
<223> E6 protein aa 49-59

<400> 56
Val Tyr Asp Phe Ala Phe Arg Asp Leu Cys Ile
1               5                   10

<210> 57

<211> 10
<212> PRT
<213> Human papillomavirus type 16

<220>
<223> E6 protein aa 50-59

<400> 57
Tyr Asp Phe Ala Phe Arg Asp Leu Cys Ile
1 5 10

<210> 58
<211> 9
<212> PRT
<213> Human papillomavirus type 16

<220>
<223> E6 protein aa 51-59

<400> 58
Asp Phe Ala Phe Arg Asp Leu Cys Ile
1 5

<210> 59
<211> 9
<212> PRT
<213> Human papillomavirus type 16

<220>
<223> E6 protein aa 52-60

<400> 59
Phe Ala Phe Arg Asp Leu Cys Ile Val
1 5

<210> 60
<211> 10
<212> PRT
<213> Human papillomavirus type 16

<220>
<223> E6 protein aa 52-61

<400> 60
Phe Ala Phe Arg Asp Leu Cys Ile Val Tyr
1 5 10

<210> 61
<211> 11
<212> PRT
<213> Human papillomavirus type 16

<220>
<223> E6 protein aa 52-62

<400> 61
Phe Ala Phe Arg Asp Leu Cys Ile Val Tyr Arg
1 5 10

<210> 62
<211> 9
<212> PRT
<213> Human papillomavirus type 16

<220>
<223> E6 protein aa 53-61

<400> 62
Ala Phe Arg Asp Leu Cys Ile Val Tyr
1               5

<210> 63
<211> 10
<212> PRT
<213> Human papillomavirus type 16

<220>
<223> E6 protein aa 53-62

<400> 63
Ala Phe Arg Asp Leu Cys Ile Val Tyr Arg
1               5                   10

<210> 64
<211> 15
<212> PRT
<213> Human papillomavirus type 16

<220>
<223> E6 protein aa 54-68

<400> 64
Phe Arg Asp Leu Cys Ile Val Tyr Arg Asp Gly Asn Pro Tyr Ala
1               5                   10                  15

<210> 65
<211> 32
<212> PRT
<213> Human papillomavirus type 16

<220>
<223> E6 protein aa 55-86

<400> 65
Arg Asp Leu Cys Ile Val Tyr Arg Asp Gly Asn Pro Tyr Ala Val Cys
1               5                   10                  15
Asp Lys Cys Leu Lys Phe Tyr Ser Lys Ile Ser Glu Tyr Arg His Tyr
            20                  25                  30

<210> 66
<211> 11
<212> PRT
<213> Human papillomavirus type 16

<220>
<223> E6 protein aa 57-67

<400> 66
Leu Cys Ile Val Tyr Arg Asp Gly Asn Pro Tyr
1 5 10

<210> 67
<211> 10
<212> PRT
<213> Human papillomavirus type 16

<220>
<223> E6 protein aa 58-67

<400> 67
Cys Ile Val Tyr Arg Asp Gly Asn Pro Tyr
1 5 10

<210> 68
<211> 9
<212> PRT
<213> Human papillomavirus type 16

<220>
<223> E6 protein aa 59-67

<400> 68
Ile Val Tyr Arg Asp Gly Asn Pro Tyr
1 5

<210> 69
<211> 10
<212> PRT
<213> Human papillomavirus type 16

<220>
<223> E6 protein aa 59-68

<400> 69
Ile Val Tyr Arg Asp Gly Asn Pro Tyr Ala
1 5 10

<210> 70
<211> 11
<212> PRT
<213> Human papillomavirus type 16

<220>
<223> E6 protein aa 59-69

<400> 70
Ile Val Tyr Arg Asp Gly Asn Pro Tyr Ala Val
1 5 10

<210> 71
<211> 8
<212> PRT
<213> Human papillomavirus type 16

<220>
<223> E6 protein aa 60-67

<400> 71
Val Tyr Arg Asp Gly Asn Pro Tyr
1               5

<210> 72
<211> 10
<212> PRT
<213> Human papillomavirus type 16

<220>
<223> E6 protein aa 60-69

<400> 72
Val Tyr Arg Asp Gly Asn Pro Tyr Ala Val
1               5                   10

<210> 73
<211> 22
<212> PRT
<213> Human papillomavirus type 16

<220>
<223> E6 protein aa 61-82

<400> 73
Tyr Arg Asp Gly Asn Pro Tyr Ala Val Cys Asp Lys Cys Leu Lys Phe
1               5                   10                  15
Tyr Ser Lys Ile Ser Glu
            20

<210> 74
<211> 11
<212> PRT
<213> Human papillomavirus type 16

<220>
<223> E6 protein aa 65-75

<400> 74
Asn Pro Tyr Ala Val Cys Asp Lys Cys Leu Lys
1               5                   10

<210> 75
<211> 9
<212> PRT
<213> Human papillomavirus type 16

<220>
<223> E6 protein aa 66-74

<400> 75
Pro Tyr Ala Val Cys Asp Lys Cys Leu
1               5

<210> 76
<211> 10
<212> PRT
<213> Human papillomavirus type 16

<220>
<223> E6 protein aa 66-75

<400> 76
Pro Tyr Ala Val Cys Asp Lys Cys Leu Lys
1               5                   10

<210> 77
<211> 11
<212> PRT
<213> Human papillomavirus type 16

<220>
<223> E6 protein aa 66-76

<400> 77
Pro Tyr Ala Val Cys Asp Lys Cys Leu Lys Phe
1               5                   10

<210> 78
<211> 9
<212> PRT
<213> Human papillomavirus type 16

<220>
<223> E6 protein aa 67-75

<400> 78
Tyr Ala Val Cys Asp Lys Cys Leu Lys
1               5

<210> 79
<211> 10
<212> PRT
<213> Human papillomavirus type 16

<220>
<223> E6 protein aa 67-76

<400> 79
Tyr Ala Val Cys Asp Lys Cys Leu Lys Phe
1               5                   10

<210> 80
<211> 11
<212> PRT
<213> Human papillomavirus type 16

<220>
<223> E6 protein aa 67-77

<400> 80
Tyr Ala Val Cys Asp Lys Cys Leu Lys Phe Tyr

1 5 10

<210> 81
<211> 8
<212> PRT
<213> Human papillomavirus type 16

<220>
<223> E6 protein aa 68-75

<400> 81
Ala Val Cys Asp Lys Cys Leu Lys
1 5

<210> 82
<211> 9
<212> PRT
<213> Human papillomavirus type 16

<220>
<223> E6 protein aa 68-76

<400> 82
Ala Val Cys Asp Lys Cys Leu Lys Phe
1 5

<210> 83
<211> 10
<212> PRT
<213> Human papillomavirus type 16

<220>
<223> E6 protein aa 68-77

<400> 83
Ala Val Cys Asp Lys Cys Leu Lys Phe Tyr
1 5 10

<210> 84
<211> 11
<212> PRT
<213> Human papillomavirus type 16

<220>
<223> E6 protein aa 68-78

<400> 84
Ala Val Cys Asp Lys Cys Leu Lys Phe Tyr Ser
1 5 10

<210> 85
<211> 11
<212> PRT
<213> Human papillomavirus type 16

<220>
<223> E6 protein aa 69-79

<400> 85
Val Cys Asp Lys Cys Leu Lys Phe Tyr Ser Lys
1 5 10

<210> 86
<211> 8
<212> PRT
<213> Human papillomavirus type 16

<220>
<223> E6 protein aa 71-78

<400> 86
Asp Lys Cys Leu Lys Phe Tyr Ser
1 5

<210> 87
<211> 9
<212> PRT
<213> Human papillomavirus type 16

<220>
<223> E6 protein aa 72-80

<400> 87
Lys Cys Leu Lys Phe Tyr Ser Lys Ile
1 5

<210> 88
<211> 11
<212> PRT
<213> Human papillomavirus type 16

<220>
<223> E6 protein aa 73-83

<400> 88
Cys Leu Lys Phe Tyr Ser Lys Ile Ser Glu Tyr
1 5 10

<210> 89
<211> 33
<212> PRT
<213> Human papillomavirus type 16

<220>
<223> E6 protein aa 73-105

<400> 89
Cys Leu Lys Phe Tyr Ser Lys Ile Ser Glu Tyr Arg His Tyr Cys Tyr
1 5 10 15
Ser Leu Tyr Gly Thr Thr Leu Glu Gln Gln Tyr Asn Lys Pro Leu Cys
20 25 30
Asp

<210> 90
<211> 10
<212> PRT

<213> Human papillomavirus type 16

<220>
<223> E6 protein aa 74-83

<400> 90
Leu Lys Phe Tyr Ser Lys Ile Ser Glu Tyr
1               5                   10

<210> 91
<211> 15
<212> PRT
<213> Human papillomavirus type 16

<220>
<223> E6 protein aa 74-88

<400> 91
Leu Lys Phe Tyr Ser Lys Ile Ser Glu Tyr Arg His Tyr Cys Tyr
1               5                   10                  15

<210> 92
<211> 9
<212> PRT
<213> Human papillomavirus type 16

<220>
<223> E6 protein aa 75-83

<400> 92
Lys Phe Tyr Ser Lys Ile Ser Glu Tyr
1               5

<210> 93
<211> 10
<212> PRT
<213> Human papillomavirus type 16

<220>
<223> E6 protein aa 75-84

<400> 93
Lys Phe Tyr Ser Lys Ile Ser Glu Tyr Arg
1               5                   10

<210> 94
<211> 11
<212> PRT
<213> Human papillomavirus type 16

<220>
<223> E6 protein aa 75-85

<400> 94
Lys Phe Tyr Ser Lys Ile Ser Glu Tyr Arg His
1               5                   10

<210> 95

<211> 8
<212> PRT
<213> Human papillomavirus type 16

<220>
<223> E6 protein aa 76-83

<400> 95
Phe Tyr Ser Lys Ile Ser Glu Tyr
1               5

<210> 96
<211> 10
<212> PRT
<213> Human papillomavirus type 16

<220>
<223> E6 protein aa 76-85

<400> 96
Phe Tyr Ser Lys Ile Ser Glu Tyr Arg His
1               5                   10

<210> 97
<211> 11
<212> PRT
<213> Human papillomavirus type 16

<220>
<223> E6 protein aa 76-86

<400> 97
Phe Tyr Ser Lys Ile Ser Glu Tyr Arg His Tyr
1               5                   10

<210> 98
<211> 10
<212> PRT
<213> Human papillomavirus type 16

<220>
<223> E6 protein aa 77-86

<400> 98
Tyr Ser Lys Ile Ser Glu Tyr Arg His Tyr
1               5                   10

<210> 99
<211> 9
<212> PRT
<213> Human papillomavirus type 16

<220>
<223> E6 protein aa 78-86

<400> 99
Ser Lys Ile Ser Glu Tyr Arg His Tyr
1               5

<210> 100
<211> 11
<212> PRT
<213> Human papillomavirus type 16

<220>
<223> E6 protein aa 78-88

<400> 100
Ser Lys Ile Ser Glu Tyr Arg His Tyr Cys Tyr
1               5                   10

<210> 101
<211> 8
<212> PRT
<213> Human papillomavirus type 16

<220>
<223> E6 protein aa 79-86

<400> 101
Lys Ile Ser Glu Tyr Arg His Tyr
1               5

<210> 102
<211> 9
<212> PRT
<213> Human papillomavirus type 16

<220>
<223> E6 protein aa 79-87

<400> 102
Lys Ile Ser Glu Tyr Arg His Tyr Cys
1               5

<210> 103
<211> 10
<212> PRT
<213> Human papillomavirus type 16

<220>
<223> E6 protein aa 79-88

<400> 103
Lys Ile Ser Glu Tyr Arg His Tyr Cys Tyr
1               5                   10

<210> 104
<211> 9
<212> PRT
<213> Human papillomavirus type 16

<220>
<223> E6 protein aa 80-88

<400> 104

Ile Ser Glu Tyr Arg His Tyr Cys Tyr
1               5

<210> 105
<211> 8
<212> PRT
<213> Human papillomavirus type 16

<220>
<223> E6 protein aa 81-88

<400> 105
Ser Glu Tyr Arg His Tyr Cys Tyr
1               5

<210> 106
<211> 10
<212> PRT
<213> Human papillomavirus type 16

<220>
<223> E6 protein aa 81-90

<400> 106
Ser Glu Tyr Arg His Tyr Cys Tyr Ser Leu
1               5                   10

<210> 107
<211> 11
<212> PRT
<213> Human papillomavirus type 16

<220>
<223> E6 protein aa 81-91

<400> 107
Ser Glu Tyr Arg His Tyr Cys Tyr Ser Leu Tyr
1               5                   10

<210> 108
<211> 9
<212> PRT
<213> Human papillomavirus type 16

<220>
<223> E6 protein aa 82-90

<400> 108
Glu Tyr Arg His Tyr Cys Tyr Ser Leu
1               5

<210> 109
<211> 10
<212> PRT
<213> Human papillomavirus type 16

<220>
<223> E6 protein aa 82-91

<400> 109
Glu Tyr Arg His Tyr Cys Tyr Ser Leu Tyr
1 5 10

<210> 110
<211> 8
<212> PRT
<213> Human papillomavirus type 16

<220>
<223> E6 protein aa 83-90

<400> 110
Tyr Arg His Tyr Cys Tyr Ser Leu
1 5

<210> 111
<211> 9
<212> PRT
<213> Human papillomavirus type 16

<220>
<223> E6 protein aa 83-91

<400> 111
Tyr Arg His Tyr Cys Tyr Ser Leu Tyr
1 5

<210> 112
<211> 8
<212> PRT
<213> Human papillomavirus type 16

<220>
<223> E6 protein aa 84-91

<400> 112
Arg His Tyr Cys Tyr Ser Leu Tyr
1 5

<210> 113
<211> 11
<212> PRT
<213> Human papillomavirus type 16

<220>
<223> E6 protein aa 84-94

<400> 113
Arg His Tyr Cys Tyr Ser Leu Tyr Gly Thr Thr
1 5 10

<210> 114
<211> 11
<212> PRT
<213> Human papillomavirus type 16

<220>
<223> E6 protein aa 85-95

<400> 114
His Tyr Cys Tyr Ser Leu Tyr Gly Thr Thr Leu
1 5 10

<210> 115
<211> 11
<212> PRT
<213> Human papillomavirus type 16

<220>
<223> E6 protein aa 86-96

<400> 115
Tyr Cys Tyr Ser Leu Tyr Gly Thr Thr Leu Glu
1 5 10

<210> 116
<211> 9
<212> PRT
<213> Human papillomavirus type 16

<220>
<223> E6 protein aa 87-95

<400> 116
Cys Tyr Ser Leu Tyr Gly Thr Thr Leu
1 5

<210> 117
<211> 10
<212> PRT
<213> Human papillomavirus type 16

<220>
<223> E6 protein aa 87-96

<400> 117
Cys Tyr Ser Leu Tyr Gly Thr Thr Leu Glu
1 5 10

<210> 118
<211> 8
<212> PRT
<213> Human papillomavirus type 16

<220>
<223> E6 protein aa 88-95

<400> 118
Tyr Ser Leu Tyr Gly Thr Thr Leu
1 5

<210> 119
<211> 11
<212> PRT
<213> Human papillomavirus type 16

<220>
<223> E6 protein aa 89-99

<400> 119
Ser Leu Tyr Gly Thr Thr Leu Glu Gln Gln Tyr
1 5 10

<210> 120
<211> 10
<212> PRT
<213> Human papillomavirus type 16

<220>
<223> E6 protein aa 90-99

<400> 120
Leu Tyr Gly Thr Thr Leu Glu Gln Gln Tyr
1 5 10

<210> 121
<211> 10
<212> PRT
<213> Human papillomavirus type 16

<220>
<223> E6 protein aa 91-100

<400> 121
Tyr Gly Thr Thr Leu Glu Gln Gln Tyr Asn
1 5 10

<210> 122
<211> 11
<212> PRT
<213> Human papillomavirus type 16

<220>
<223> E6 protein aa 91-101

<400> 122
Tyr Gly Thr Thr Leu Glu Gln Gln Tyr Asn Lys
1 5 10

<210> 123
<211> 22
<212> PRT
<213> Human papillomavirus type 16

<220>
<223> E6 protein aa 91-112

<400> 123
Tyr Gly Thr Thr Leu Glu Gln Gln Tyr Asn Lys Pro Leu Cys Asp Leu
1 5 10 15
Leu Ile Arg Cys Ile Asn
20

<210> 124
<211> 8
<212> PRT
<213> Human papillomavirus type 16

<220>
<223> E6 protein aa 92-99

<400> 124
Gly Thr Thr Leu Glu Gln Gln Tyr
1 5

<210> 125
<211> 10
<212> PRT
<213> Human papillomavirus type 16

<220>
<223> E6 protein aa 92-101

<400> 125
Gly Thr Thr Leu Glu Gln Gln Tyr Asn Lys
1 5 10

<210> 126
<211> 9
<212> PRT
<213> Human papillomavirus type 16

<220>
<223> E6 protein aa 93-101

<400> 126
Thr Thr Leu Glu Gln Gln Tyr Asn Lys
1 5

<210> 127
<211> 8
<212> PRT
<213> Human papillomavirus type 16

<220>
<223> E6 protein aa 94-101

<400> 127
Thr Leu Glu Gln Gln Tyr Asn Lys
1 5

<210> 128
<211> 9
<212> PRT
<213> Human papillomavirus type 16

<220>
<223> E6 protein aa 95-103

<400> 128
Leu Glu Gln Gln Tyr Asn Lys Pro Leu

1 5

<210> 129
<211> 10
<212> PRT
<213> Human papillomavirus type 16

<220>
<223> E6 protein aa 97-106

<400> 129
Gln Gln Tyr Asn Lys Pro Leu Cys Asp Leu
1 5 10

<210> 130
<211> 9
<212> PRT
<213> Human papillomavirus type 16

<220>
<223> E6 protein aa 98-106

<400> 130
Gln Tyr Asn Lys Pro Leu Cys Asp Leu
1 5

<210> 131
<211> 10
<212> PRT
<213> Human papillomavirus type 16

<220>
<223> E6 protein aa 98-107

<400> 131
Gln Tyr Asn Lys Pro Leu Cys Asp Leu Leu
1 5 10

<210> 132
<211> 11
<212> PRT
<213> Human papillomavirus type 16

<220>
<223> E6 protein aa 98-108

<400> 132
Gln Tyr Asn Lys Pro Leu Cys Asp Leu Leu Ile
1 5 10

<210> 133
<211> 8
<212> PRT
<213> Human papillomavirus type 16

<220>
<223> E6 protein aa 99-106

<400> 133
Tyr Asn Lys Pro Leu Cys Asp Leu
1 5

<210> 134
<211> 8
<212> PRT
<213> Human papillomavirus type 16

<220>
<223> E6 protein aa 101-108

<400> 134
Lys Pro Leu Cys Asp Leu Leu Ile
1 5

<210> 135
<211> 11
<212> PRT
<213> Human papillomavirus type 16

<220>
<223> E6 protein aa 101-111

<400> 135
Lys Pro Leu Cys Asp Leu Leu Ile Arg Cys Ile
1 5 10

<210> 136
<211> 22
<212> PRT
<213> Human papillomavirus type 16

<220>
<223> E6 protein aa 101-122

<400> 136
Lys Pro Leu Cys Asp Leu Leu Ile Arg Cys Ile Asn Cys Gln Lys Pro
1 5 10 15
Leu Cys Pro Glu Glu Lys
20

<210> 137
<211> 11
<212> PRT
<213> Human papillomavirus type 16

<220>
<223> E6 protein aa 105-115

<400> 137
Asp Leu Leu Ile Arg Cys Ile Asn Cys Gln Lys
1 5 10

<210> 138
<211> 10
<212> PRT
<213> Human papillomavirus type 16

<220>
<223> E6 protein aa 106-115

<400> 138
Leu Leu Ile Arg Cys Ile Asn Cys Gln Lys
1 5 10

<210> 139
<211> 9
<212> PRT
<213> Human papillomavirus type 16

<220>
<223> E6 protein aa 107-115

<400> 139
Leu Ile Arg Cys Ile Asn Cys Gln Lys
1 5

<210> 140
<211> 11
<212> PRT
<213> Human papillomavirus type 16

<220>
<223> E6 protein aa 107-117

<400> 140
Leu Ile Arg Cys Ile Asn Cys Gln Lys Pro Leu
1 5 10

<210> 141
<211> 11
<212> PRT
<213> Human papillomavirus type 16

<220>
<223> E6 protein aa 109-119

<400> 141
Arg Cys Ile Asn Cys Gln Lys Pro Leu Cys Pro
1 5 10

<210> 142
<211> 9
<212> PRT
<213> Human papillomavirus type 16

<220>
<223> E6 protein aa 113-121

<400> 142
Cys Gln Lys Pro Leu Cys Pro Glu Glu
1 5

<210> 143
<211> 9
<212> PRT

<213> Human papillomavirus type 16

<220>
<223> E6 protein aa 118-126

<400> 143
Cys Pro Glu Glu Lys Gln Arg His Leu
1               5

<210> 144
<211> 22
<212> PRT
<213> Human papillomavirus type 16

<220>
<223> E6 protein aa 121-142

<400> 144
Glu Lys Gln Arg His Leu Asp Lys Lys Gln Arg Phe His Asn Ile Arg
1               5                   10                  15
Gly Arg Trp Thr Gly Arg
            20

<210> 145
<211> 11
<212> PRT
<213> Human papillomavirus type 16

<220>
<223> E6 protein aa 122-132

<400> 145
Lys Gln Arg His Leu Asp Lys Lys Gln Arg Phe
1               5                   10

<210> 146
<211> 9
<212> PRT
<213> Human papillomavirus type 16

<220>
<223> E6 protein aa 125-133

<400> 146
His Leu Asp Lys Lys Gln Arg Phe His
1               5

<210> 147
<211> 11
<212> PRT
<213> Human papillomavirus type 16

<220>
<223> E6 protein aa 125-135

<400> 147
His Leu Asp Lys Lys Gln Arg Phe His Asn Ile
1               5                   10

<210> 148
<211> 8
<212> PRT
<213> Human papillomavirus type 16

<220>
<223> E6 protein aa 127-134

<400> 148
Asp Lys Lys Gln Arg Phe His Asn
1               5

<210> 149
<211> 9
<212> PRT
<213> Human papillomavirus type 16

<220>
<223> E6 protein aa 127-135

<400> 149
Asp Lys Lys Gln Arg Phe His Asn Ile
1               5

<210> 150
<211> 15
<212> PRT
<213> Human papillomavirus type 16

<220>
<223> E6 protein aa 127-141

<400> 150
Asp Lys Lys Gln Arg Phe His Asn Ile Arg Gly Arg Trp Thr Gly
1               5                   10                  15

<210> 151
<211> 8
<212> PRT
<213> Human papillomavirus type 16

<220>
<223> E6 protein aa 128-135

<400> 151
Lys Lys Gln Arg Phe His Asn Ile
1               5

<210> 152
<211> 9
<212> PRT
<213> Human papillomavirus type 16

<220>
<223> E6 protein aa 128-136

<400> 152

Lys Lys Gln Arg Phe His Asn Ile Arg
1               5

<210> 153
<211> 10
<212> PRT
<213> Human papillomavirus type 16

<220>
<223> E6 protein aa 129-138

<400> 153
Lys Gln Arg Phe His Asn Ile Arg Gly Arg
1               5                   10

<210> 154
<211> 11
<212> PRT
<213> Human papillomavirus type 16

<220>
<223> E6 protein aa 129-139

<400> 154
Lys Gln Arg Phe His Asn Ile Arg Gly Arg Trp
1               5                   10

<210> 155
<211> 9
<212> PRT
<213> Human papillomavirus type 16

<220>
<223> E6 protein aa 131-139

<400> 155
Arg Phe His Asn Ile Arg Gly Arg Trp
1               5

<210> 156
<211> 11
<212> PRT
<213> Human papillomavirus type 16

<220>
<223> E6 protein aa 134-144

<400> 156
Asn Ile Arg Gly Arg Trp Thr Gly Arg Cys Met
1               5                   10

<210> 157
<211> 9
<212> PRT
<213> Human papillomavirus type 16

<220>
<223> E6 protein aa 136-144

<400> 157
Arg Gly Arg Trp Thr Gly Arg Cys Met
1 5

<210> 158
<211> 10
<212> PRT
<213> Human papillomavirus type 16

<220>
<223> E6 protein aa 137-146

<400> 158
Gly Arg Trp Thr Gly Arg Cys Met Ser Cys
1 5 10

<210> 159
<211> 10
<212> PRT
<213> Human papillomavirus type 16

<220>
<223> E6 protein aa 139-148

<400> 159
Trp Thr Gly Arg Cys Met Ser Cys Cys Arg
1 5 10

<210> 160
<211> 10
<212> PRT
<213> Human papillomavirus type 16

<220>
<223> E6 protein aa 142-151

<400> 160
Arg Cys Met Ser Cys Cys Arg Ser Ser Arg
1 5 10

<210> 161
<211> 11
<212> PRT
<213> Human papillomavirus type 16

<220>
<223> E6 protein aa 148-158

<400> 161
Arg Ser Ser Arg Thr Arg Arg Glu Thr Gln Leu
1 5 10

<210> 162
<211> 10
<212> PRT
<213> Human papillomavirus type 16

<220>
<223> E6 protein aa 149-158

<400> 162
Ser Ser Arg Thr Arg Arg Glu Thr Gln Leu
1 5 10

<210> 163
<211> 8
<212> PRT
<213> Human papillomavirus type 16

<220>
<223> E6 protein aa 151-158

<400> 163
Arg Thr Arg Arg Glu Thr Gln Leu
1 5

<210> 164
<211> 12
<212> PRT
<213> Human papillomavirus type 16

<220>
<223> E7 protein aa 1-12

<400> 164
Met His Gly Asp Thr Pro Thr Leu His Glu Tyr Met
1 5 10

<210> 165
<211> 10
<212> PRT
<213> Human papillomavirus type 16

<220>
<223> E7 protein aa 2-11

<400> 165
His Gly Asp Thr Pro Thr Leu His Glu Tyr
1 5 10

<210> 166
<211> 8
<212> PRT
<213> Human papillomavirus type 16

<220>
<223> E7 protein aa 4-11

<400> 166
Asp Thr Pro Thr Leu His Glu Tyr
1 5

<210> 167
<211> 8
<212> PRT
<213> Human papillomavirus type 16

<220>
<223> E7 protein aa 5-12

<400> 167
Thr Pro Thr Leu His Glu Tyr Met
1 5

<210> 168
<211> 9
<212> PRT
<213> Human papillomavirus type 16

<220>
<223> E7 protein aa 5-13

<400> 168
Thr Pro Thr Leu His Glu Tyr Met Leu
1 5

<210> 169
<211> 14
<212> PRT
<213> Human papillomavirus type 16

<220>
<223> E7 protein aa 5-18

<400> 169
Thr Pro Thr Leu His Glu Tyr Met Leu Asp Leu Gln Pro Glu
1 5 10

<210> 170
<211> 9
<212> PRT
<213> Human papillomavirus type 16

<220>
<223> E7 protein aa 7-15

<400> 170
Thr Leu His Glu Tyr Met Leu Asp Leu
1 5

<210> 171
<211> 11
<212> PRT
<213> Human papillomavirus type 16

<220>
<223> E7 protein aa 7-17

<400> 171
Thr Leu His Glu Tyr Met Leu Asp Leu Gln Pro
1 5 10

<210> 172
<211> 21

<212> PRT
<213> Human papillomavirus type 16

<220>
<223> E7 protein aa 7-27

<400> 172
Thr Leu His Glu Tyr Met Leu Asp Leu Gln Pro Glu Thr Thr Asp Leu
1 5 10 15
Tyr Cys Tyr Glu Gln
20

<210> 173
<211> 8
<212> PRT
<213> Human papillomavirus type 16

<220>
<223> E7 protein aa 11-18

<400> 173
Tyr Met Leu Asp Leu Gln Pro Glu
1 5

<210> 174
<211> 9
<212> PRT
<213> Human papillomavirus type 16

<220>
<223> E7 protein aa 11-19

<400> 174
Tyr Met Leu Asp Leu Gln Pro Glu Thr
1 5

<210> 175
<211> 10
<212> PRT
<213> Human papillomavirus type 16

<220>
<223> E7 protein aa 11-20

<400> 175
Tyr Met Leu Asp Leu Gln Pro Glu Thr Thr
1 5 10

<210> 176
<211> 11
<212> PRT
<213> Human papillomavirus type 16

<220>
<223> E7 protein aa 11-21

<400> 176
Tyr Met Leu Asp Leu Gln Pro Glu Thr Thr Asp

1 5 10

<210> 177
<211> 16
<212> PRT
<213> Human papillomavirus type 16

<220>
<223> E7 protein aa 11-26

<400> 177
Tyr Met Leu Asp Leu Gln Pro Glu Thr Thr Asp Leu Tyr Cys Tyr Glu
1 5 10 15

<210> 178
<211> 8
<212> PRT
<213> Human papillomavirus type 16

<220>
<223> E7 protein aa 12-19

<400> 178
Met Leu Asp Leu Gln Pro Glu Thr
1 5

<210> 179
<211> 8
<212> PRT
<213> Human papillomavirus type 16

<220>
<223> E7 protein aa 12-20

<400> 179
Met Leu Asp Leu Gln Pro Glu Thr
1 5

<210> 180
<211> 15
<212> PRT
<213> Human papillomavirus type 16

<220>
<223> E7 protein aa 12-26

<400> 180
Met Leu Asp Leu Gln Pro Glu Thr Thr Asp Leu Tyr Cys Tyr Glu
1 5 10 15

<210> 181
<211> 10
<212> PRT
<213> Human papillomavirus type 16

<220>

<223> E7 protein aa 14-23

<400> 181
Asp Leu Gln Pro Glu Thr Thr Asp Leu Tyr
1 5 10

<210> 182
<211> 9
<212> PRT
<213> Human papillomavirus type 16

<220>
<223> E7 protein aa 15-23

<400> 182
Leu Gln Pro Glu Thr Thr Asp Leu Tyr
1 5

<210> 183
<211> 10
<212> PRT
<213> Human papillomavirus type 16

<220>
<223> E7 protein aa 15-24

<400> 183
Leu Gln Pro Glu Thr Thr Asp Leu Tyr Cys
1 5 10

<210> 184
<211> 11
<212> PRT
<213> Human papillomavirus type 16

<220>
<223> E7 protein aa 15-25

<400> 184
Leu Gln Pro Glu Thr Thr Asp Leu Tyr Cys Tyr
1 5 10

<210> 185
<211> 22
<212> PRT
<213> Human papillomavirus type 16

<220>
<223> E7 protein aa 17-38

<400> 185
Pro Glu Thr Thr Asp Leu Tyr Cys Tyr Glu Gln Leu Asn Asp Ser Ser
1 5 10 15
Glu Glu Glu Asp Glu Ile
20

<210> 186
<211> 8
<212> PRT

<213> Human papillomavirus type 16

<220>
<223> E7 protein aa 18-25

<400> 186
Glu Thr Thr Asp Leu Tyr Cys Tyr
1               5

<210> 187
<211> 9
<212> PRT
<213> Human papillomavirus type 16

<220>
<223> E7 protein aa 18-26

<400> 187
Glu Thr Thr Asp Leu Tyr Cys Tyr Glu
1               5

<210> 188
<211> 9
<212> PRT
<213> Human papillomavirus type 16

<220>
<223> E7 protein aa 19-27

<400> 188
Thr Thr Asp Leu Tyr Cys Tyr Glu Gln
1               5

<210> 189
<211> 11
<212> PRT
<213> Human papillomavirus type 16

<220>
<223> E7 protein aa 19-29

<400> 189
Thr Thr Asp Leu Tyr Cys Tyr Glu Gln Leu Asn
1               5                   10

<210> 190
<211> 20
<212> PRT
<213> Human papillomavirus type 16

<220>
<223> E7 protein aa 21-40

<400> 190
Asp Leu Tyr Cys Tyr Glu Gln Leu Asn Asp Ser Ser Glu Glu Glu Asp
1               5                   10                  15
Glu Ile Asp Gly
            20

<210> 191
<211> 22
<212> PRT
<213> Human papillomavirus type 16

<220>
<223> E7 protein aa 21-42

<400> 191
Asp Leu Tyr Cys Tyr Glu Gln Leu Asn Asp Ser Ser Glu Glu Glu Asp
1               5                   10                  15
Glu Ile Asp Gly Pro Ala
            20

<210> 192
<211> 16
<212> PRT
<213> Human papillomavirus type 16

<220>
<223> E7 protein aa 35-50

<400> 192
Glu Asp Glu Ile Asp Gly Pro Ala Gly Gln Ala Glu Pro Asp Arg Ala
1               5                   10                  15

<210> 193
<211> 11
<212> PRT
<213> Human papillomavirus type 16

<220>
<223> E7 protein 42-52

<400> 193
Ala Gly Gln Ala Glu Pro Asp Arg Ala His Tyr
1               5                   10

<210> 194
<211> 9
<212> PRT
<213> Human papillomavirus type 16

<220>
<223> E7 protein aa 43-51

<400> 194
Gly Gln Ala Glu Pro Asp Arg Ala His
1               5

<210> 195
<211> 10
<212> PRT
<213> Human papillomavirus type 16

<220>
<223> E7 protein aa 43-52

<400> 195
Gly Gln Ala Glu Pro Asp Arg Ala His Tyr
1 5 10

<210> 196
<211> 11
<212> PRT
<213> Human papillomavirus type 16

<220>
<223> E7 protein aa 43-53

<400> 196
Gly Gln Ala Glu Pro Asp Arg Ala His Tyr Asn
1 5 10

<210> 197
<211> 35
<212> PRT
<213> Human papillomavirus type 16

<220>
<223> E7 protein aa 43-77

<400> 197
Gly Gln Ala Glu Pro Asp Arg Ala His Tyr Asn Ile Val Thr Phe Cys
1 5 10 15
Cys Lys Cys Asp Ser Thr Leu Arg Leu Cys Val Gln Ser Thr His Val
20 25 30
Asp Ile Arg
35

<210> 198
<211> 9
<212> PRT
<213> Human papillomavirus type 16

<220>
<223> E7 protein aa 44-52

<400> 198
Gln Ala Glu Pro Asp Arg Ala His Tyr
1 5

<210> 199
<211> 19
<212> PRT
<213> Human papillomavirus type 16

<220>
<223> E7 protein aa 44-62

<400> 199
Gln Ala Glu Pro Asp Arg Ala His Tyr Asn Ile Val Thr Phe Cys Cys
1 5 10 15
Lys Cys Asp

<210> 200
<211> 10
<212> PRT
<213> Human papillomavirus type 16

<220>
<223> E7 protein aa 46-55

<400> 200
Glu Pro Asp Arg Ala His Tyr Asn Ile Val
1 5 10

<210> 201
<211> 11
<212> PRT
<213> Human papillomavirus type 16

<220>
<223> E7 protein aa 47-57

<400> 201
Pro Asp Arg Ala His Tyr Asn Ile Val Thr Phe
1 5 10

<210> 202
<211> 10
<212> PRT
<213> Human papillomavirus type 16

<220>
<223> E7 protein aa 48-57

<400> 202
Asp Arg Ala His Tyr Asn Ile Val Thr Phe
1 5 10

<210> 203
<211> 15
<212> PRT
<213> Human papillomavirus type 16

<220>
<223> E7 protein aa 48-62

<400> 203
Asp Arg Ala His Tyr Asn Ile Val Thr Phe Cys Cys Lys Cys Asp
1 5 10 15

<210> 204
<211> 9
<212> PRT
<213> Human papillomavirus type 16

<220>
<223> E7 protein aa 49-57

<400> 204
Arg Ala His Tyr Asn Ile Val Thr Phe
1 5

<210> 205
<211> 8
<212> PRT
<213> Human papillomavirus type 16

<220>
<223> E7 protein aa 50-57

<400> 205
Ala His Tyr Asn Ile Val Thr Phe
1 5

<210> 206
<211> 11
<212> PRT
<213> Human papillomavirus type 16

<220>
<223> E7 protein aa 50-60

<400> 206
Ala His Tyr Asn Ile Val Thr Phe Cys Cys Lys
1 5 10

<210> 207
<211> 13
<212> PRT
<213> Human papillomavirus type 16

<220>
<223> E7 protein aa 50-62

<400> 207
Ala His Tyr Asn Ile Val Thr Phe Cys Cys Lys Cys Asp
1 5 10

<210> 208
<211> 8
<212> PRT
<213> Human papillomavirus type 16

<220>
<223> E7 protein aa 51-58

<400> 208
His Tyr Asn Ile Val Thr Phe Cys
1 5

<210> 209
<211> 9
<212> PRT
<213> Human papillomavirus type 16

<220>

<223> E7 protein aa 51-59

<400> 209
His Tyr Asn Ile Val Thr Phe Cys Cys
1 5

<210> 210
<211> 22
<212> PRT
<213> Human papillomavirus type 16

<220>
<223> E7 protein aa 51-72

<400> 210
His Tyr Asn Ile Val Thr Phe Cys Cys Lys Cys Asp Ser Thr Leu Arg
1 5 10 15
Leu Cys Val Gln Ser Thr
20

<210> 211
<211> 9
<212> PRT
<213> Human papillomavirus type 16

<220>
<223> E7 protein aa 52-60

<400> 211
Tyr Asn Ile Val Thr Phe Cys Cys Lys
1 5

<210> 212
<211> 8
<212> PRT
<213> Human papillomavirus type 16

<220>
<223> E7 protein aa 53-60

<400> 212
Asn Ile Val Thr Phe Cys Cys Lys
1 5

<210> 213
<211> 10
<212> PRT
<213> Human papillomavirus type 16

<220>
<223> E7 protein aa 56-65

<400> 213
Thr Phe Cys Cys Lys Cys Asp Ser Thr Leu
1 5 10

<210> 214
<211> 14
<212> PRT

<213> Human papillomavirus type 16

<220>
<223> E7 protein aa 62-75

<400> 214
Asp Ser Thr Leu Arg Leu Cys Val Gln Ser Thr His Val Asp
1               5                   10

<210> 215
<211> 15
<212> PRT
<213> Human papillomavirus type 16

<220>
<223> E7 protein aa 64-78

<400> 215
Thr Leu Arg Leu Cys Val Gln Ser Thr His Val Asp Ile Arg Thr
1               5                   10                  15

<210> 216
<211> 9
<212> PRT
<213> Human papillomavirus type 16

<220>
<223> E7 protein aa 66-74

<400> 216
Arg Leu Cys Val Gln Ser Thr His Val
1               5

<210> 217
<211> 10
<212> PRT
<213> Human papillomavirus type 16

<220>
<223> E7 protein aa 67-76

<400> 217
Leu Cys Val Gln Ser Thr His Val Asp Ile
1               5                   10

<210> 218
<211> 32
<212> PRT
<213> Human papillomavirus type 16

<220>
<223> E7 protein aa 67-98

<400> 218
Leu Cys Val Gln Ser Thr His Val Asp Ile Arg Thr Leu Glu Asp Leu
1               5                   10                  15
Leu Met Gly Thr Leu Gly Ile Val Cys Pro Ile Cys Ser Gln Lys Pro
            20                  25                  30

<210> 219
<211> 8
<212> PRT
<213> Human papillomavirus type 16

<220>
<223> E7 protein aa 69-76

<400> 219
Val Gln Ser Thr His Val Asp Ile
1               5

<210> 220
<211> 18
<212> PRT
<213> Human papillomavirus type 16

<220>
<223> E7 protein aa 69-86

<400> 220
Val Gln Ser Thr His Val Asp Ile Arg Thr Leu Glu Asp Leu Leu Met
1               5                   10                  15
Gly Thr

<210> 221
<211> 15
<212> PRT
<213> Human papillomavirus type 16

<220>
<223> E7 protein aa 71-85

<400> 221
Ser Thr His Val Asp Ile Arg Thr Leu Glu Asp Leu Leu Met Gly
1               5                   10                  15

<210> 222
<211> 26
<212> PRT
<213> Human papillomavirus type 16

<220>
<223> E7 protein aa 72-97

<400> 222
Thr His Val Asp Ile Arg Thr Leu Glu Asp Leu Leu Met Gly Thr Leu
1               5                   10                  15
Gly Ile Val Cys Pro Ile Cys Ser Gln Lys
            20                  25

<210> 223
<211> 12
<212> PRT
<213> Human papillomavirus type 16

<220>
<223> E7 protein aa 73-84

<400> 223
His Val Asp Ile Arg Thr Leu Glu Asp Leu Leu Met
1               5                   10

<210> 224
<211> 11
<212> PRT
<213> Human papillomavirus type 16

<220>
<223> E7 protein aa 76-86

<400> 224
Ile Arg Thr Leu Glu Asp Leu Leu Met Gly Thr
1               5                   10

<210> 225
<211> 10
<212> PRT
<213> Human papillomavirus type 16

<220>
<223> E7 protein aa 77-86

<400> 225
Arg Thr Leu Glu Asp Leu Leu Met Gly Thr
1               5                   10

<210> 226
<211> 11
<212> PRT
<213> Human papillomavirus type 16

<220>
<223> E7 protein aa 77-87

<400> 226
Arg Thr Leu Glu Asp Leu Leu Met Gly Thr Leu
1               5                   10

<210> 227
<211> 9
<212> PRT
<213> Human papillomavirus type 16

<220>
<223> E7 protein aa 78-86

<400> 227
Thr Leu Glu Asp Leu Leu Met Gly Thr
1               5

<210> 228
<211> 9

<212> PRT
<213> Human papillomavirus type 16

<220>
<223> E7 protein aa 79-87

<400> 228
Leu Glu Asp Leu Leu Met Gly Thr Leu
1               5

<210> 229
<211> 11
<212> PRT
<213> Human papillomavirus type 16

<220>
<223> E7 protein aa 80-90

<400> 229
Glu Asp Leu Leu Met Gly Thr Leu Gly Ile Val
1               5                   10

<210> 230
<211> 10
<212> PRT
<213> Human papillomavirus type 16

<220>
<223> E7 protein aa 81-90

<400> 230
Asp Leu Leu Met Gly Thr Leu Gly Ile Val
1               5                   10

<210> 231
<211> 11
<212> PRT
<213> Human papillomavirus type 16

<220>
<223> E7 protein aa 81-91

<400> 231
Asp Leu Leu Met Gly Thr Leu Gly Ile Val Cys
1               5                   10

<210> 232
<211> 8
<212> PRT
<213> Human papillomavirus type 16

<220>
<223> E7 protein aa 82-89

<400> 232
Leu Leu Met Gly Thr Leu Gly Ile
1               5

<210> 233
<211> 9
<212> PRT
<213> Human papillomavirus type 16

<220>
<223> E7 protein aa 82-90

<400> 233
Leu Leu Met Gly Thr Leu Gly Ile Val
1               5

<210> 234
<211> 10
<212> PRT
<213> Human papillomavirus type 16

<220>
<223> E7 protein aa 82-91

<400> 234
Leu Leu Met Gly Thr Leu Gly Ile Val Cys
1               5                   10

<210> 235
<211> 11
<212> PRT
<213> Human papillomavirus type 16

<220>
<223> E7 protein aa 82-92

<400> 235
Leu Leu Met Gly Thr Leu Gly Ile Val Cys Pro
1               5                   10

<210> 236
<211> 11
<212> PRT
<213> Human papillomavirus type 16

<220>
<223> E7 protein aa 83-93

<400> 236
Leu Met Gly Thr Leu Gly Ile Val Cys Pro Ile
1               5                   10

<210> 237
<211> 10
<212> PRT
<213> Human papillomavirus type 16

<220>
<223> E7 protein aa 84-93

<400> 237
Met Gly Thr Leu Gly Ile Val Cys Pro Ile

1 5 10

<210> 238
<211> 9
<212> PRT
<213> Human papillomavirus type 16

<220>
<223> E7 protein aa 85-93

<400> 238
Gly Thr Leu Gly Ile Val Cys Pro Ile
1 5

<210> 239
<211> 8
<212> PRT
<213> Human papillomavirus type 16

<220>
<223> E7 protein aa 86-93

<400> 239
Thr Leu Gly Ile Val Cys Pro Ile
1 5

<210> 240
<211> 9
<212> PRT
<213> Human papillomavirus type 16

<220>
<223> E7 protein aa 86-94

<400> 240
Thr Leu Gly Ile Val Cys Pro Ile Cys
1 5

<210> 241
<211> 11
<212> PRT
<213> Human papillomavirus type 16

<220>
<223> E7 protein aa 87-97

<400> 241
Leu Gly Ile Val Cys Pro Ile Cys Ser Gln Lys
1 5 10

<210> 242
<211> 10
<212> PRT
<213> Human papillomavirus type 16

<220>
<223> E7 protein aa 88-97

<400> 242
Gly Ile Val Cys Pro Ile Cys Ser Gln Lys
1               5                   10

<210> 243
<211> 9
<212> PRT
<213> Human papillomavirus type 16

<220>
<223> E7 protein aa 89-97

<400> 243
Ile Val Cys Pro Ile Cys Ser Gln Lys
1               5

<210> 244
<211> 9
<212> PRT
<213> Human papillomavirus type 16

<220>
<223> E7 protein aa 90-98

<400> 244
Val Cys Pro Ile Cys Ser Gln Lys Pro
1               5

<210> 245
<211> 9
<212> PRT
<213> Human papillomavirus type 18

<220>
<223> E6 protein aa 13-21

<400> 245
Lys Leu Pro Asp Leu Cys Thr Glu Leu
1               5

<210> 246
<211> 8
<212> PRT
<213> Human papillomavirus type 18

<220>
<223> E6 protein aa 36-43

<400> 246
Lys Thr Val Leu Glu Leu Thr Glu
1               5

<210> 247
<211> 9
<212> PRT
<213> Human papillomavirus type 18

<220>

<223> E6 protein aa 40-48

<400> 247
Glu Leu Thr Glu Val Phe Glu Phe Ala
1 5

<210> 248
<211> 15
<212> PRT
<213> Human papillomavirus type 18

<220>
<223> E6 protein aa 43-57

<400> 248
Glu Val Phe Glu Phe Ala Phe Lys Asp Leu Phe Val Val Tyr Arg
1 5 10 15

<210> 249
<211> 22
<212> PRT
<213> Human papillomavirus type 18

<220>
<223> E6 protein aa 51-72

<400> 249
Asp Leu Phe Val Val Tyr Arg Asp Ser Ile Pro His Ala Ala Cys His
1 5 10 15
Lys Cys Ile Asp Phe Tyr
20

<210> 250
<211> 22
<212> PRT
<213> Human papillomavirus type 18

<220>
<223> E6 protein aa 71-92

<400> 250
Phe Tyr Ser Arg Ile Arg Glu Leu Arg His Tyr Ser Asp Ser Val Tyr
1 5 10 15
Gly Asp Thr Leu Glu Lys
20

<210> 251
<211> 11
<212> PRT
<213> Human papillomavirus type 18

<220>
<223> E6 protein aa 125-135

<400> 251
Arg Arg Phe His Asn Ile Ala Gly His Tyr Arg
1 5 10

<210> 252

<211> 32
<212> PRT
<213> Human papillomavirus type 18

<220>
<223> E7 protein aa 1-32

<400> 252
Met His Gly Pro Lys Ala Thr Leu Gln Asp Ile Val Leu His Leu Glu
1 5 10 15
Pro Gln Asn Glu Ile Pro Val Asp Leu Leu Cys His Glu Gln Leu Ser
20 25 30

<210> 253
<211> 12
<212> PRT
<213> Human papillomavirus type 18

<220>
<223> E7 protein aa 5-16

<400> 253
Lys Ala Thr Leu Gln Asp Ile Val Leu His Leu Glu
1 5 10

<210> 254
<211> 9
<212> PRT
<213> Human papillomavirus type 18

<220>
<223> E7 protein aa 7-15

<400> 254
Thr Leu Gln Asp Ile Val Leu His Leu
1 5

<210> 255
<211> 22
<212> PRT
<213> Human papillomavirus type 18

<220>
<223> E7 protein aa 21-42

<400> 255
Ile Pro Val Asp Leu Leu Cys His Glu Gln Leu Ser Asp Ser Glu Glu
1 5 10 15
Glu Asn Asp Glu Ile Asp
20

<210> 256
<211> 9
<212> PRT
<213> Human papillomavirus type 18

<220>
<223> E7 protein aa 86-94

<400> 256
Phe Gln Gln Leu Phe Leu Asn Thr Leu
1               5

<210> 257
<211> 10
<212> PRT
<213> Human papillomavirus type 18

<220>
<223> E7 protein aa 88-97

<400> 257
Gln Leu Phe Leu Asn Thr Leu Ser Phe Val
1               5                   10

<210> 258
<211> 9
<212> PRT
<213> Human papillomavirus type 16

<220>
<223> E6V (R17I) aa 9-17

<400> 258
Phe Gln Asp Pro Gln Glu Arg Pro Ile
1               5

<210> 259
<211> 11
<212> PRT
<213> Human papillomavirus type 16

<220>
<223> E6V (R17I) aa 9-19

<400> 259
Phe Gln Asp Pro Gln Glu Arg Pro Ile Lys Leu
1               5                   10

<210> 260
<211> 11
<212> PRT
<213> Human papillomavirus type 16

<220>
<223> E6V (R17T) aa 15-25

<400> 260
Arg Pro Thr Lys Leu Pro Gln Leu Cys Thr Glu
1               5                   10

<210> 261
<211> 11
<212> PRT
<213> Human papillomavirus type 16

<220>
<223> E6V (Q21D) aa 18-28

<400> 261
Lys Leu Pro Asp Leu Cys Thr Glu Leu Gln Thr
1 5 10

<210> 262
<211> 9
<212> PRT
<213> Human papillomavirus type 16

<220>
<223> E6V (D32E) aa 25-33

<400> 262
Glu Leu Gln Thr Thr Ile His Glu Ile
1 5

<210> 263
<211> 9
<212> PRT
<213> Human papillomavirus type 16

<220>
<223> E6V (D32E) aa 26-34

<400> 263
Leu Gln Thr Thr Ile His Glu Ile Ile
1 5

<210> 264
<211> 11
<212> PRT
<213> Human papillomavirus type 16

<220>
<223> E6V (D32E) aa 28-38

<400> 264
Thr Thr Ile His Glu Ile Ile Leu Glu Cys Val
1 5 10

<210> 265
<211> 11
<212> PRT
<213> Human papillomavirus type 16

<220>
<223> E6V (D32E, I34R) aa 28-38

<400> 265
Thr Thr Ile His Glu Ile Arg Leu Glu Cys Val
1 5 10

<210> 266
<211> 10

<212> PRT
<213> Human papillomavirus type 16

<220>
<223> E6V (D32E) aa 29-38

<400> 266
Thr Ile His Glu Ile Ile Leu Glu Cys Val
1 5 10

<210> 267
<211> 10
<212> PRT
<213> Human papillomavirus type 16

<220>
<223> E6V (D32E, I34R) aa 29-38

<400> 267
Thr Ile His Glu Ile Arg Leu Glu Cys Val
1 5 10

<210> 268
<211> 11
<212> PRT
<213> Human papillomavirus type 16

<220>
<223> E6V (D32E) aa 31-41

<400> 268
His Glu Ile Ile Leu Glu Cys Val Tyr Cys Lys
1 5 10

<210> 269
<211> 11
<212> PRT
<213> Human papillomavirus type 16

<220>
<223> E6V (D32E, I34R) aa 31-41

<400> 269
His Glu Ile Arg Leu Glu Cys Val Tyr Cys Lys
1 5 10

<210> 270
<211> 10
<212> PRT
<213> Human papillomavirus type 16

<220>
<223> E6V (D32E) aa 32-41

<400> 270
Glu Ile Ile Leu Glu Cys Val Tyr Cys Lys
1 5 10

<210> 271
<211> 8
<212> PRT
<213> Human papillomavirus type 16

<220>
<223> E6V (I34R) aa 34-41

<400> 271
Arg Leu Glu Cys Val Tyr Cys Lys
1               5

<210> 272
<211> 10
<212> PRT
<213> Human papillomavirus type 16

<220>
<223> E6V (A68G) aa 60-69

<400> 272
Val Tyr Arg Asp Gly Asn Pro Tyr Gly Val
1               5                   10

<210> 273
<211> 11
<212> PRT
<213> Human papillomavirus type 16

<220>
<223> E6V (H85Y) aa 79-89

<400> 273
Lys Ile Ser Glu Tyr Arg Tyr Tyr Cys Tyr Ser
1               5                   10

<210> 274
<211> 10
<212> PRT
<213> Human papillomavirus type 16

<220>
<223> E6V (H85Y) aa 81-90

<400> 274
Ser Glu Tyr Arg Tyr Tyr Cys Tyr Ser Leu
1               5                   10

<210> 275
<211> 10
<212> PRT
<213> Human papillomavirus type 16

<220>
<223> E6V (H85Y, L90V) aa 81-90

<400> 275
Ser Glu Tyr Arg Tyr Tyr Cys Tyr Ser Val

1 5 10

<210> 276
<211> 11
<212> PRT
<213> Human papillomavirus type 16

<220>
<223> E6V (L90V) aa 81-91

<400> 276
Ser Glu Tyr Arg His Tyr Cys Tyr Ser Val Tyr
1 5 10

<210> 277
<211> 9
<212> PRT
<213> Human papillomavirus type 16

<220>
<223> E6V (L90V) aa 82-90

<400> 277
Glu Tyr Arg His Tyr Cys Tyr Ser Val
1 5

<210> 278
<211> 8
<212> PRT
<213> Human papillomavirus type 16

<220>
<223> E6V (H85Y) aa 83-90

<400> 278
Tyr Arg Tyr Tyr Cys Tyr Ser Leu
1 5

<210> 279
<211> 8
<212> PRT
<213> Human papillomavirus type 16

<220>
<223> E6V (L90V) aa 83-90

<400> 279
Tyr Arg His Tyr Cys Tyr Ser Val
1 5

<210> 280
<211> 8
<212> PRT
<213> Human papillomavirus type 16

<220>
<223> E6V (H85Y, L90V) aa 83-90

<400> 280
Tyr Arg Tyr Tyr Cys Tyr Ser Val
1 5

<210> 281
<211> 9
<212> PRT
<213> Human papillomavirus type 16

<220>
<223> E6V (L90V) aa 83-91

<400> 281
Tyr Arg His Tyr Cys Tyr Ser Val Tyr
1 5

<210> 282
<211> 8
<212> PRT
<213> Human papillomavirus type 16

<220>
<223> E6V (L90V) aa 84-91

<400> 282
Arg His Tyr Cys Tyr Ser Val Tyr
1 5

<210> 283
<211> 10
<212> PRT
<213> Human papillomavirus type 16

<220>
<223> E6V (H85Y) aa 84-93

<400> 283
Arg Tyr Tyr Cys Tyr Ser Leu Tyr Gly Thr
1 5 10

<210> 284
<211> 11
<212> PRT
<213> Human papillomavirus type 16

<220>
<223> E6V (L90V) aa 84-94

<400> 284
Arg His Tyr Cys Tyr Ser Val Tyr Gly Thr Thr
1 5 10

<210> 285
<211> 10
<212> PRT
<213> Human papillomavirus type 16

<220>

<223> E6V (L90V) aa 86-95

<400> 285
Tyr Cys Tyr Ser Val Tyr Gly Thr Thr Leu
1 5 10

<210> 286
<211> 9
<212> PRT
<213> Human papillomavirus type 16

<220>
<223> E6V (L90V) aa 87-95

<400> 286
Cys Tyr Ser Val Tyr Gly Thr Thr Leu
1 5

<210> 287
<211> 10
<212> PRT
<213> Human papillomavirus type 16

<220>
<223> E6V (L90V) aa 87-96

<400> 287
Cys Tyr Ser Val Tyr Gly Thr Thr Leu Glu
1 5 10

<210> 288
<211> 8
<212> PRT
<213> Human papillomavirus type 16

<220>
<223> E6V (L90V) aa 88-95

<400> 288
Tyr Ser Val Tyr Gly Thr Thr Leu
1 5

<210> 289
<211> 11
<212> PRT
<213> Human papillomavirus type 16

<220>
<223> E6V (L90V) aa 89-99

<400> 289
Ser Val Tyr Gly Thr Thr Leu Glu Gln Gln Tyr
1 5 10

<210> 290
<211> 8
<212> PRT
<213> Human papillomavirus type 16

<220>
<223> E6V (L90V) aa 90-97

<400> 290
Val Tyr Gly Thr Thr Leu Glu Gln
1               5

<210> 291
<211> 10
<212> PRT
<213> Human papillomavirus type 16

<220>
<223> E6V (L90V) aa 90-99

<400> 291
Val Tyr Gly Thr Thr Leu Glu Gln Gln Tyr
1               5                   10

<210> 292
<211> 8
<212> PRT
<213> Human papillomavirus type 16

<220>
<223> E7V (L28F) aa 21-28

<400> 292
Asp Leu Tyr Cys Tyr Glu Gln Phe
1               5

<210> 293
<211> 8
<212> PRT
<213> Human papillomavirus type 16

<220>
<223> E7V (S63F) aa 56-63

<400> 293
Thr Phe Cys Cys Lys Cys Asp Phe
1               5

<210> 294
<211> 10
<212> PRT
<213> Human papillomavirus type 16

<220>
<223> E7V (S63F) aa 56-65

<400> 294
Thr Phe Cys Cys Lys Cys Asp Phe Thr Leu
1               5                   10

<210> 295
<211> 11
<212> PRT

```
<213> Human papillomavirus type 16


<220>
<223> E7V (S63F) aa 56-66

<400> 295
Thr Phe Cys Cys Lys Cys Asp Phe Thr Leu Arg
1               5                   10

<210> 296
<211> 8
<212> PRT
<213> Gallus gallus


<220>
<223> Ovalbumin aa 258-265

<400> 296
Ser Ile Ile Asn Phe Glu Lys Leu
1               5

<210> 297
<211> 9
<212> PRT
<213> Homo sapiens


<220>
<223> Cancer/testis antigen 1 NY-ESO-1 aa 127-135

<400> 297
Thr Val Ser Gly Asn Ile Leu Thr Ile
1               5
```